# EUROPEAN PATENT SPECIFICATION

(11) **EP 2 894 155 B1**
(45) Date of publication and mention of the grant of the patent: **14.11.2018**
(21) Application number: 13832748.1
(22) Date of filing: 27.08.2013
(51) Int. Cl.: C07D 401/04, H01L 51/50

(54) **COMPOUND HAVING INDENO ACRIDAN RING STRUCTURE, AND ORGANIC ELECTROLUMINESCENCE ELEMENT**
VERBINDUNG MIT EINER INDENOACRIDANRINGSTRUKTUR UND EINEM ORGANISCHEN ELEKTROLUMINESZENZELEMENT
COMPOSÉ POSSÉDANT UNE STRUCTURE DE NOYAU INDÉNO ACRIDANE, ET ÉLÉMENT ÉLECTROLUMINESCENT ORGANIQUE

(30) Priority: 03.09.2012 JP 2012192845
(43) Date of publication of application: 15.07.2015
(73) Proprietor: Hodogaya Chemical Co., Ltd., Tokyo 104-0028 (JP)
(72) Inventor: YOKOYAMA, Norimasa, Tokyo 104-0028 (JP); KANDA, Daizou, Tokyo 104-0028 (JP); HAYASHI, Shuichi, Tokyo 104-0028 (JP)
(74) Representative: Müller-Boré & Partner Patentanwälte PartG mbB
(86) International application number: PCT/JP2013/005042
(87) International publication number: WO 2014/034092

(56) References cited:
- WO-A1-2011/060867
- WO-A1-2011/093609
- KR-A- 20120 084 238
- KR-A- 20130 029 132
- KR-A- 20130 064 601

## Description

The present invention relates to compounds suitable for an organic electroluminescent device which is a preferred self-luminous device for various display devices, and relates to the organic electroluminescent device. Specifically, this invention relates to compounds having an indenoacridan ring structure, and organic electroluminescent devices using the compounds.

The organic electroluminescent device is a self-luminous device and has been actively studied for their brighter, superior visibility and the ability to display clearer images in-comparison with liquid crystal devices.

In 1987, C. W. Tang and colleagues at Eastman Kodak developed a laminated structure device using materials assigned with different roles, realizing practical applications of an organic electroluminescent device with organic materials. These researchers laminated an electron-transporting phosphor which is tris(8-hydroxyquinoline)aluminum (hereinafter referred to as Alq₃) and a hole-transporting aromatic amine compound, and injected both charges into a phosphor layer to cause emission in order to obtain a high luminance of 1,000 cd/m² or more at a voltage of 10 V or less (refer to Patent Documents 1 and 2, for example).

To date, various improvements have been made for practical applications of the organic electroluminescent device. In order to realize high efficiency and durability, various roles are further subdivided to provide an electroluminescence device that includes an anode, a hole injection layer, a hole transport layer, a light emitting layer, an electron transport layer, an electron injection layer, and a cathode successively formed on a substrate (refer to Non-Patent Document 1, for example).

Further, there have been attempts to use triplet excitons for further improvements of luminous efficiency, and the use of phosphorescent materials has been examined (refer to Non-Patent Document 2, for example).

Then, there have been developed devices that use a thermally activated delayed fluorescence (TADF). In 2011, Adachi et al. at Kyushu University, National University Corporation realized 5.3% external quantum efficiency with a device using a thermally activated delayed fluorescent material (refer to Non-Patent Document 3, for example).

The light emitting layer can be also fabricated by doping a charge-transporting compound generally called a host material, with a phosphor or a phosphorescent material. As described in the foregoing lecture preprints, the selection of organic materials in an organic electroluminescent device greatly influences various device characteristics such as efficiency and durability.

In an organic electroluminescent device, charges injected from both electrodes recombine in a light emitting layer to cause emission. What is important here is how efficiently the hole and electron charges are transferred to the light emitting layer. The probability of hole-electron recombination can be improved by improving hole injectability and electron blocking performance of blocking injected electrons from the cathode, and high luminous efficiency can be obtained by confining excitons generated in the light emitting layer. The role of a hole transport material is therefore important, and there is a need for a hole transport material that has high hole injectability, high hole mobility, high electron blocking performance, and high durability to electrons.

Heat resistance and amorphousness of the materials are also important with respect to a lifetime of the device. The materials with low heat resistance cause thermal decomposition even at a low temperature by heat generated during the drive of the device, which leads to the deterioration of the materials. The materials with low amorphousness cause crystallization of a thin film even in a short time and lead to the deterioration of the device. The materials in use are therefore required to have characteristics of high heat resistance and satisfactory amorphousness.

N,N'-diphenyl-N,N'-di(α-naphthyl)benzidine (hereinafter referred to as NPD) and various aromatic amine derivatives are known as the hole transport materials used for the organic electroluminescent device (refer to Patent Documents 1 and 2, for example). Although NPD has desirable hole transportability, it has a low glass transition point (Tg) of 96°C which is an index of heat resistance and therefore causes the degradation of device characteristics by crystallization under a high-temperature condition (refer to Non-Patent Document 4, for example). The aromatic amine derivatives described in the Patent Documents 1 and 2 include a compound known to have an excellent hole mobility of 10⁻³ cm²/Vs or higher. However, since the compound is insufficient in terms of electron blocking performance, some of the electrons pass through the light emitting layer, and improvements in luminous efficiency cannot be expected. For such a reason, a material with higher electron blocking performance, a more stable thin-film state and higher heat resistance is needed for higher efficiency.

Arylamine compounds of the following formulae having a substituted acridan structure (for example, Compounds A to C) are proposed as compounds improved in the characteristics such as heat resistance, hole injectability and electron blocking performance (refer to Patent Documents 3 to 5, for example).

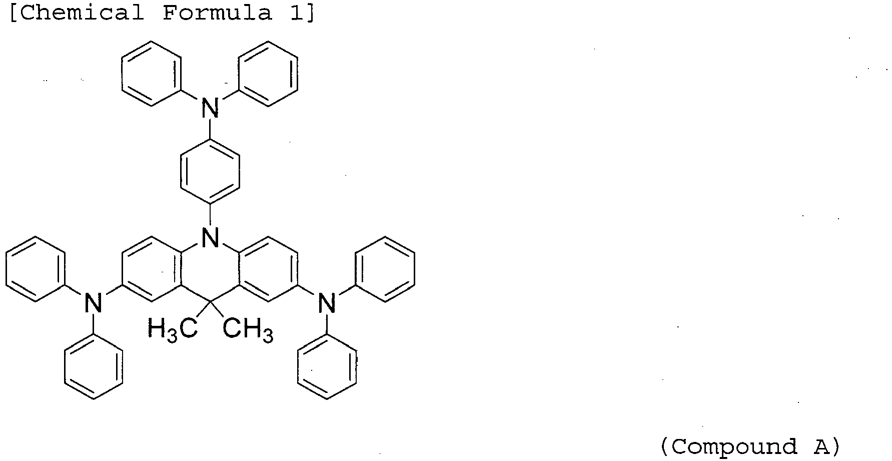

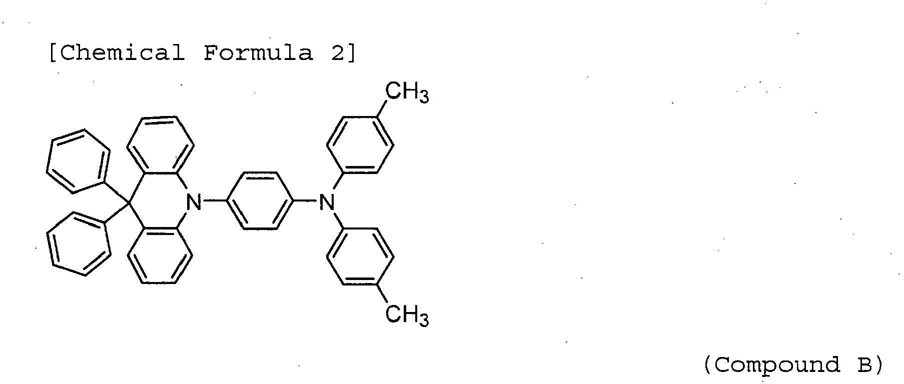

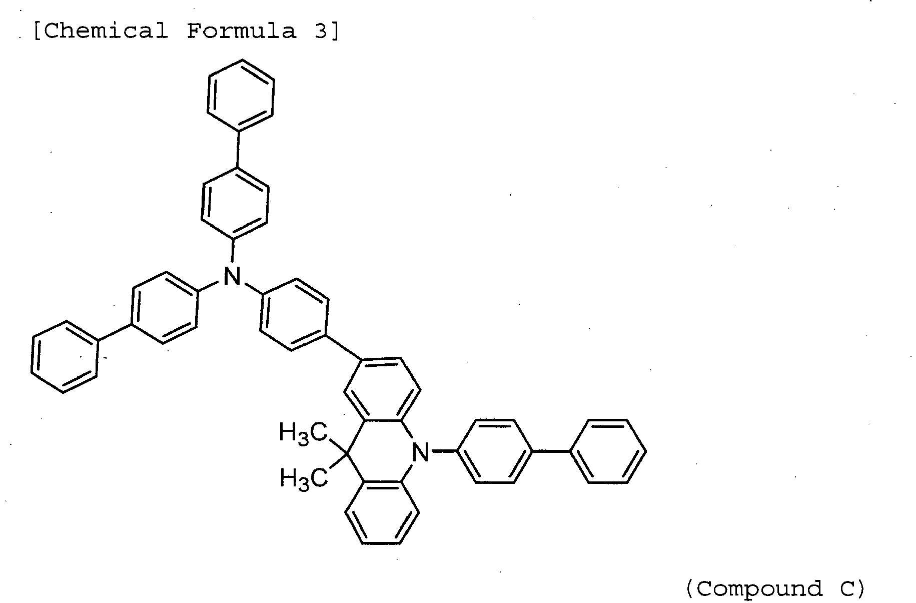

However, while the devices using these compounds for the hole injection layer or the hole transport layer have been improved in heat resistance, luminous efficiency and the like, the improvements are still insufficient. Further, it cannot be said to have a sufficiently low driving voltage and sufficient current efficiency, and there is a problem also in amorphousness. Further improvements of a low driving voltage and luminous efficiency while increasing amorphousness are therefore needed.

Patent Document 1: JP-A-8-048656
Patent Document 2: Japanese Patent No. 3194657
Patent Document 3: WO2006/033563
Patent Document 4: WO2007/110228
Patent Document 5: WO2010/147319
Non-Patent Document 1: The Japan Society of Applied Physics, 9th Lecture Preprints, pp. 55 to 61 (2001)
Non-Patent Document 2: The Japan Society of Applied Physics, 9th Lecture Preprints, pp. 23 to 31 (2001)
Non-Patent Document 3: Appl. Phys. Let., 98, 083302 (2011)
Non-Patent Document 4: Organic EL Symposium, the 3rd Regular presentation Preprints, pp. 13 to 14 (2006)
Non-Patent Document 5: J. Org. Chem., 60, 7508 (1995)
Non-Patent Document 6: Synth. Commun., 11, 513 (1981)

An object of the present invention is to provide an organic compound with characteristics excelling in hole-injecting/transporting performance and having electron blocking ability, high stability in a thin-film state and excellent heat resistance, the organic compound being provided as material for an organic electroluminescent device having high luminous efficiency and high durability. This invention also provides the organic electroluminescent device of high efficiency and high durability using this compound.

Physical properties of the organic compound to be provided by the present invention include (1) good hole injection characteristics, (2) large hole mobility, (3) excellent electron blocking ability, (4) stability in the thin-film state, and (5) excellent heat resistance. Physical properties of the organic electroluminescent device to be provided by the present invention include (1) high luminous efficiency and high power efficiency, (2) low turn on voltage, and (3) low actual driving voltage.

In order to achieve the above objects, the present inventors designed compounds having an indenoacridan ring structure in anticipation of the high hole-injecting/transporting ability of an aromatic tertiary amine structure, the electron blocking performance of the indenoacridan ring structure, and the effect of heat resistance and thin-film stability of these partial structures. The present inventors produced various test organic electroluminescent devices using the compounds chemically synthesized to have the indenoacridan ring structure, and the present invention was completed after thorough evaluations of the device characteristics.
1) Specifically, the present invention is a compound of the following general formula (1) having an indenoacridan ring structure. 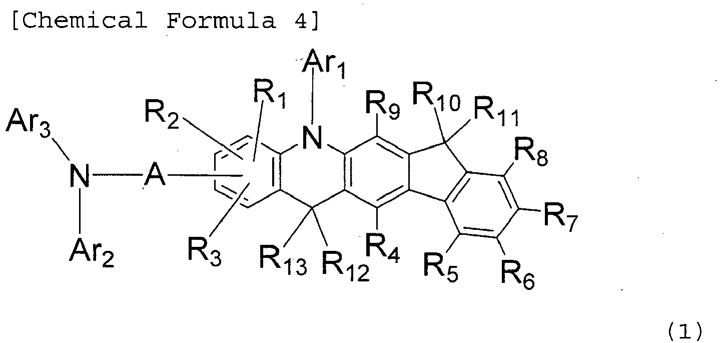 In the formula, A represents a divalent group of a substituted or unsubstituted aromatic hydrocarbon, a divalent group of a substituted or unsubstituted aromatic heterocyclic ring, or a divalent group of substituted or unsubstituted condensed polycyclic aromatics; Ar₁, Ar₂, and Ar₃ may be the same or different, and represent a substituted or unsubstituted aromatic hydrocarbon group, a substituted or unsubstituted aromatic heterocyclic group, or a substituted or unsubstituted condensed polycyclic aromatic group, where the substituted or unsubstituted aromatic hydrocarbon group of Ar₁ is a substituted or unsubstituted biphenyl group, a substituted or unsubstituted terphenyl group, a substituted or unsubstituted fluorenyl group, and where Ar₂ and Ar₃ may bind to each other via a single bond, substituted or unsubstituted methylene, an oxygen atom, or a sulfur atom to form a ring; R₁ to R₉ may be the same or different, and represent a hydrogen atom, a deuterium atom, a fluorine atom, a chlorine atom, cyano, nitro, linear or branched alkyl of 1 to 6 carbon atoms that may have a substituent, cycloalkyl of 5 to 10 carbon atoms that may have a substituent, linear or branched alkenyl of 2 to 6 carbon atoms that may have a substituent, linear or branched alkyloxy of 1 to 6 carbon atoms that may have a substituent, cycloalkyloxy of 5 to 10 carbon atoms that may have a substituent, a substituted or unsubstituted aromatic hydrocarbon group, a substituted or unsubstituted aromatic heterocyclic group, a substituted or unsubstituted condensed polycyclic aromatic group, or substituted or unsubstituted aryloxy, which may bind to each other via a single bond, substituted or unsubstituted methylene, an oxygen atom, or a sulfur atom to form a ring; R₁₀ to R₁₃ may be the same or different, and represent linear or branched alkyl of 1 to 6 carbon atoms that may have a substituent, cycloalkyl of 5 to 10 carbon atoms that may have a substituent, linear or branched alkenyl of 2 to 6 carbon atoms that may have a substituent, linear or branched alkyloxy of 1 to 6 carbon atoms that may have a substituent, cycloalkyloxy of 5 to 10 carbon atoms that may have a substituent, a substituted or unsubstituted aromatic hydrocarbon group, a substituted or unsubstituted aromatic heterocyclic group, a substituted or unsubstituted condensed polycyclic aromatic group, or substituted or unsubstituted aryloxy, where R₁₀ and R₁₁, or R₁₂ and R₁₃ may bind to each other via a single bond, substituted or unsubstituted methylene, an oxygen atom, or a sulfur atom to form a ring; and when A is a divalent group of a substituted or unsubstituted aromatic hydrocarbon, a divalent group of a substituted or unsubstituted aromatic heterocyclic ring, or a divalent group of substituted or unsubstituted condensed polycyclic aromatics, A and Ar₂ bind to each other via a single bond, substituted or unsubstituted methylene, an oxygen atom, or a sulfur atom to form a ring, and wherein the substituent in the group represented by Ar₁ in general formula (1) is selected from a deuterium atom; halogen atoms such as a fluorine atom, a chlorine atom, a bromine atom, and an iodine atom; linear or branched alkyl of 1 to 6 carbon atoms such as methyl, ethyl, n-propyl, isopropyl, n-butyl, isobutyl, tert-butyl, n-pentyl, isopentyl, neopentyl, and n-hexyl; linear or branched alkyloxy of 1 to 6 carbon atoms such as methyloxy, ethyloxy, and propyloxy; alkenyls such as allyl; aryloxys such as phenyloxy and tolyloxy; arylalkyloxys such as benzyloxy and phenethyloxy; aromatic hydrocarbon groups or condensed polycyclic aromatic groups such as phenyl, biphenylyl, terphenylyl, naphthyl, anthracenyl, phenanthryl, fluorenyl, indenyl, pyrenyl, perylenyl, fluoranthenyl, and triphenylenyl; aromatic heterocyclic groups such as pyridyl, thienyl, furyl, pyrrolyl, quinolyl, isoquinolyl, benzofuranyl, benzothienyl, indolyl, carbazolyl, benzooxazolyl, benzothiazolyl, quinoxalyl, benzoimidazolyl, pyrazolyl, dibenzofuranyl, dibenzothienyl, and carbolinyl; arylvinyls such as styryl and naphthylvinyl; and acyls such as acetyl and benzoyl, wherein the substituents may be further substituted with the substituents above, and the substituents may bind to each other, or to Ar₁ via a single bond, substituted or unsubstituted methylene, an oxygen atom, or a sulfur atom to form a ring.
2) Further, the present invention is a compound of the following general formula (1-1) having an indenoacridan ring structure according to 1). 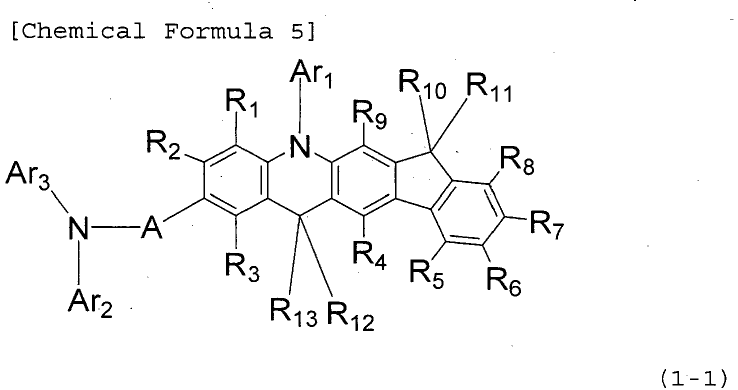 In the formula, A, Ar₁ to Ar₃ and R₁ to R₁₃ are as defined in 1).
3) Further, the present invention is a compound of the general formula (1) or (1-1) having an indenoacridan ring structure according to 1) or 2) in which A is a divalent group of a substituted or unsubstituted aromatic hydrocarbon, or a divalent group of substituted or unsubstituted condensed polycyclic aromatics.
4) Further, the present invention is a compound of the following general formula (1-2) having an indenoacridan ring structure according to 3). 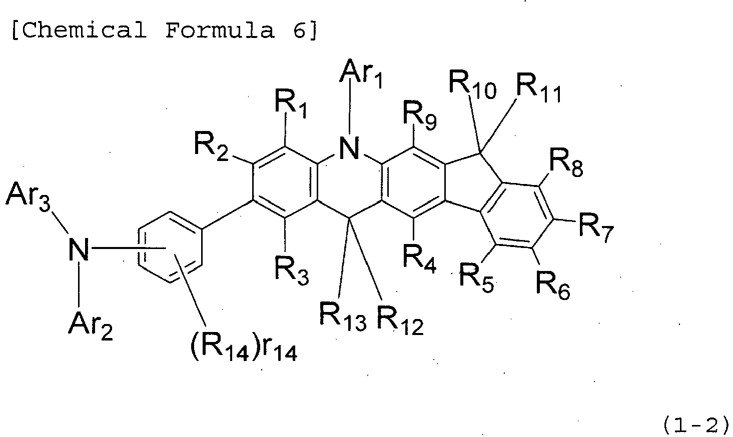 In the formula, Ar₁ to Ar₃ and R₁ to R₁₃ are as defined in 1), R₁₄ is a bonding group, r₁₄ is 1, and the benzene ring binding with R₁₄ and Ar₂ bind to each other via a single bond, substituted or unsubstituted methylene, an oxygen atom, or a sulfur atom to form a ring.
5) Further, the present invention is a compound of the following general formula (1-3) having an indenoacridan ring structure according to 3). 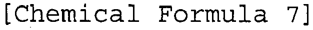 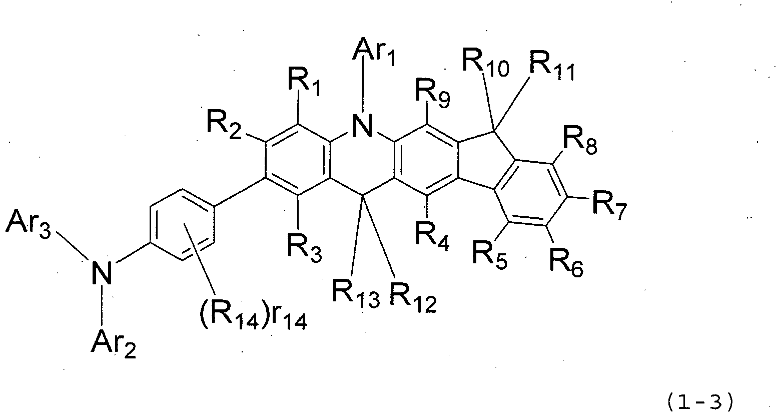 In the formula, Ar₁ to Ar₃ and R₁ to R₁₃ are as defined in 1), R₁₄ is a bonding group, r₁₄ is 1, and the benzene ring binding with R₁₄ and Ar₂ may bind to each other via a single bond, substituted or unsubstituted methylene, an oxygen atom, or a sulfur atom to form a ring.
6) Further, the present invention is a compound of the following general formula (1-4) having an indenoacridan ring structure according to 4). 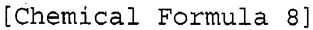 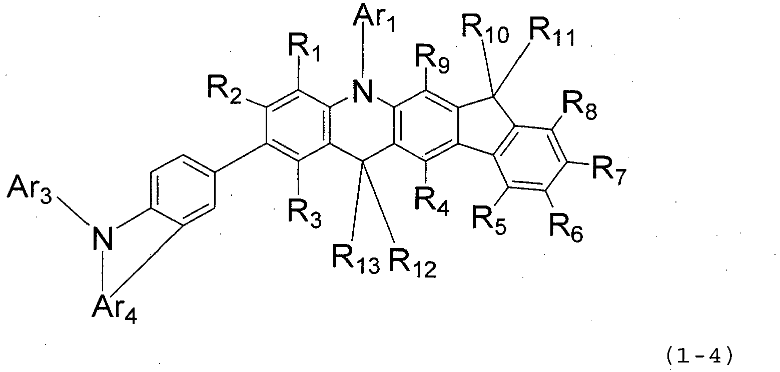 In the formula, Ar₃ represents a substituted or unsubstituted aromatic hydrocarbon group, a substituted or unsubstituted aromatic heterocyclic group, or a substituted or unsubstituted condensed polycyclic aromatic group. Ar₄ represents a divalent group derived from a substituted or unsubstituted aromatic hydrocarbon group, a divalent group derived from a substituted or unsubstituted aromatic heterocyclic group, or a divalent group derived from a substituted or unsubstituted condensed polycyclic aromatic group. Ar₃ and Ar₄ may bind to each other via a single bond, substituted or unsubstituted methylene, an oxygen atom, or a sulfur atom to form a ring. R₁ to R₁₃ are as defined in 1).
7) Further, the present invention is a compound of the following general formula (1-4a) having an indenoacridan ring structure according to 4). 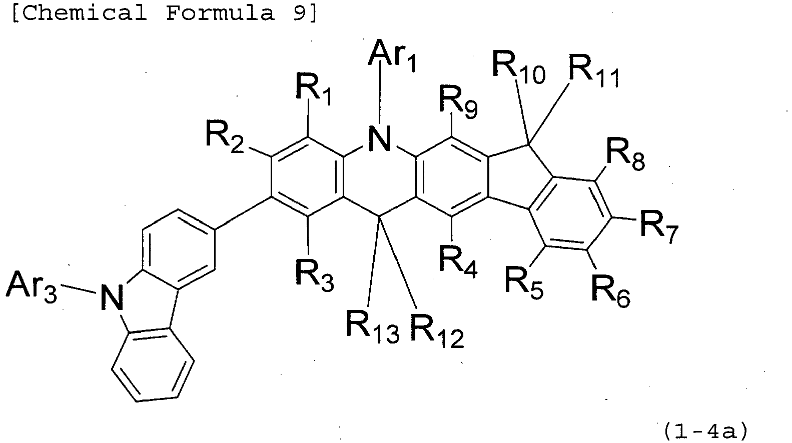 In the formula, Ar₃ represents a substituted or unsubstituted aromatic hydrocarbon group, a substituted or unsubstituted aromatic heterocyclic group, or a substituted or unsubstituted condensed polycyclic aromatic group. R₁ to R₁₃ are as defined in 1).
8) Further, the present invention is a compound of the following general formula (1-4b) having an indenoacridan ring structure according to 4). 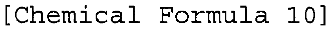 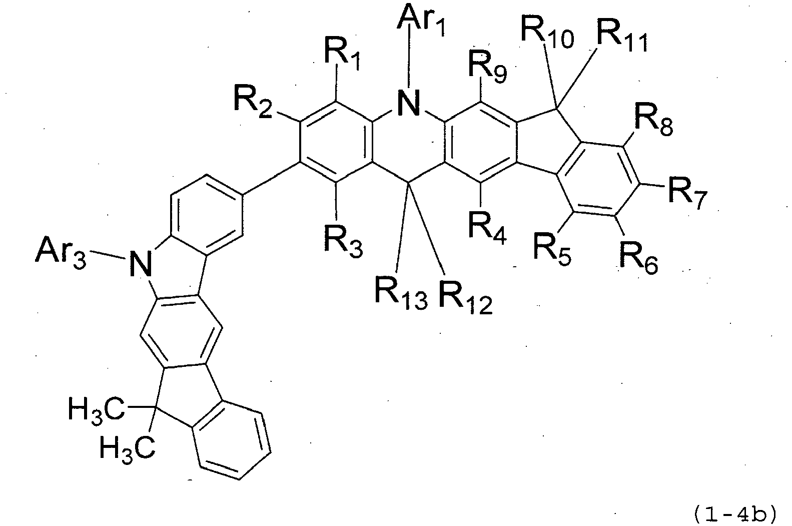 In the formula, Ar₃ represents a substituted or unsubstituted aromatic hydrocarbon group, a substituted or unsubstituted aromatic heterocyclic group, or a substituted or unsubstituted condensed polycyclic aromatic group. R₁ to R₁₃ are as defined in 1).
9) Further, the present invention is an organic electroluminescent device that includes a pair of electrodes, and one or more organic layers sandwiched between the pair of electrodes, wherein the compound having an indenoacridan ring structure according to 1) is used as a constituent material of at least one organic layer.
10) Further, the present invention is an organic electroluminescent device according to 9) in which the organic layer is a hole transport layer.
11) Further, the present invention is an organic electroluminescent device according to 9) in which the organic layer is an electron blocking layer.
12) Further, the present invention is an organic electroluminescent device according to 9) in which the organic layer is a hole injection layer.
13) Further, the present invention is an organic electroluminescent device according to 9) in which the organic layer is a light emitting layer.

Specific examples of the "substituted or unsubstituted aromatic hydrocarbon", "substituted or unsubstituted aromatic heterocyclic ring", or "substituted or unsubstituted condensed polycyclic aromatics" in the "divalent group of a substituted or unsubstituted aromatic hydrocarbon", "divalent group of a substituted or unsubstituted aromatic heterocyclic ring", or "divalent group of substituted or unsubstituted condensed polycyclic aromatics" represented by A in general formula (1) include benzene, biphenyl, terphenyl, tetrakisphenyl, styrene, naphthalene, anthracene, acenaphthalene, fluorene, phenanthrene, indane, pyrene, pyridine, pyrimidine, triazine, furan, pyrrole, thiophene, quinoline, isoquinoline, benzofuran, benzothiophene, indoline, carbazole, carboline, benzoxazole, benzothiazole, quinoxaline, benzimidazole, pyrazole, dibenzofuran, dibenzothiophene, naphthyridine, phenanthroline, and acridine.

The "divalent group of a substituted or unsubstituted aromatic hydrocarbon", "divalent group of a substituted or unsubstituted aromatic heterocyclic ring", or "divalent group of substituted or unsubstituted condensed polycyclic aromatics" represented by A in general formula (1) is a divalent group that result from the removal of two hydrogen atoms from the above "aromatic hydrocarbon", "aromatic heterocyclic ring", or "condensed polycyclic aromatics".

It is preferable that the "aromatic heterocyclic ring" in the "divalent group of a substituted or unsubstituted aromatic heterocyclic ring" is a sulfur-containing aromatic heterocyclic ring such as thiophene, benzothiophene, benzothiazole, or dibenzothiophene; or an oxygen-containing aromatic heterocyclic ring such as furan, benzofuran, benzoxazole, or dibenzofuran.

It is preferable that A in general formula (1) is the "divalent group of a substituted or unsubstituted aromatic hydrocarbon" or the "divalent group of substituted condensed polycyclic aromatics" particularly preferably the divalent group derived from benzene.

Specific examples of the "substituent" of the "aromatic hydrocarbon", "aromatic heterocyclic ring", or "condensed polycyclic aromatics" in the "divalent group of a substituted or unsubstituted aromatic hydrocarbon", "divalent group of a substituted or un substituted aromatic heterocyclic ring", or "divalent group of substituted or unsubstituted condensed polycyclic aromatics" represented by A in general formula (1) can be a deuterium atom; cyano; nitro; halogen atoms such as a fluorine atom, a chlorine atom, a bromine atom, and an iodine atom; linear or branched alkyls of 1 to 6 carbon atoms such as methyl, ethyl, n-propyl, isopropyl, n-butyl, isobutyl, tert-butyl, n-pentyl, isopentyl, neopentyl, and n-hexyl; linear or branched alkyloxy of 1 to 6 carbon atoms such as methyloxy, ethyloxy, and propyloxy; alkenyls such as allyl; aryloxys such as phenyloxy and tolyloxy; arylalkyloxys such as benzyloxy and phenethyloxy; aromatic hydrocarbon groups or condensed polycyclic aromatic groups such as phenyl, biphenylyl, terphenylyl, naphthyl, anthracenyl, phenanthryl, fluorenyl, indenyl, pyrenyl, perylenyl, fluoranthenyl, and triphenylenyl; aromatic heterocyclic groups such as pyridyl, thienyl, furyl, pyrrolyl, quinolyl, isoquinolyl, benzofuranyl, benzothienyl, indolyl, carbazolyl, benzooxazolyl, benzothiazolyl, quinoxalyl, benzoimidazolyl, pyrazolyl, dibenzofuranyl, dibenzothienyl, and carbolinyl; arylvinyls such as styryl and naphthylvinyl; and acyls such as acetyl and benzoyl. These substituents may be further substituted with the above exemplified substituents.

Further, these substituents may bind to each other via a single bond, substituted or unsubstituted methylene, an oxygen atom, or a sulfur atom to form a ring.

Specific examples of the "aromatic hydrocarbon group", "aromatic heterocyclic group", or "condensed polycyclic aromatic group" in the "substituted or unsubstituted aromatic hydrocarbon group", "substituted or unsubstituted aromatic heterocyclic group", or "substituted or unsubstituted condensed polycyclic aromatic group" represented by Ar₁, Ar₂, and Ar₃ in general formula (1) include phenyl, biphenylyl, terphenylyl, naphthyl, anthryl, phenanthryl, fluorenyl, indenyl, pyrenyl, perylenyl, fluoranthenyl, triphenylenyl, pyridyl, furyl, pyrrolyl, thienyl, quinolyl, isoquinolyl, benzofuranyl, benzothienyl, indolyl, carbazolyl, benzoxazolyl, benzothiazolyl, quinoxalyl, benzoimidazolyl, pyrazolyl, dibenzofuranyl, dibenzothienyl, and carbolinyl, where the substituted or unsubstituted aromatic hydrocarbon group of Ar₁ is a substituted or unsubstituted biphenyl group, a substituted or unsubstituted terphenyl group, a substituted or unsubstituted fluorenyl group. These groups may bind to each other via a single bond, substituted or unsubstituted methylene, an oxygen atom, or a sulfur atom to form a ring.

It is preferable that the "aromatic heterocyclic group" in the "substituted or unsubstituted aromatic heterocyclic group" represented by Ar₁, Ar₂ and Ar₃ in general formula (1) is a sulfur-containing aromatic heterocyclic group such as thienyl, benzothienyl, benzothiazolyl, and dibenzothienyl; or an oxygen-containing aromatic heterocyclic group such as furyl, benzofuranyl, benzoxazolyl, and dibenzofuranyl.

It is preferable that Ar₂ in general formula (1) is phenyl, naphthyl, fluorenyl, thienyl, benzothienyl, and dibenzothienyl, particularly preferably phenyl and fluorenyl.

The substituent in the substituted aromatic hydrocarbon group, substituted aromatic heterocyclic group, or substituted condensed polycyclic aromatic group represented by Ar₁ in general formula (1) is selected from a deuterium atom; halogen atoms such as a fluorine atom, a chlorine atom, a bromine atom, and an iodine atom; linear or branched alkyl of 1 to 6 carbon atoms such as methyl, ethyl, n-propyl, isopropyl, n-butyl, isobutyl, tert-butyl, n-pentyl, isopentyl, neopentyl, and n-hexyl; linear or branched alkyloxy of 1 to 6 carbon atoms such as methyloxy, ethyloxy, and propyloxy; alkenyls such as allyl; aryloxys such as phenyloxy and tolyloxy; arylalkyloxys such as benzyloxy and phenethyloxy; aromatic hydrocarbon groups or condensed polycyclic aromatic groups such as phenyl, biphenylyl, terphenylyl, naphthyl, anthracenyl, phenanthryl, fluorenyl, indenyl, pyrenyl, perylenyl, fluoranthenyl, and triphenylenyl; aromatic heterocyclic groups such as pyridyl, thienyl, furyl, pyrrolyl, quinolyl, isoquinolyl, benzofuranyl, benzothienyl, indolyl, carbazolyl, benzooxazolyl, benzothiazolyl, quinoxalyl, benzoimidazolyl, pyrazolyl, dibenzofuranyl, dibenzothienyl, and carbolinyl; arylvinyls such as styryl and naphthylvinyl; and acyls such as acetyl and benzoyl, wherein the substituents may be further substituted with the substituents above, and the substituents may bind to each other, or to Ar₁ via a single bond, substituted or unsubstituted methylene, an oxygen atom, or a sulfur atom to form a ring. Specific examples of the "substituent" in the "substituted aromatic hydrocarbon group", "substituted aromatic heterocyclic group", or "substituted condensed polycyclic aromatic group" represented by Ar₂ and Ar₃ in general formula (1) include a deuterium atom, cyano, nitro; halogen atoms such as a fluorine atom, a chlorine atom, a bromine atom, and an iodine atom; linear or branched alkyl of 1 to 6 carbon atoms such as methyl, ethyl, n-propyl, isopropyl, n-butyl, isobutyl, tert-butyl, n-pentyl, isopentyl, neopentyl, and n-hexyl; linear or branched alkyloxy of 1 to 6 carbon atoms such as methyloxy, ethyloxy, and propyloxy; alkenyls such as allyl; aryloxys such as phenyloxy and tolyloxy; arylalkyloxys such as benzyloxy and phenethyloxy; aromatic hydrocarbon groups or condensed polycyclic aromatic groups such as phenyl, biphenylyl, terphenylyl, naphthyl, anthracenyl, phenanthryl, fluorenyl, indenyl, pyrenyl, perylenyl, fluoranthenyl, and triphenylenyl; aromatic heterocyclic groups such as pyridyl, thienyl, furyl, pyrrolyl, quinolyl, isoquinolyl, benzofuranyl, benzothienyl, indolyl, carbazolyl, benzooxazolyl, benzothiazolyl, quinoxalyl, benzoimidazolyl, pyrazolyl, dibenzofuranyl, dibenzothienyl, and carbolinyl; arylvinyls such as styryl and naphthylvinyl; and acyls such as acetyl and benzoyl. These substituents may be further substituted with the exemplified substituents above. These substituents may bind to each other, or to Ar₂ or Ar₃ via a single bond, substituted or unsubstituted methylene, an oxygen atom, or a sulfur atom to form a ring.

Specific examples of the divalent group derived from a substituted or unsubstituted aromatic hydrocarbon group, divalent group derived from a substituted or unsubstituted aromatic heterocyclic group, or divalent group derived from a substituted or unsubstituted condensed polycyclic aromatic group represented by Ar₄ in general formula (1-4) may include the divalent group derived from the Ar₂ above, and specifically may be the divalent group derived from the same group exemplified as the group for Ar₂ in the general formula (1). Specific examples of the substituent that these groups may have may include the same substituent exemplified as the substituent for Ar₂ above. Further, preferable embodiments may be the same embodiments exemplified as the embodiments for Ar₂ above. The divalent group may bind to Ar₃ via a single bond, substituted or unsubstituted methylene, an oxygen atom, or a sulfur atom to form a ring.

Specific examples of the "linear or branched alkyl of 1 to 6 carbon atoms", "cycloalkyl of 5 to 10 carbon atoms", or "linear or branched alkenyl of 2 to 6 carbon atoms" in the "linear or branched alkyl of 1 to 6 carbon atoms that may have a substituent", "cycloalkyl of 5 to 10 carbon atoms that may have a substituent", or "linear or branched alkenyl of 2 to 6 carbon atoms that may have a substituent" represented by R₁ to R₉ in the general formula (1) include methyl, ethyl, n-propyl, isopropyl, n-butyl, isobutyl, tert-butyl, n-pentyl, isopentyl, neopentyl, n-hexyl, cyclopentyl, cyclohexyl, 1-adamantyl, 2-adamantyl, vinyl, allyl, isopropenyl, and 2-butenyl. These groups may bind to each other via a single bond, substituted or unsubstituted methylene, an oxygen atom, or a sulfur atom to form a ring.

Specific examples of the "substituent" in the "linear or branched alkyl of 1 to 6 carbon atoms that has a substituent", the "cycloalkyl of 5 to 10 carbon atoms that has a substituent", or the "linear or branched alkenyl of 2 to 6 carbon atoms that has a substituent" represented by R₁ to R₉ in the general formula (1) include a deuterium atom; cyano; nitro; halogen atoms such as a fluorine atom, a chlorine atom, a bromine atom, and an iodine atom; linear or branched alkyloxys of 1 to 6 carbon atoms such as methyloxy, ethyloxy, and propyloxy; alkenyls such as allyl; aryloxys such as phenyloxy and tolyloxy; arylalkyloxys such as benzyloxy and phenethyloxy; aromatic hydrocarbon groups or condensed polycyclic aromatic groups such as phenyl, biphenylyl, terphenylyl, naphthyl, anthracenyl, phenanthryl, fluorenyl, indenyl, pyrenyl, perylenyl, fluoranthenyl, and triphenylenyl; and aromatic heterocyclic groups such as pyridyl, thienyl, furyl, pyrrolyl, quinolyl, isoquinolyl, benzofuranyl, benzothienyl, indolyl, carbazolyl, benzooxazolyl, benzothiazolyl, quinoxalyl, benzoimidazolyl, pyrazolyl, dibenzofuranyl, dibenzothienyl, and carbolinyl. These substituents may be further substituted with the exemplified substituents above. These substituents may bind to each other via a single bond, substituted or unsubstituted methylene, an oxygen atom, or a sulfur atom to form a ring.

Specific examples of the "linear or branched alkyloxy of 1 to 6 carbon atoms" or the "cycloalkyloxy of 5 to 10 carbon atoms" in the "linear or branched alkyloxy of 1 to 6 carbon atoms that may have a substituent" or the "cycloalkyloxy of 5 to 10 carbon atoms that may have a substituent" represented by R₁ to R₉ in the general formula (1) include methyloxy, ethyloxy, n-propyloxy, isopropyloxy, n-butyloxy, tert-butyloxy, n-pentyloxy, n-hexyloxy, cyclopentyloxy, cyclohexyloxy, cycloheptyloxy, cyclooctyloxy, 1-adamantyloxy, and 2-adamantyloxy. These groups may bind to each other via a single bond, substituted or unsubstituted methylene, an oxygen atom, or a sulfur atom to form a ring.

Specific examples of the "substituent" in the "linear or branched alkyloxy of 1 to 6 carbon atoms that has a substituent" or the "cycloalkyloxy of 5 to 10 carbon atoms that has a substituent" represented by R₁ to R₉ in the general formula (1) include a deuterium atom; cyano; nitro; halogen atoms such as a fluorine atom, a chlorine atom, a bromine atom, and an iodine atom; linear or branched alkyloxys of 1 to 6 carbon atoms such as methyloxy, ethyloxy, and propyloxy; alkenyls such as allyl; aryloxys such as phenyloxy and tolyloxy; arylalkyloxys such as benzyloxy and phenethyloxy; aromatic hydrocarbon groups or condensed polycyclic aromatic groups such as phenyl, biphenylyl, terphenylyl, naphthyl, anthracenyl, phenanthryl, fluorenyl, indenyl, pyrenyl, perylenyl, fluoranthenyl, and triphenylenyl; and aromatic heterocyclic groups such as pyridyl, thienyl, furyl, pyrrolyl, quinolyl, isoquinolyl, benzofuranyl, benzothienyl, indolyl, carbazolyl, benzooxazolyl, benzothiazolyl, quinoxalyl, benzoimidazolyl, pyrazolyl, dibenzofuranyl, dibenzothienyl, and carbolinyl. These substituents may be further substituted with the exemplified substituents above. These substituents may bind to each other via a single bond, substituted or unsubstituted methylene, an oxygen atom, or a sulfur atom to form a ring.

Specific examples of the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by R₁ to R₉ in the general formula (1) include phenyl, biphenylyl, terphenylyl, naphthyl, anthryl, phenanthryl, fluorenyl, indenyl, pyrenyl, perylenyl, fluoranthenyl, triphenylenyl, pyridyl, furyl, pyrrolyl, thienyl, quinolyl, isoquinolyl, benzofuranyl, benzothienyl, indolyl, carbazolyl, benzooxazolyl, benzothiazolyl, quinoxalyl, benzoimidazolyl, pyrazolyl, dibenzofuranyl, dibenzothienyl, and carbolinyl. These groups may bind to each other via a single bond, substituted or unsubstituted methylene, an oxygen atom, or a sulfur atom to form a ring.

It is preferable that the "aromatic heterocyclic group" in the "substituted or unsubstituted aromatic heterocyclic group" represented by R₁ to R₉ in general formula (1) is a sulfur-containing aromatic heterocyclic group such as thienyl, benzothienyl, benzothiazolyl, and dibenzothienyl.

Specific examples of the "substituent" in the "substituted aromatic hydrocarbon group", the "substituted aromatic heterocyclic group", or the "substituted condensed polycyclic aromatic group" represented by R₁ to Rg in general formula (1) include a deuterium atom; trifluoromethyl; cyano; nitro; halogen atoms such as a fluorine atom, a chlorine atom, a bromine atom, and an iodine atom; linear or branched alkyls of 1 to 6 carbon atoms such as methyl, ethyl, n-propyl, isopropyl, n-butyl, isobutyl, tert-butyl, n-pentyl, isopentyl, neopentyl, and n-hexyl; linear or branched alkyloxys of 1 to 6 carbon atoms such as methyloxy, ethyloxy, and propyloxy; alkenyls such as allyl; aralkyls such as benzyl, naphthylmethyl, and phenethyl; aryloxys such as phenyloxy, tolyloxy; arylalkyloxys such as benzyloxy and phenethyloxy; aromatic hydrocarbon groups or condensed polycyclic aromatic groups such as phenyl, biphenylyl, terphenylyl, naphthyl, anthracenyl, phenanthryl, fluorenyl, indenyl, pyrenyl, perylenyl, fluoranthenyl, and triphenylenyl; aromatic heterocyclic groups such as pyridyl, thienyl, furyl, pyrrolyl, quinolyl, isoquinolyl, benzofuranyl, benzothienyl, indolyl, carbazolyl, benzooxazolyl, benzothiazolyl, quinoxalyl, benzoimidazolyl, pyrazolyl, dibenzofuranyl, dibenzothienyl, and carbolinyl; arylvinyls such as styryl and naphthylvinyl; acyls such as acetyl and benzoyl; dialkylamino groups such as dimethylamino and diethylamino; disubstituted amino groups such as diphenylamino and dinaphthylamino, substituted with aromatic hydrocarbon groups or condensed polycyclic aromatic groups; diaralkylamino groups such as dibenzylamino and diphenethylamino; disubstituted amino groups such as dipyridylamino and dithienylamino, substituted with aromatic heterocyclic groups; dialkenylamino groups such as diallylamino; and disubstituted amino groups substituted with a substituent selected from alkyl, an aromatic hydrocarbon group, a condensed polycyclic aromatic group, aralkyl, an aromatic heterocyclic group, and alkenyl. These substituents may be further substituted with the exemplified substituents above. These substituents may bind to each other via a single bond, substituted or unsubstituted methylene, an oxygen atom, or a sulfur atom to form a ring.

Specific examples of the "aryloxy" in the "substituted or unsubstituted aryloxy" represented by R₁ to R₉ in general formula (1) include phenyloxy, biphenylyloxy, terphenylyloxy, naphthyloxy, anthryloxy, phenanthryloxy, fluorenyloxy, indenyloxy, pyrenyloxy, and perylenyloxy. These groups may bind to each other via a single bond, substituted or unsubstituted methylene, an oxygen atom, or a sulfur atom to form a ring.

Specific examples of the "substituent" in the "substituted aryloxy" represented by R₁ to R₉ in general formula (1) include a deuterium atom; trifluoromethyl; cyano; nitro; halogen atoms such as a fluorine atom, a chlorine atom, a bromine atom, and an iodine atom; linear or branched alkyls of 1 to 6 carbon atoms such as methyl, ethyl, n-propyl, isopropyl, n-butyl, isobutyl, tert-butyl, n-pentyl, isopentyl, neopentyl, and n-hexyl; linear or branched alkyloxys of 1 to 6 carbon atoms such as methyloxy, ethyloxy, and propyloxy; alkenyls such as allyl; aralkyls such as benzyl, naphthylmethyl, and phenethyl; aryloxys such as phenyloxy, tolyloxy; arylalkyloxys such as benzyloxy and phenethyloxy; aromatic hydrocarbon groups or condensed polycyclic aromatic groups such as phenyl, biphenylyl, terphenylyl, naphthyl, anthracenyl, phenanthryl, fluorenyl, indenyl, pyrenyl, perylenyl, fluoranthenyl, and triphenylenyl; aromatic heterocyclic groups such as pyridyl, thienyl, furyl, pyrrolyl, quinolyl, isoquinolyl, benzofuranyl, benzothienyl, indolyl, carbazolyl, benzooxazolyl, benzothiazolyl, quinoxalyl, benzoimidazolyl, pyrazolyl, dibenzofuranyl, dibenzothienyl, and carbolinyl; arylvinyls such as styryl and naphthylvinyl; acyls such as acetyl and benzoyl; dialkylamino groups such as dimethylamino and diethylamino; disubstituted amino groups such as diphenylamino and dinaphthylamino, substituted with aromatic hydrocarbon groups or condensed polycyclic aromatic groups; diaralkylamino groups such as dibenzylamino and diphenethylamino; disubstituted amino groups such as dipyridylamino and dithienylamino, substituted with aromatic heterocyclic groups; dialkenylamino groups such as diallylamino; and disubstituted amino groups substituted with a substituent selected from alkyl, an aromatic hydrocarbon group, a condensed polycyclic aromatic group, aralkyl, an aromatic heterocyclic group, and alkenyl. These substituents may be further substituted with the exemplified substituents above. These substituents may bind to each other via a single bond, substituted or unsubstituted methylene, an oxygen atom, or a sulfur atom to form a ring.

Specific examples of the "linear or branched alkyl of 1 to 6 carbon atoms", "cycloalkyl of 5 to 10 carbon atoms", or "linear or branched alkenyl of 2 to 6 carbon atoms" in the "linear or branched alkyl of 1 to 6 carbon atoms that may have a substituent", "cycloalkyl of 5 to 10 carbon atoms that may have a substituent", or "linear or branched alkenyl of 2 to 6 carbon atoms that may have a substituent" represented by R₁₀ to R₁₃ in the general formula (1) include methyl, ethyl, n-propyl, isopropyl, n-butyl, isobutyl, tert-butyl, n-pentyl, isopentyl, neopentyl, n-hexyl, cyclopentyl, cyclohexyl, 1-adamantyl, 2-adamantyl, vinyl, allyl, isopropenyl, and 2-butenyl. These groups may bind to each other via a single bond, substituted or unsubstituted methylene, an oxygen atom, or a sulfur atom to form a ring.

Specific examples of the "substituent" in the "linear or branched alkyl of 1 to 6 carbon atoms that has a substituent", the "cycloalkyl of 5 to 10 carbon atoms that has a substituent", or the "linear or branched alkenyl of 2 to 6 carbon atoms that has a substituent" represented by R₁₀ to R₁₃ in the general formula (1) include a deuterium atom; cyano; nitro; halogen atoms such as a fluorine atom, a chlorine atom, a bromine atom, and an iodine atom; linear or branched alkyloxys of 1 to 6 carbon atoms such as methyloxy, ethyloxy, and propyloxy; alkenyls such as allyl; aryloxys such as phenyloxy, tolyloxy; arylalkyloxys such as benzyloxy and phenethyloxy; aromatic hydrocarbon groups or condensed polycyclic aromatic groups such as phenyl, biphenylyl, terphenylyl, naphthyl, anthracenyl, phenanthryl, fluorenyl, indenyl, pyrenyl, perylenyl, fluoranthenyl, and triphenylenyl; and aromatic heterocyclic groups such as pyridyl, thienyl, furyl, pyrrolyl, quinolyl, isoquinolyl, benzofuranyl, benzothienyl, indolyl, carbazolyl, benzooxazolyl, benzothiazolyl, quinoxalyl, benzoimidazolyl, pyrazolyl, dibenzofuranyl, dibenzothienyl, and carbolinyl. These substituents may be further substituted with the exemplified substituents above. These substituents may bind to each other via a single bond, substituted or unsubstituted methylene, an oxygen atom, or a sulfur atom to form a ring.

Specific examples of the "linear or branched alkyloxy of 1 to 6 carbon atoms" or the "cycloalkyloxy of 5 to 10 carbon atoms" in the "linear or branched alkyloxy of 1 to 6 carbon atoms that may have a substituent" or the "cycloalkyloxy of 5 to 10 carbon atoms that may have a substituent" represented by R₁₀ to R₁₃ in the general formula (1) include methyloxy, ethyloxy, n-propyloxy, isopropyloxy, n-butyloxy, tert-butyloxy, n-pentyloxy, n-hexyloxy, cyclopentyloxy, cyclohexyloxy, cycloheptyloxy, cyclooctyloxy, 1-adamantyloxy, and 2-adamantyloxy. These groups may bind to each other via a single bond, substituted or unsubstituted methylene, an oxygen atom, or a sulfur atom to form a ring.

Specific examples of the "substituent" in the "linear or branched alkyloxy of 1 to 6 carbon atoms that has a substituent" or the "cycloalkyloxy of 5 to 10 carbon atoms that has a substituent" represented by R₁₀ to R₁₃ in the general formula (1) include a deuterium atom; cyano; nitro; halogen atoms such as a fluorine atom, a chlorine atom, a bromine atom, and an iodine atom; linear or branched alkyloxys of 1 to 6 carbon atoms such as methyloxy, ethyloxy, and propyloxy; alkenyls such as allyl; aryloxys such as phenyloxy and tolyloxy; arylalkyloxys such as benzyloxy and phenethyloxy; aromatic hydrocarbon groups or condensed polycyclic aromatic groups such as phenyl, biphenylyl, terphenylyl, naphthyl, anthracenyl, phenanthryl, fluorenyl, indenyl, pyrenyl, perylenyl, fluoranthenyl, and triphenylenyl; and aromatic heterocyclic groups such as pyridyl, thienyl, furyl, pyrrolyl, quinolyl, isoquinolyl, benzofuranyl, benzothienyl, indolyl, carbazolyl, benzooxazolyl, benzothiazolyl, quinoxalyl, benzoimidazolyl, pyrazolyl, dibenzofuranyl, dibenzothienyl, and carbolinyl. These substituents may be further substituted with the exemplified substituents above. These substituents may bind to each other via a single bond, substituted or unsubstituted methylene, an oxygen atom, or a sulfur atom to form a ring.

Specific examples of the "aromatic hydrocarbon group", "aromatic heterocyclic group", or "condensed polycyclic aromatic group" in the "substituted or unsubstituted aromatic hydrocarbon group", "substituted or unsubstituted aromatic heterocyclic group", or "substituted or unsubstituted condensed polycyclic aromatic group" represented by R₁₀ to R₁₃ in general formula (1) include phenyl, biphenylyl, terphenylyl, naphthyl, anthryl, phenanthryl, fluorenyl, indenyl, pyrenyl, perylenyl, fluoranthenyl, triphenylenyl, pyridyl, furyl, pyrrolyl, thienyl, quinolyl, isoquinolyl, benzofuranyl, benzothienyl, indolyl, carbazolyl, benzoxazolyl, benzothiazolyl, quinoxalyl, benzoimidazolyl, pyrazolyl, dibenzofuranyl, dibenzothienyl, and carbolinyl. These groups may bind to each other via a single bond, substituted or unsubstituted methylene, an oxygen atom, or a sulfur atom to form a ring.

It is preferable that the "aromatic heterocyclic group" in the "substituted or unsubstituted aromatic heterocyclic group" represented by R₁₀ to R₁₃ in general formula (1) is a sulfur-containing aromatic heterocyclic group such as thienyl, benzothienyl, benzothiazolyl, and dibenzothienyl.

Specific examples of the "substituent" in the "substituted aromatic hydrocarbon group", the "substituted aromatic heterocyclic group", or the "substituted condensed polycyclic aromatic group" represented by R₁₀ to R₁₃ in general formula (1) include a deuterium atom; trifluoromethyl; cyano; nitro; halogen atoms such as a fluorine atom, a chlorine atom, a bromine atom, and an iodine atom; linear or branched alkyls of 1 to 6 carbon atoms such as methyl, ethyl, n-propyl, isopropyl, n-butyl, isobutyl, tert-butyl, n-pentyl, isopentyl, neopentyl, and n-hexyl; linear or branched alkyloxys of 1 to 6 carbon atoms such as methyloxy, ethyloxy, and propyloxy; alkenyls such as allyl; aralkyls such as benzyl, naphthylmethyl, and phenethyl; aryloxys such as phenyloxy, tolyloxy; arylalkyloxys such as benzyloxy and phenethyloxy; aromatic hydrocarbon groups or condensed polycyclic aromatic groups such as phenyl, biphenylyl, terphenylyl, naphthyl, anthracenyl, phenanthryl, fluorenyl, indenyl, pyrenyl, perylenyl, fluoranthenyl, and triphenylenyl; aromatic heterocyclic groups such as pyridyl, thienyl, furyl, pyrrolyl, quinolyl, isoquinolyl, benzofuranyl, benzothienyl, indolyl, carbazolyl, benzooxazolyl, benzothiazolyl, quinoxalyl, benzoimidazolyl, pyrazolyl, dibenzofuranyl, dibenzothienyl, and carbolinyl; arylvinyls such as styryl and naphthylvinyl; acyls such as acetyl and benzoyl; dialkylamino groups such as dimethylamino and diethylamino; disubstituted amino groups such as diphenylamino and dinaphthylamino, substituted with aromatic hydrocarbon groups or condensed polycyclic aromatic groups; diaralkylamino groups such as dibenzylamino and diphenethylamino; disubstituted amino groups such as dipyridylamino and dithienylamino, substituted with aromatic heterocyclic groups; dialkenylamino groups such as diallylamino; and disubstituted amino groups substituted with a substituent selected from alkyl, an aromatic hydrocarbon group, a condensed polycyclic aromatic group, aralkyl, an aromatic heterocyclic group, and alkenyl. These substituents may be further substituted with the exemplified substituents above. These substituents may bind to each other via a single bond, substituted or unsubstituted methylene, an oxygen atom, or a sulfur atom to form a ring.

Specific examples of the "aryloxy" in the "substituted or unsubstituted aryloxy" represented by R₁₀ to R₁₃ in general formula (1) include phenyloxy, biphenylyloxy, terphenylyloxy, naphthyloxy, anthryloxy, phenanthryloxy, fluorenyloxy, indenyloxy, pyrenyloxy, and perylenyloxy. These groups may bind to each other via a single bond, substituted or unsubstituted methylene, an oxygen atom, or a sulfur atom to form a ring.

Specific examples of the "substituent" in the "substituted aryloxy" represented by R₁₀ to R₁₃ in general formula (1) include a deuterium atom; trifluoromethyl; cyano; nitro; halogen atoms such as a fluorine atom, a chlorine atom, a bromine atom, and an iodine atom; linear or branched alkyls of 1 to 6 carbon atoms such as methyl, ethyl, n-propyl, isopropyl, n-butyl, isobutyl, tert-butyl, n-pentyl, isopentyl, neopentyl, and n-hexyl; linear or branched alkyloxys of 1 to 6 carbon atoms such as methyloxy, ethyloxy, and propyloxy; alkenyls such as allyl; aralkyls such as benzyl, naphthylmethyl, and phenethyl; aryloxys such as phenyloxy, tolyloxy; arylalkyloxys such as benzyloxy and phenethyloxy; aromatic hydrocarbon groups or condensed polycyclic aromatic groups such as phenyl, biphenylyl, terphenylyl, naphthyl, anthracenyl, phenanthryl, fluorenyl, indenyl, pyrenyl, perylenyl, fluoranthenyl, and triphenylenyl; aromatic heterocyclic groups such as pyridyl, thienyl, furyl, pyrrolyl, quinolyl, isoquinolyl, benzofuranyl, benzothienyl, indolyl, carbazolyl, benzooxazolyl, benzothiazolyl, quinoxalyl, benzoimidazolyl, pyrazolyl, dibenzofuranyl, dibenzothienyl, and carbolinyl; arylvinyls such as styryl and naphthylvinyl; acyls such as acetyl and benzoyl; dialkylamino groups such as dimethylamino and diethylamino; disubstituted amino groups such as diphenylamino and dinaphthylamino, substituted with aromatic hydrocarbon groups or condensed polycyclic aromatic groups; diaralkylamino groups such as dibenzylamino and diphenethylamino; disubstituted amino groups such as dipyridylamino and dithienylamino, substituted with aromatic heterocyclic groups; dialkenylamino groups such as diallylamino; and disubstituted amino groups substituted with a substituent selected from alkyl, an aromatic hydrocarbon group, a condensed polycyclic aromatic group, aralkyl, an aromatic heterocyclic group, and alkenyl. These substituents may be further substituted with the exemplified substituents above. These substituents may bind to each other via a single bond, substituted or unsubstituted methylene, an oxygen atom, or a sulfur atom to form a ring.

It is preferable that R₁₀ to R₁₃ in general formula (1) are "linear or branched alkyl of 1 to 6 carbon atoms that may have a substituent" and "substituted or unsubstituted aromatic hydrocarbon group", further preferably methyl and phenyl, and particularly preferably methyl.

Specific examples of the "linear or branched alkyl of 1 to 6 carbon atoms", "cycloalkyl of 5 to 10 carbon atoms", or "linear or branched alkenyl of 2 to 6 carbon atoms" in the "linear or branched alkyl of 1 to 6 carbon atoms that may have a substituent", "cycloalkyl of 5 to 10 carbon atoms that may have a substituent", or "linear or branched alkenyl of 2 to 6 carbon atoms that may have a substituent" represented by R₁₄ in the general formulae (1-2) and (1-3) may include the same group exemplified as the group for R₁ to R₉ in the general formula (1) above. Specific examples of the substituent that these groups may have may be the same substituent exemplified as the substituent for R₁ to R₉ above. Further, preferable embodiments may be the same embodiments exemplified as the embodiments for R₁ to R₉ above. These groups may bind to each other via a single bond, substituted or unsubstituted methylene, an oxygen atom, or a sulfur atom to form a ring when a plurality of R₁₄ are present (when r₁₄ is 2 or more).

Specific examples of the "linear or branched alkyloxy of 1 to 6 carbon atoms" or "cycloalkyloxy of 5 to 10 carbon atoms" in the "linear or branched alkyloxy of 1 to 6 carbon atoms that may have a substituent" or "cycloalkyloxy of 5 to 10 carbon atoms that may have a substituent" represented by R₁₄ in the general formulae (1-2) and (1-3) may include the same group exemplified as the group for R₁ to R₉ in the general formula (1) above. Specific examples of the substituent that these groups may have may include the same substituent exemplified as the substituent for R₁ to R₉ above. Further, preferable embodiments may be the same embodiments exemplified, as embodiments for R₁ to R₉ above. These groups may bind to each other via a single bond, substituted or unsubstituted methylene, an oxygen atom, or a sulfur atom to form a ring when a plurality of R₁₄ are present (when r₁₄ is 2 or more).

Specific examples of the "aromatic hydrocarbon group", "aromatic heterocyclic group", or "condensed polycyclic aromatic group" in the "substituted or unsubstituted aromatic hydrocarbon group", "substituted or unsubstituted aromatic heterocyclic group", or "substituted or unsubstituted condensed polycyclic aromatic group" represented by R₁₄ in the general formulae (1-2) and (1-3) may include the same group exemplified as the group for R₁ to R₉ in the general formula (1) above. Specific examples of the substituent that these groups may have may include the same substituent exemplified as the substituent for R₁ to R₉ above. Further, preferable embodiments may be the same embodiments exemplified as the embodiments for R₁ to R₉ above. These groups may bind to each other via a single bond, substituted or unsubstituted methylene, an oxygen atom, or a sulfur atom to form a ring when a plurality of R₁₄ are present (when r₁₄ is 2 or more).

Specific examples of the "aryloxy" in the "substituted or unsubstituted aryloxy" represented by R₁₄ in the general formulae (1-2) and (1-3) may include the same group exemplified as the group for R₁ to R₉ in the general formula (1) above. Specific examples of the substituent that these groups may have may include the same substituent exemplified as the substituent for R₁ to R₉ above. Further, preferable embodiments may be the same embodiments exemplified as embodiments for R₁ to R₉ above. These groups may bind to each other via a single bond, substituted or unsubstituted methylene, an oxygen atom, or a sulfur atom to form a ring when a plurality of R₁₄ are present (when r₁₄ is 2 or more).

The compounds of general formula (1) having an indenoacridan ring structure of the present invention are novel compounds and have superior electron blocking ability, superior amorphousness and a more stable thin-film state compared to conventional hole transport materials.

The compounds of general formula (1) having an indenoacridan ring structure of the present invention can be used as a constituent material of the hole injection layer and/or hole transport layer of an organic electroluminescent device (hereinafter referred to as an organic EL device). With the use of material having higher hole injectability, higher mobility, higher electron blocking performance and higher stability to electrons than conventional materials, excitons generated in a light emitting layer can be confined, and the probability of hole-electron recombination can be improved. This improves luminous efficiency, lowers driving voltage and thus improves the durability of the organic EL device.

The compounds of general formula (1) having an indenoacridan ring structure of the present invention can also be used as a constituent material of the electron blocking layer of an organic EL device. With the use of material having an excellent electron blocking ability and having superior hole transportability and higher stability in a thin-film state than conventional materials, driving voltage is lowered and current resistance is improved while maintaining high luminous efficiency. As a result, the maximum emission luminance of the organic EL device is improved.

The compounds of general formula (1) having an indenoacridan ring structure of the present invention can also be used as a constituent material of the light emitting layer of the organic EL device. The material of the present invention having superior hole transportability and a wider band gap than conventional materials is used as the host material of the light emitting layer in order to form the light emitting layer by carrying a fluorescent material or phosphorescent material called a dopant. In this way, the organic EL device with a low driving voltage and improved luminous efficiency can be achieved.

The high efficiency and high durability of the organic EL device in the present invention can be achieved because of the use of the compound having an indenoacridan ring structure, which has greater hole mobility, superior electron blocking ability and superior amorphousness than conventional hole transport materials as well as a stable thin-film state.

The compound having an indenoacridan ring structure of the present invention is useful as the constituent material of the hole injection layer, hole transport layer, electron blocking layer, or light emitting layer of the organic EL device. The compound has an excellent electron blocking ability and satisfactory amorphousness, and excels in heat resistance as well as a stable thin-film state. The organic EL device of the present invention has high luminous efficiency and high power efficiency, and the actual driving voltage of the device can thereby be lowered. Further, turn on voltage can be lowered to improve durability.

FIG. 1 is a ¹H-NMR chart of the compound of Reference Example 1 of the present invention (Compound 2).

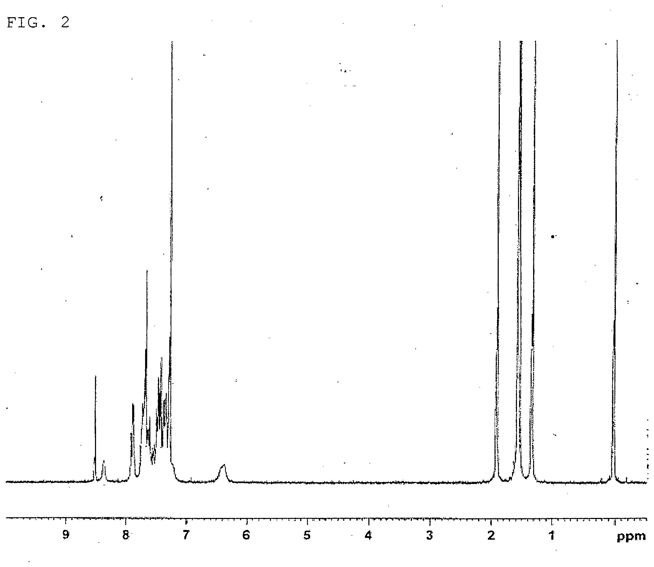

FIG. 2 is a ¹H-NMR chart of the compound of Reference Example 2 of the present invention (Compound 3).

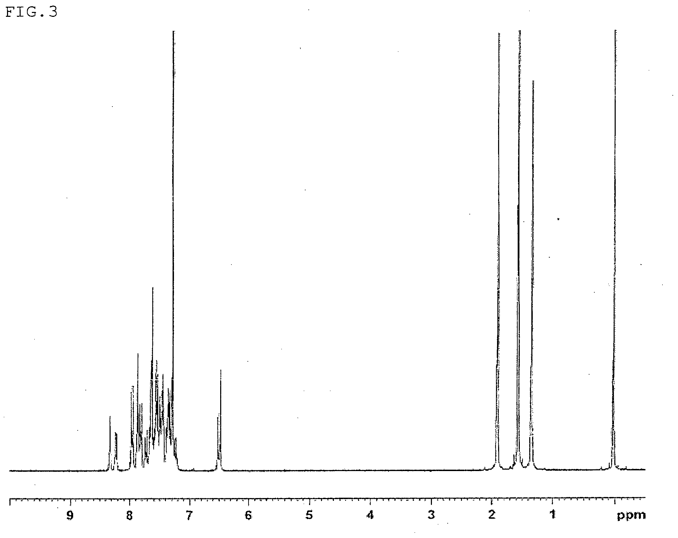

FIG. 3 is a ¹H-NMR chart of the compound of Example 3 of the present invention (Compound 18).
FIG. 4 is a ¹H-NMR chart of the compound of Example 4 of the present invention (Compound 19).
FIG. 5 is a diagram illustrating the configuration of the EL devices of Examples 7 to 10 and Comparative Example 1.

The compounds having an indenoacridan ring structure of the present invention are novel compounds, and may be synthesized, for example, as follows. For example, after 2-(9,9-dimethylfluorene-2-yl)amino methyl benzoate is synthesized by the reaction of 2- amino methyl benzoate with 9,9-dimethyl-2-iodofluorene, 2-{2-(9,9-dimethylfluorene-2-yl)amino)phenyl}propane-2-ol is synthesized by the reaction with methylmagnesium chloride, and then, 7,7,13,13-tetramethyl-7,13-dihydro-5H-indeno[1,2-b]acridin can be synthesized by a cyclization reaction. Further, the 7,7,13,13-tetramethyl-7,13-dihydro-5H-indeno[1,2-b]acridin can be subjected to a condensation reaction such as a Buchward-Hartwig reaction with aryl halide to synthesize an indenoacridan derivative substituted with an aryl group at the 5-position. Further, the indenoacridan derivative can be brominated with N-bromosuccinimide or the like to synthesize an indenoacridan derivative brominated at the 2 position. A bromo compound having substituents at different positions can be obtained by changing reagents and conditions of bromination.

Further, the above indenoacridan derivative can be reacted with various boronic acid or borate (refer to Non-Patent Document 5, for example) in a cross-coupling reaction such as Suzuki coupling (refer to Non-Patent Document 6, for example) to synthesize the compounds having an indenoacridan ring structure of the present invention.

Also, the compounds having an indenoacridan ring structure of the present invention may be synthesized, for example, as follows. The above indenoacridan derivative substituted with an aryl group at the 5-position can be reacted with various diarylamine in a cross-coupling reaction such as Buchward-Hartwig reaction to synthesize the compounds having an indenoacridan ring structure of the present invention.

The following presents specific examples (i.e. Compounds 4, 16, 18 to 22, 39 and 40) of preferred compounds among the compounds of general formula (1) having an indenoacridan ring structure. Compounds 2, 3, 5 to 17 and 23 to 38 are compounds for reference. The present invention, however, is not restricted to these compounds.

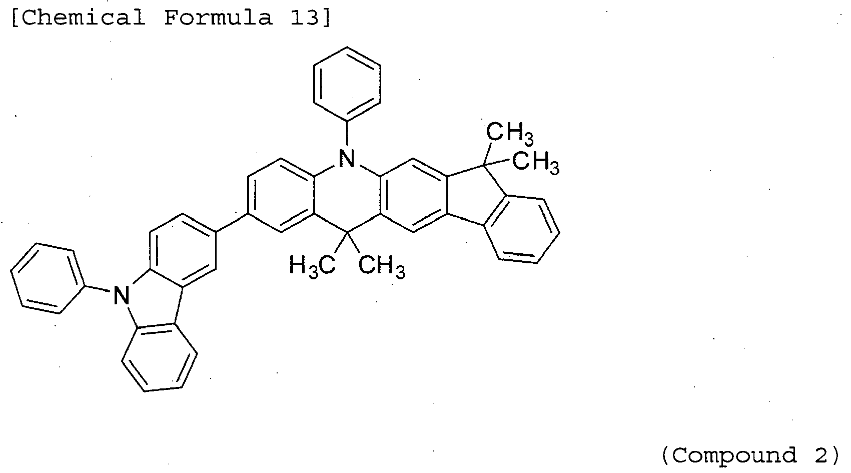

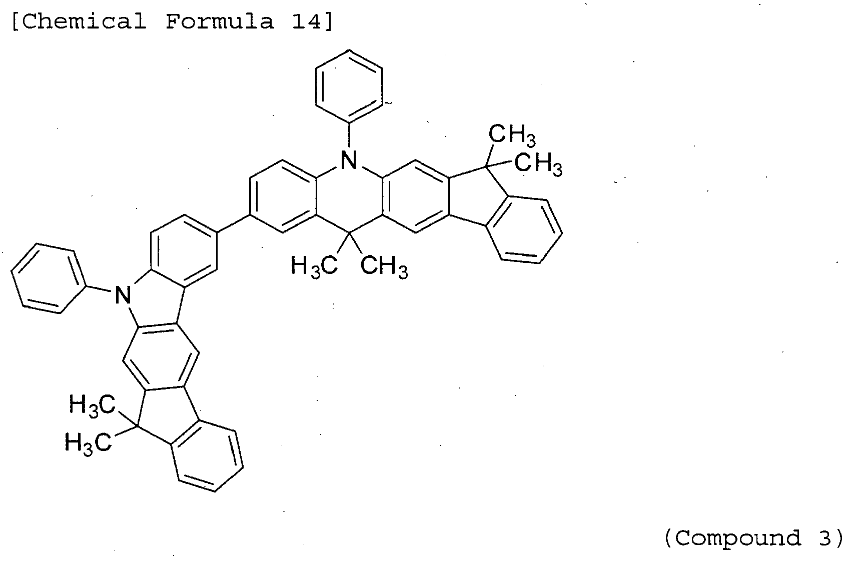

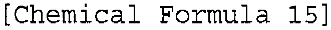

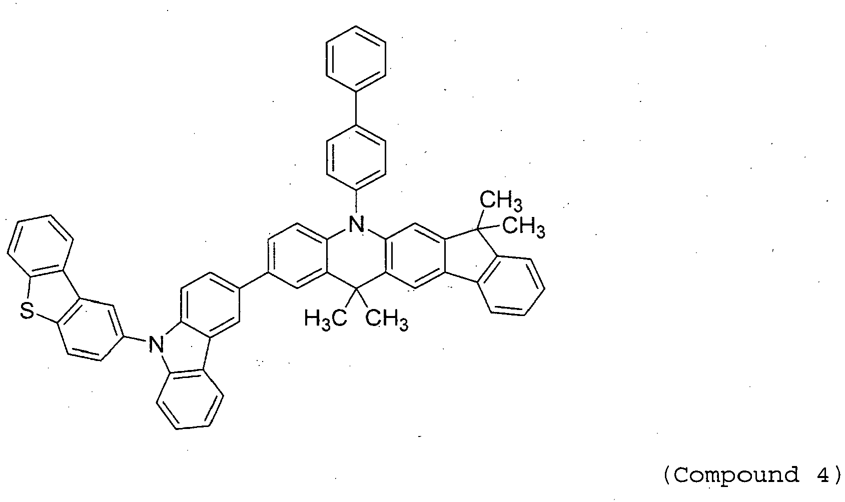

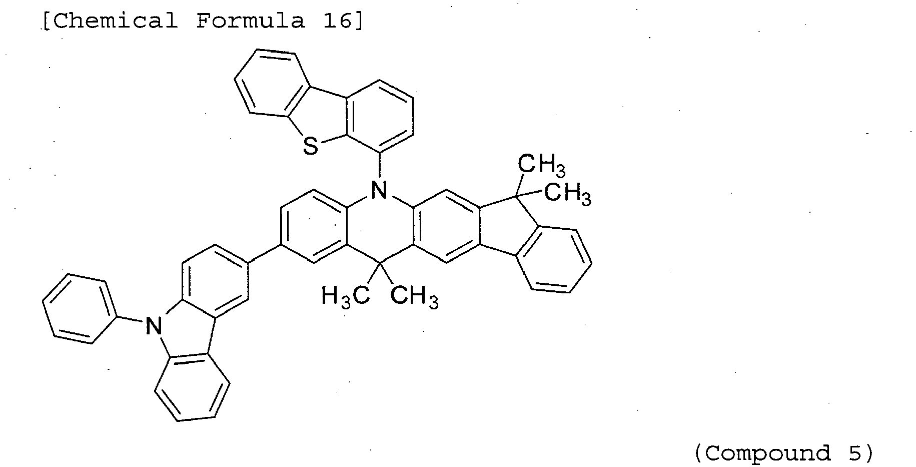

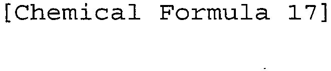

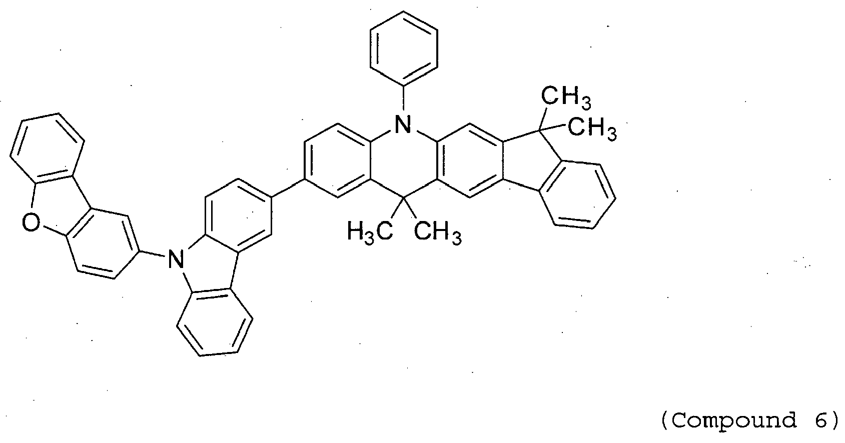

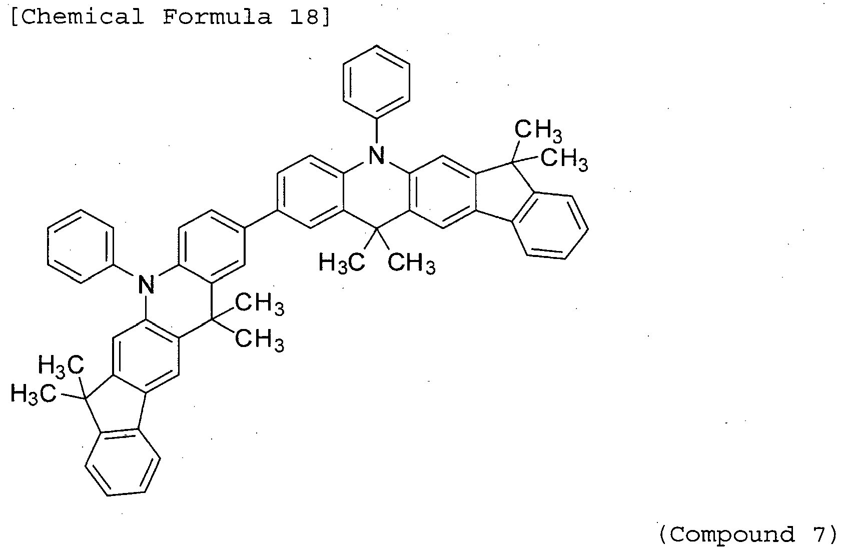

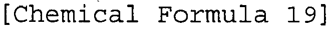

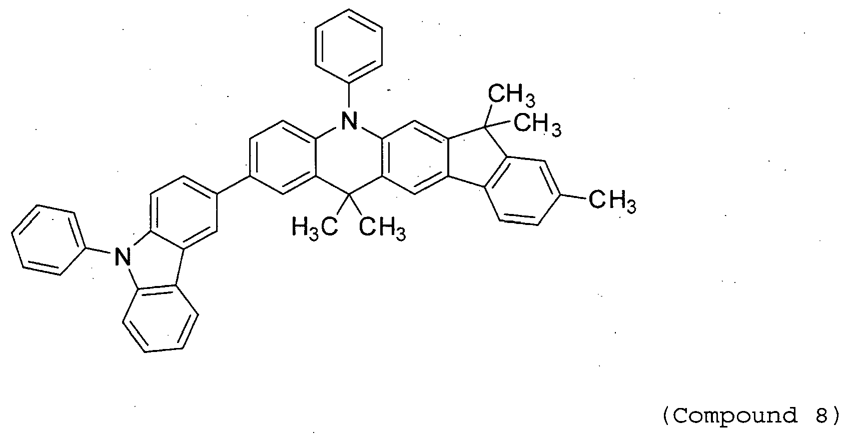

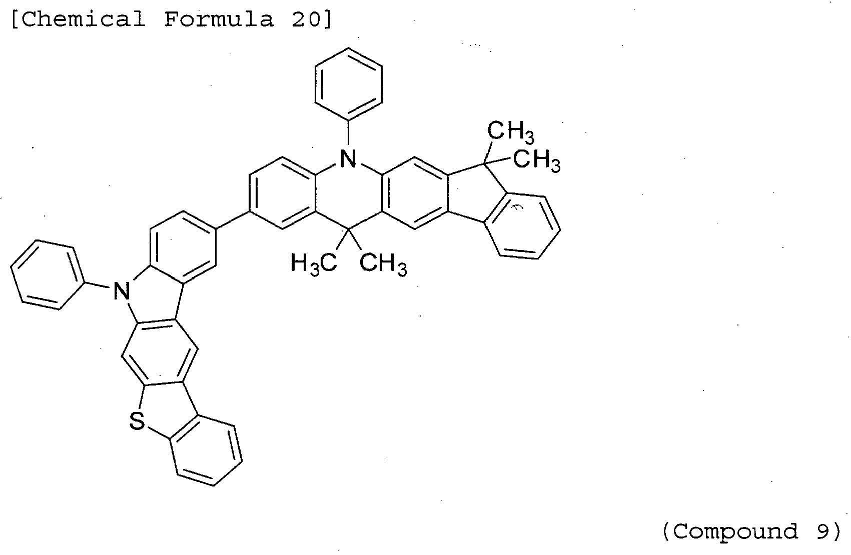

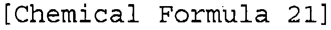

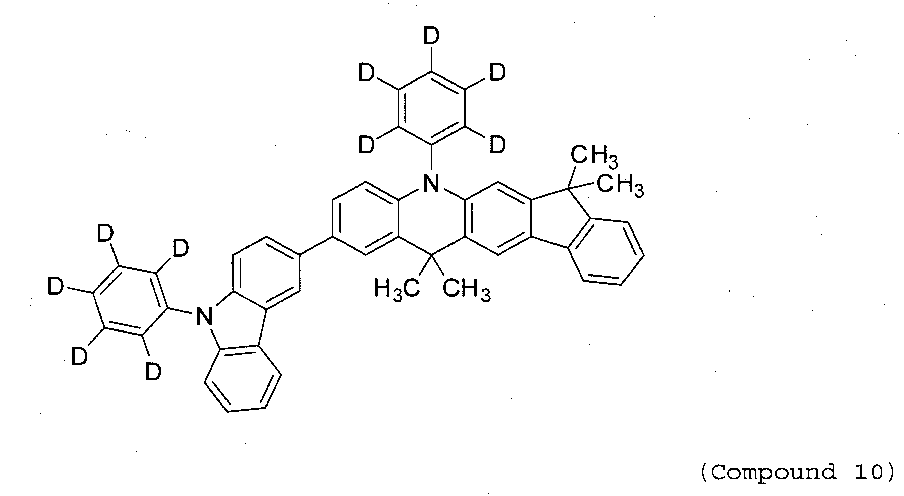

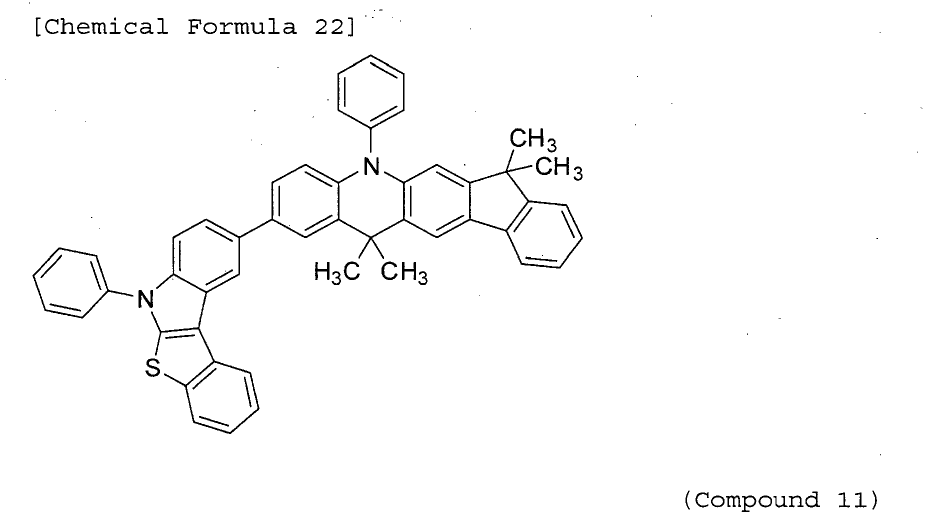

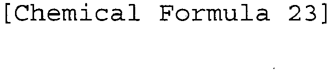

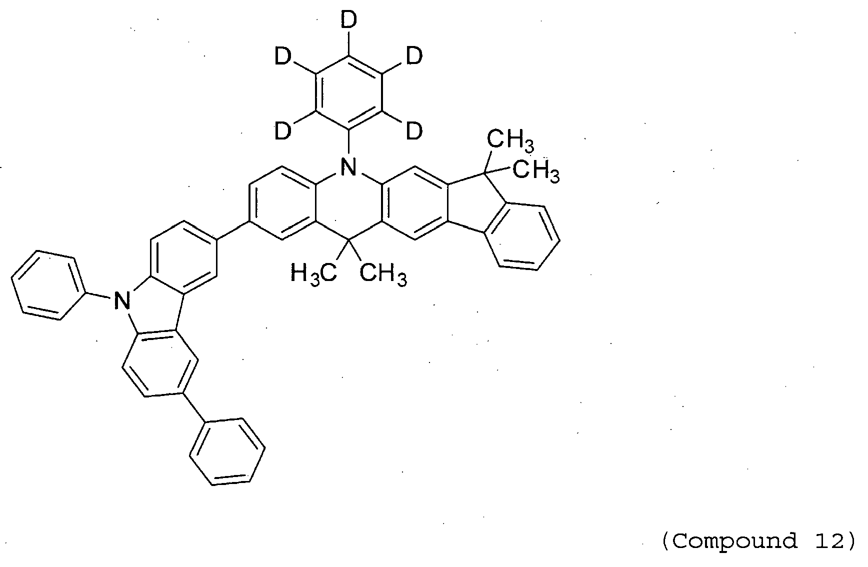

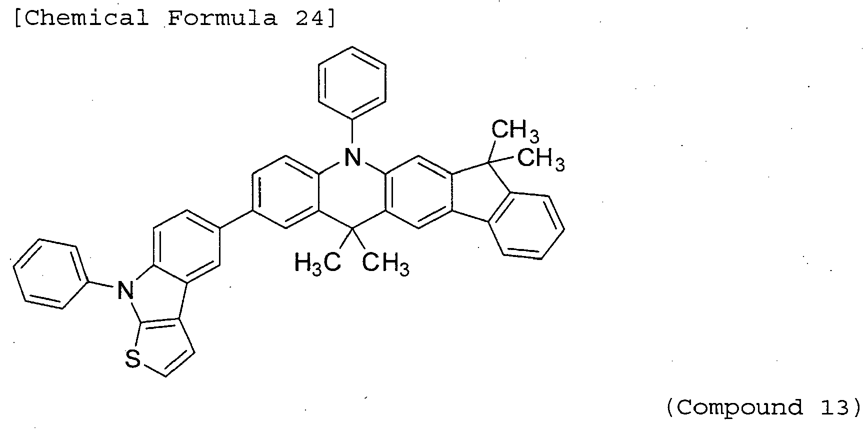

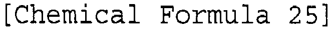

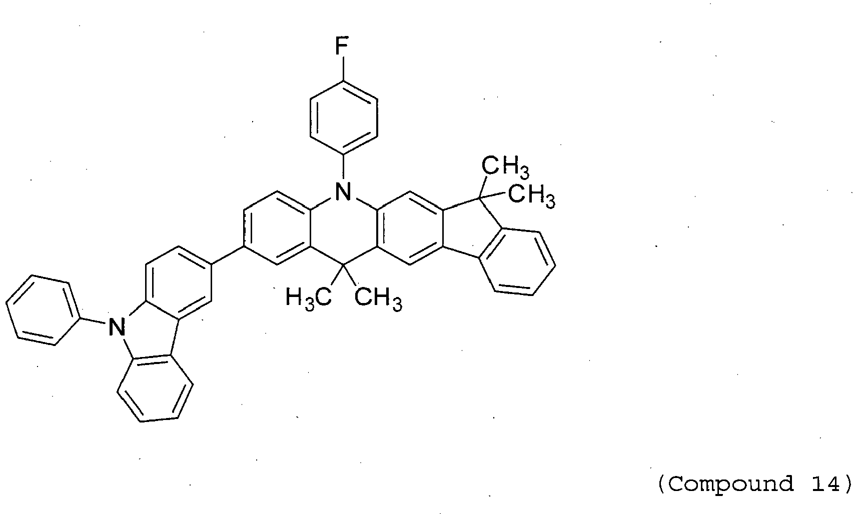

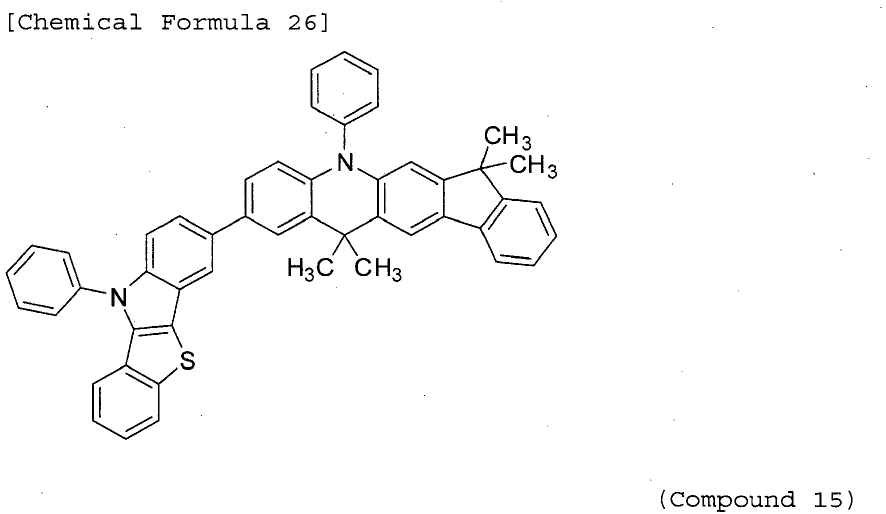

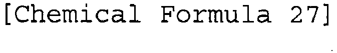

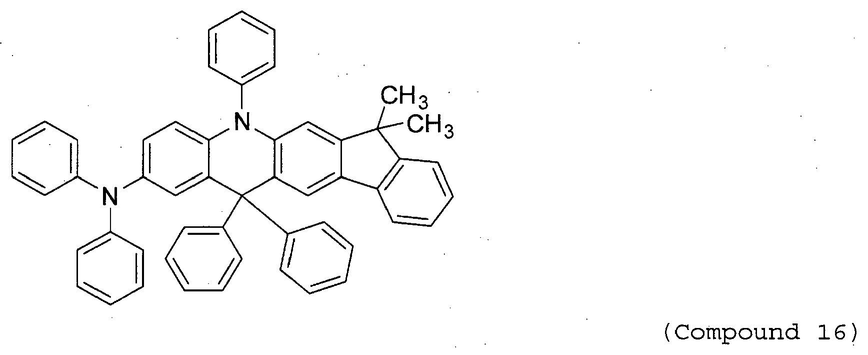

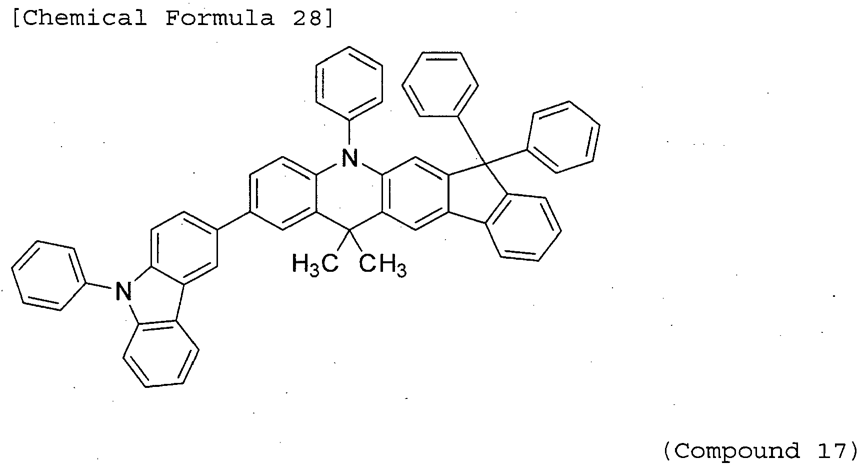

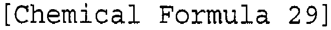

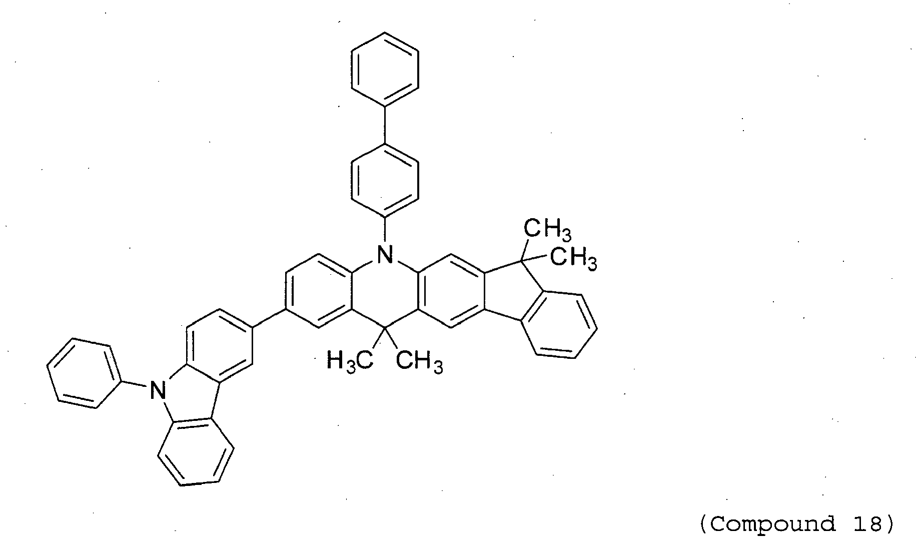

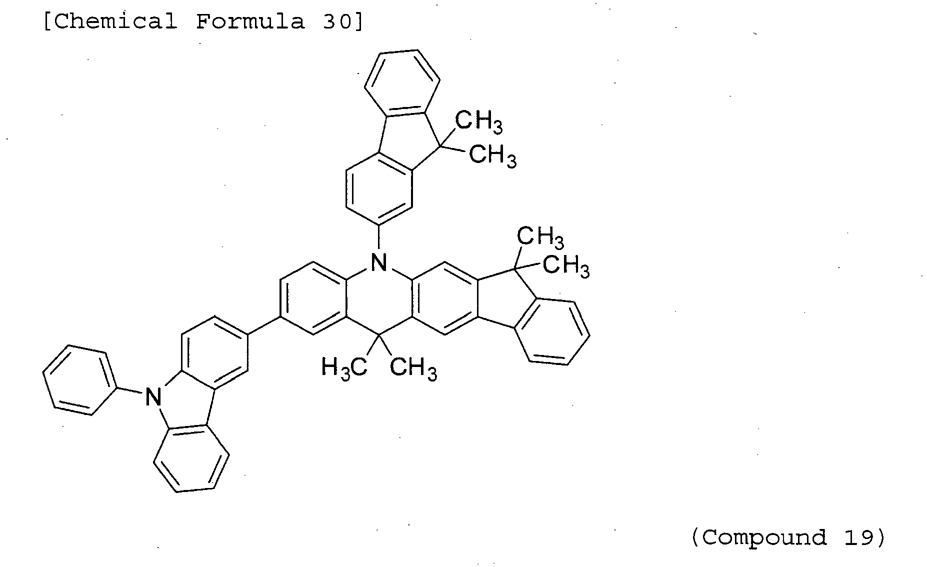

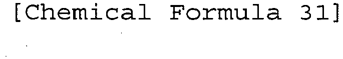

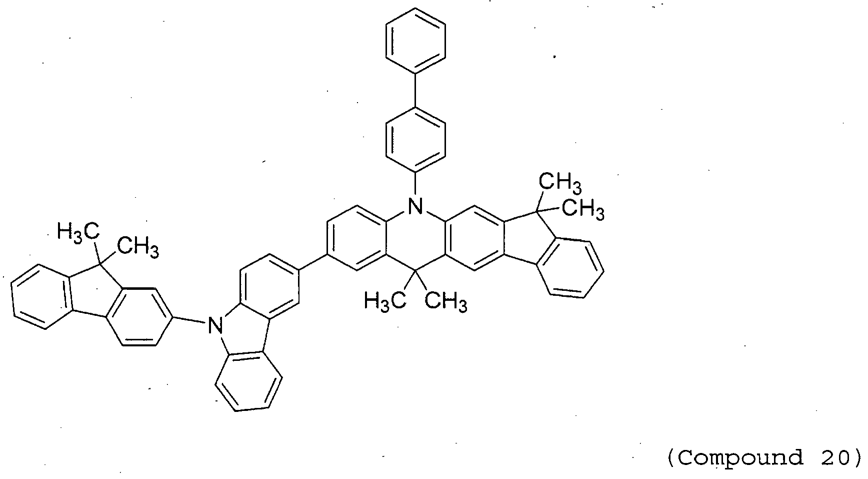

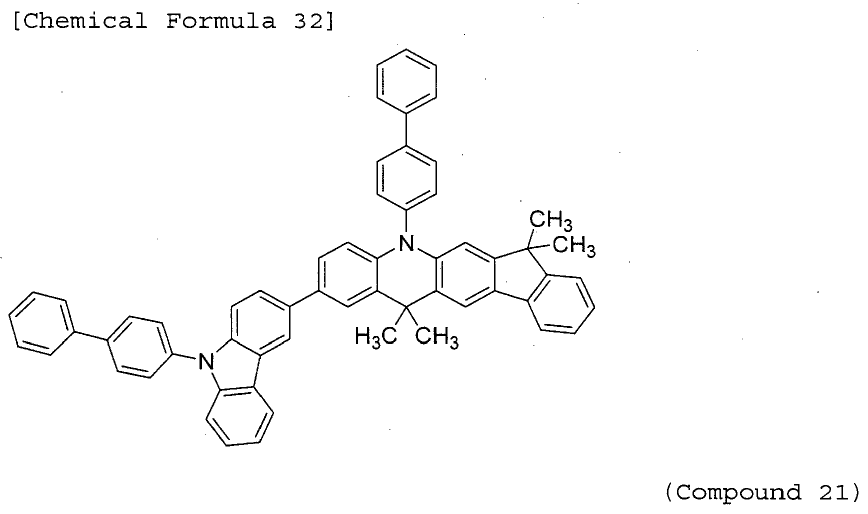

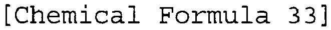

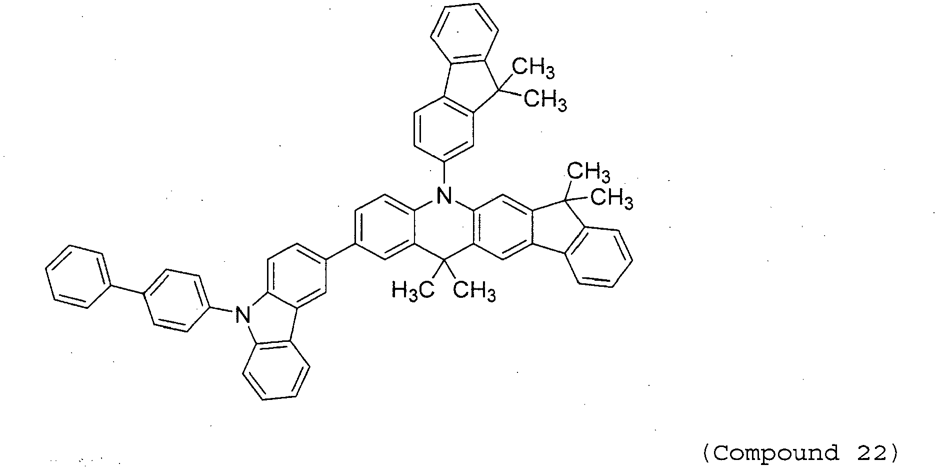

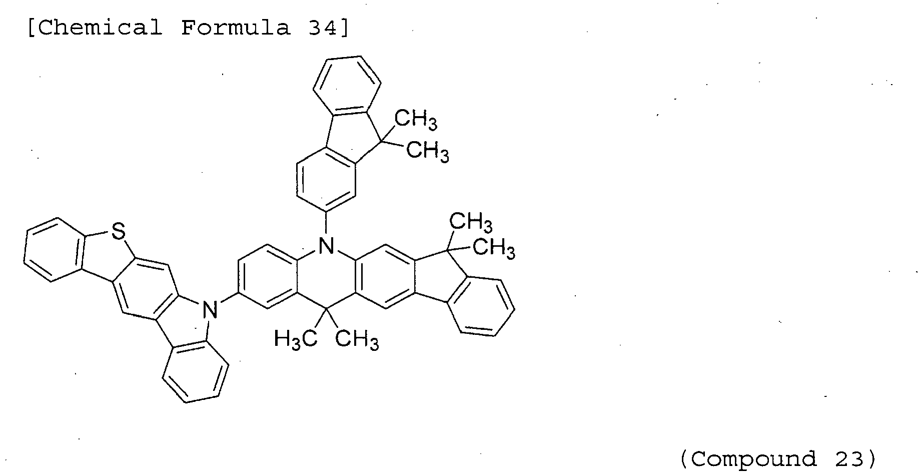

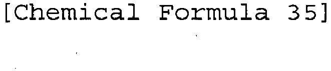

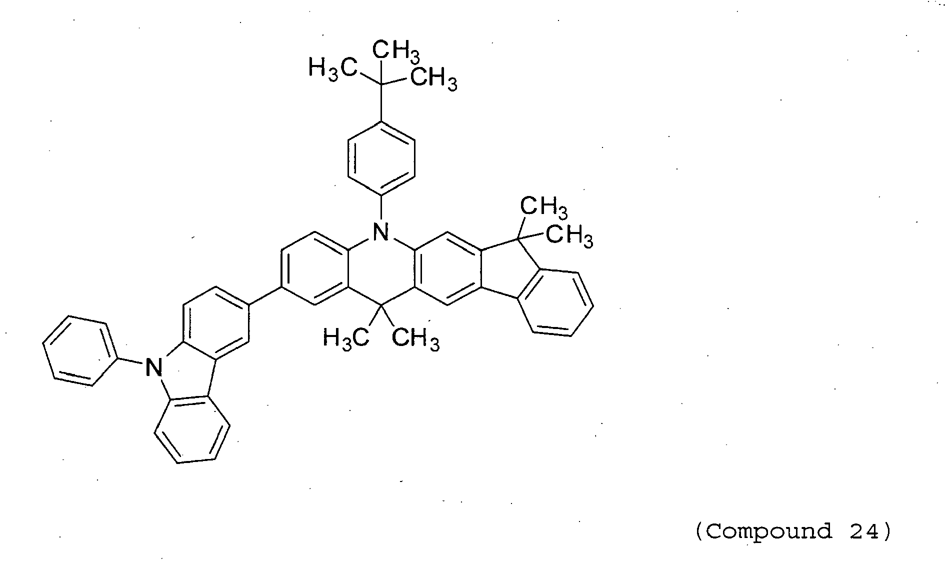

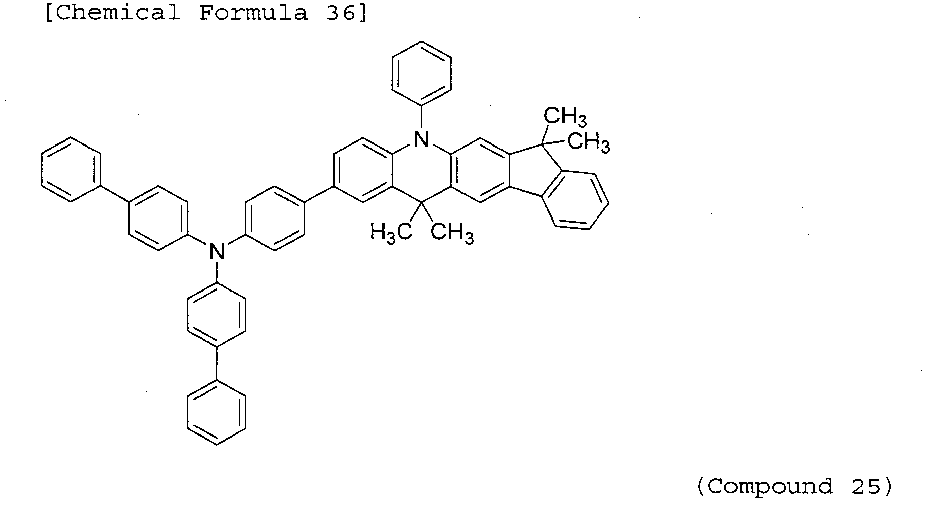

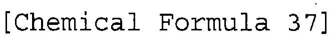

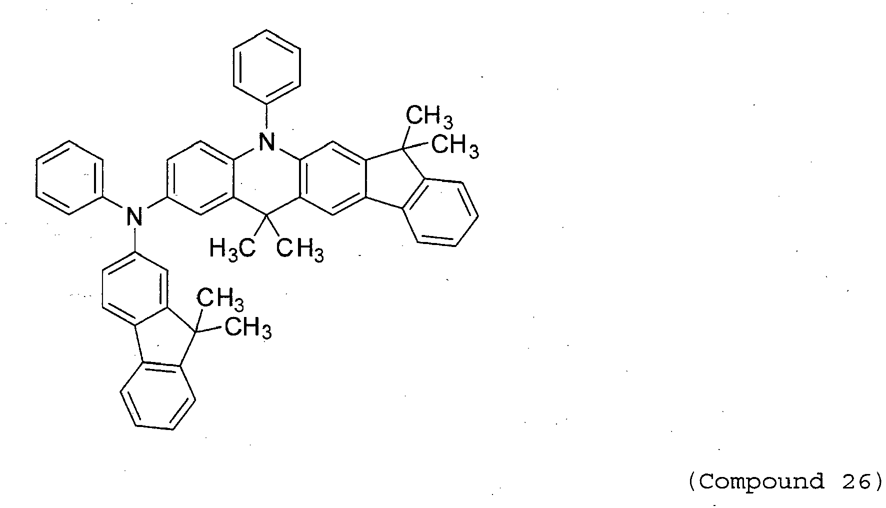

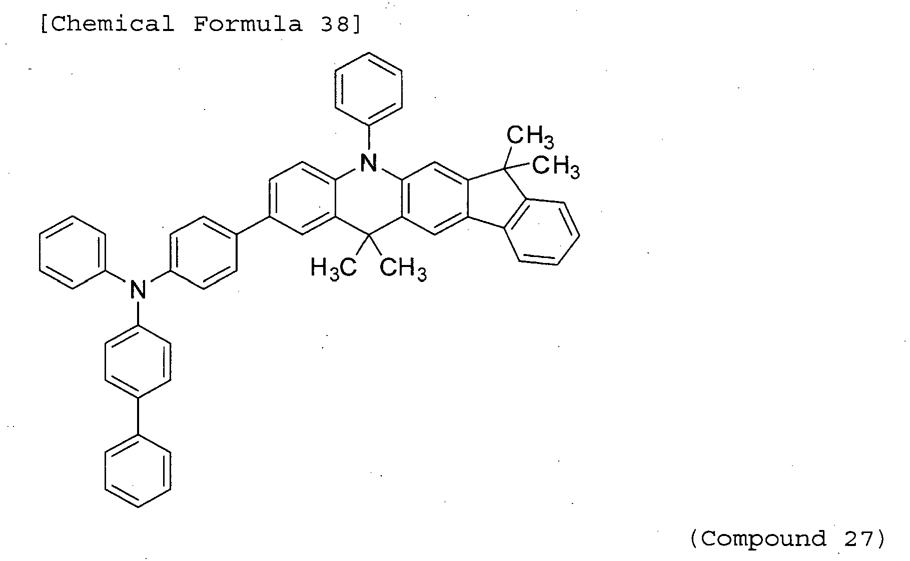

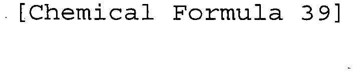

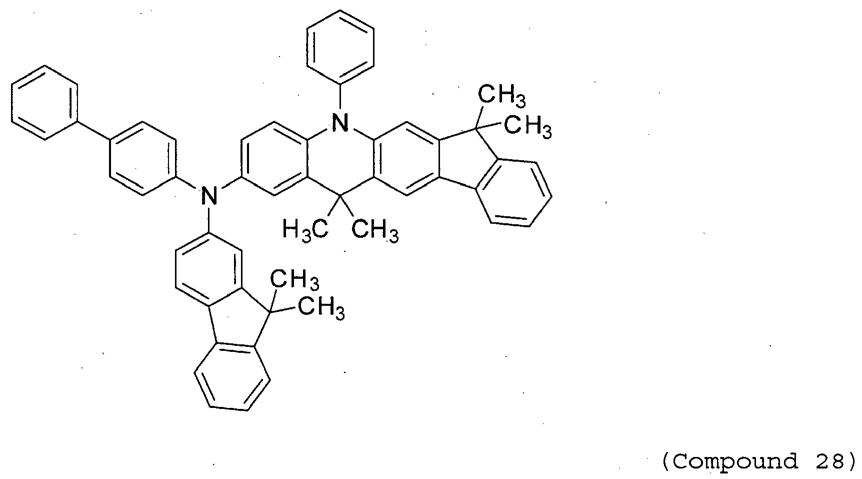

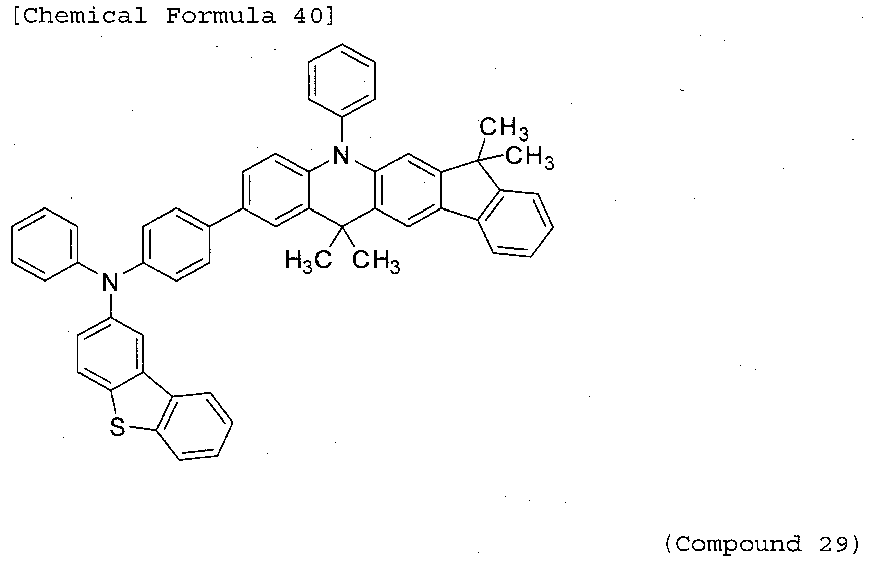

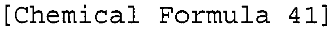

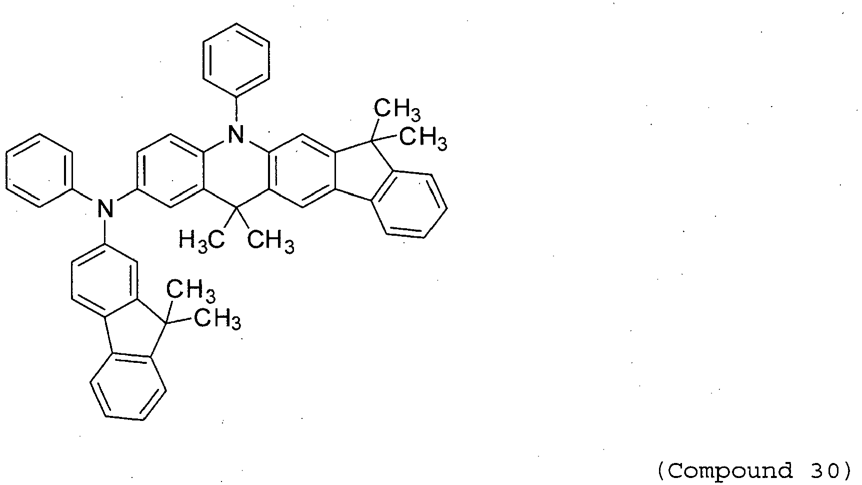

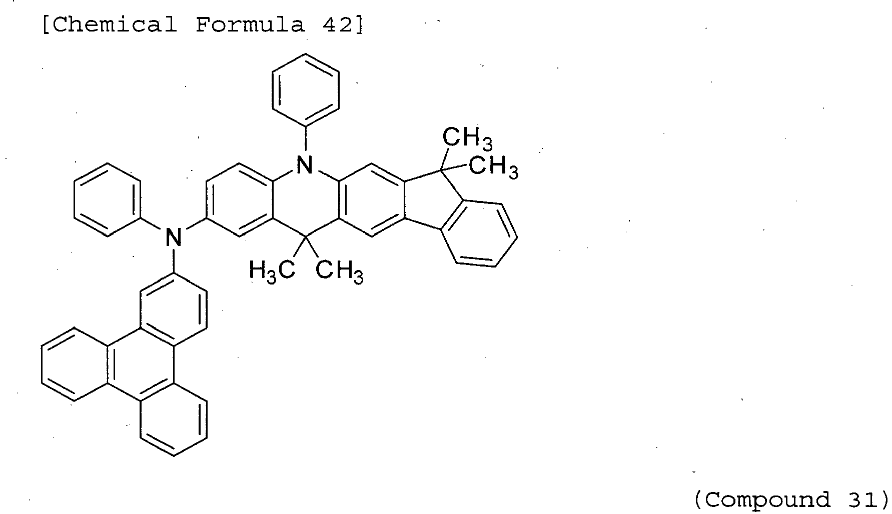

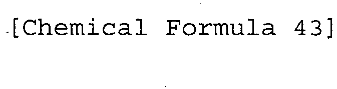

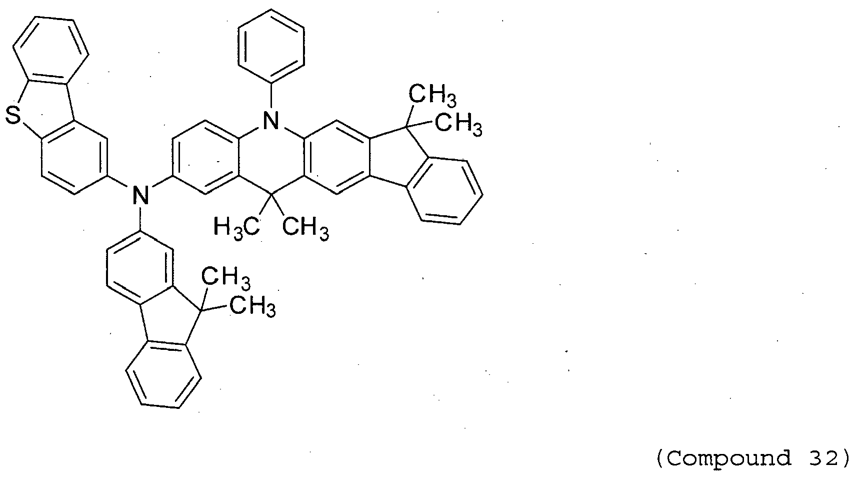

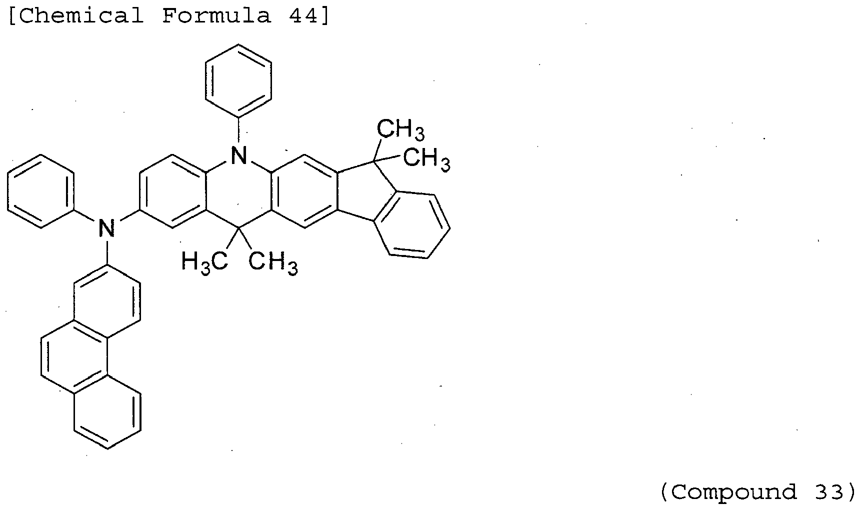

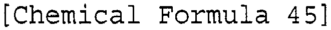

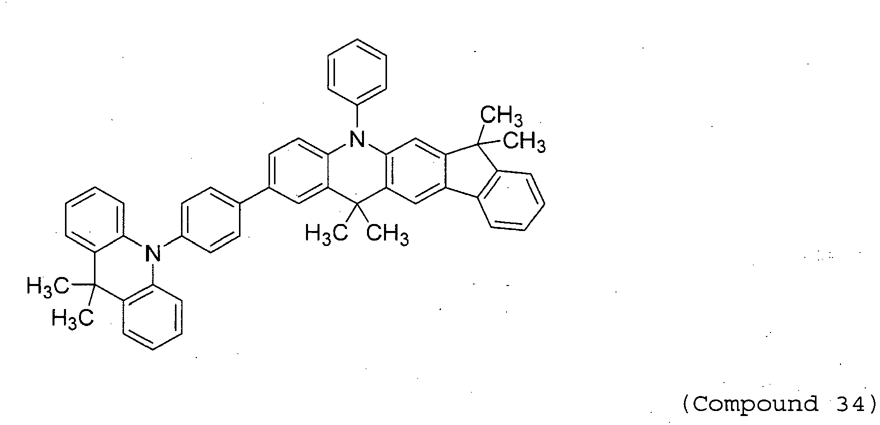

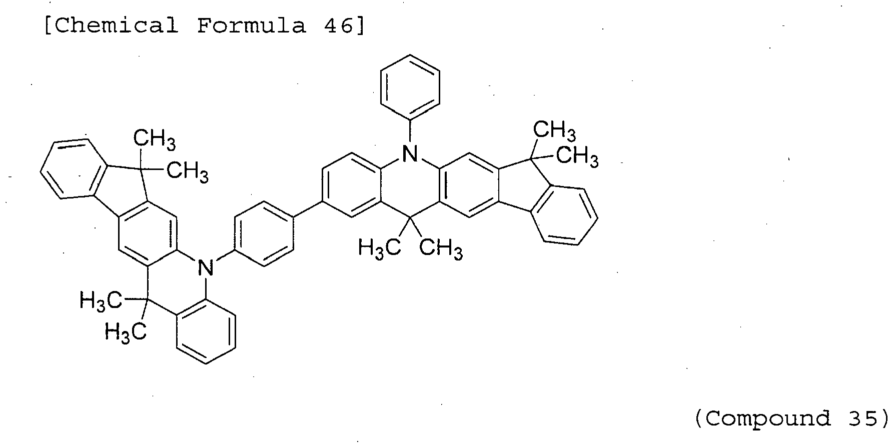

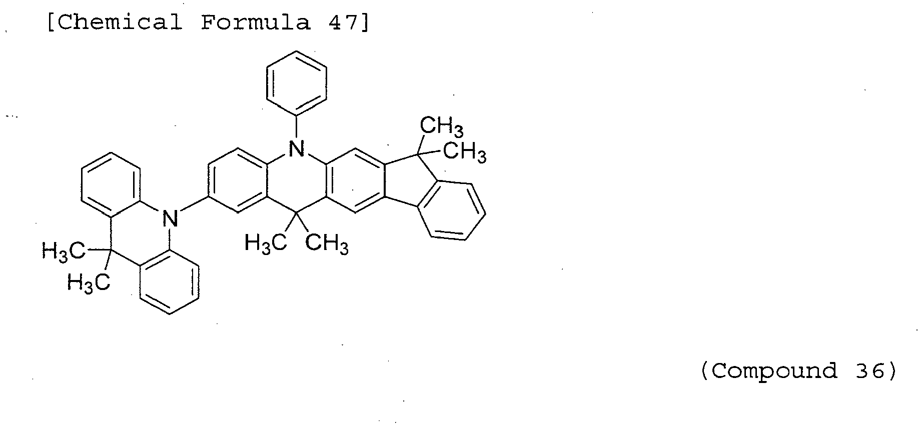

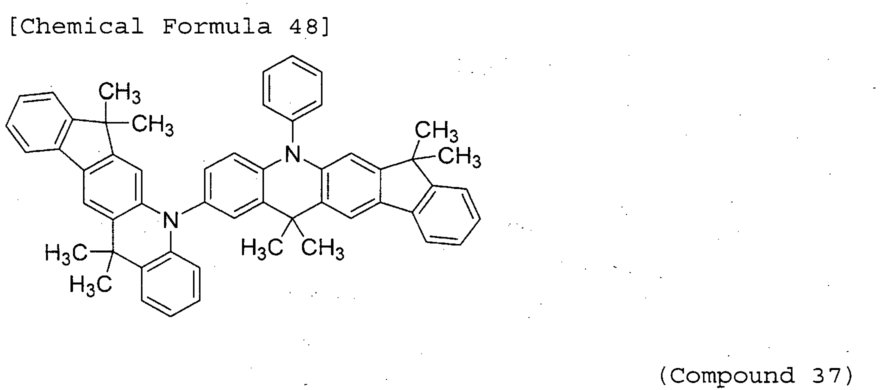

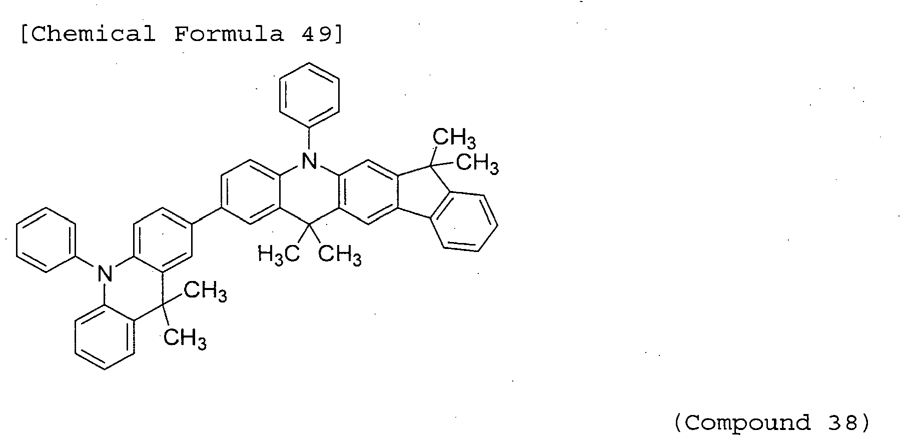

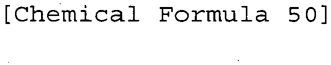

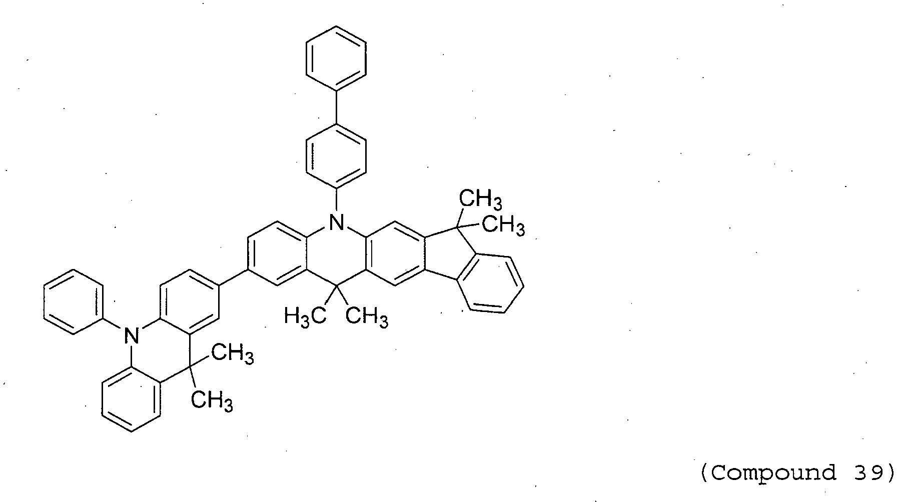

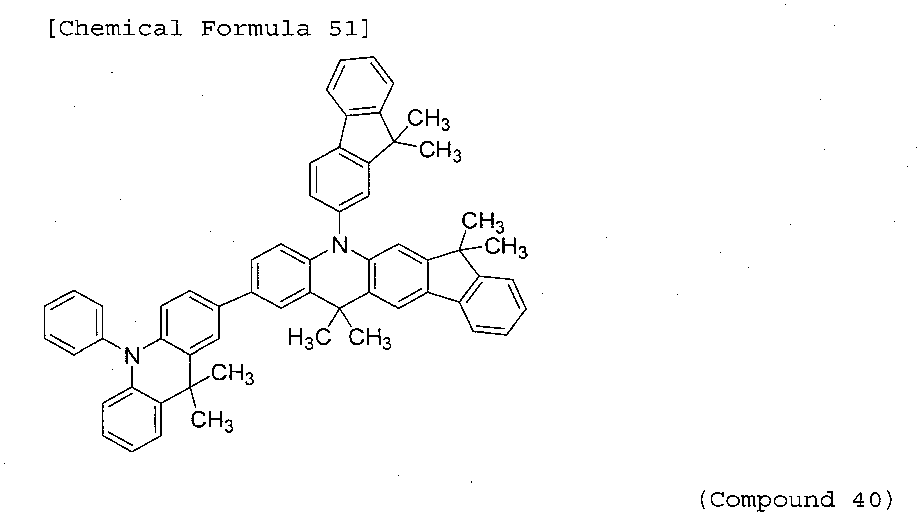

These compounds were purified by methods such as column chromatography, adsorption using, for example, silica gel, activated carbon, or activated clay, and recrystallization or crystallization using a solvent. The compounds were identified by an NMR analysis. A glass transition point (Tg) and a work function were measured as material property values. The glass transition point (Tg) can be used as an index of stability in the thin-film state, and the work function as an index of hole transportability.

The glass transition point (Tg) was measured by a high-sensitive differential scanning calorimeter (DSC3100SA produced by Bruker AXS) using powder.

For the measurement of the work function, a 100 nm-thick thin film was fabricated on an ITO substrate, and the ionization potential measurement device (PYS-202 produced by Sumitomo Heavy Industries, Ltd.) was used.

The organic EL device of the present invention may have a structure including an anode, a hole transport layer, an election blocking layer, a light emitting layer, an electron transport layer, and a cathode successively formed on a substrate, optionally with a hole injection layer between the anode and the hole transport layer, or with an electron injection layer between the electron transport layer and the cathode. In such multilayer structures, some of the organic layers may be omitted. For example, the device may be configured to include an anode, a hole transport layer, a light emitting layer, an electron transport layer, and a cathode successively formed on a substrate.

Electrode materials with high work functions such as ITO and gold are used as the anode of the organic EL device of the present invention. The hole injection layer of the organic EL device of the present invention may be made of material such as porphyrin compounds as represented by copper phthalocyanine, starburst-type triphenylamine derivatives, various triphenylamine tetramers, accepting heterocyclic compounds such as hexacyano azatriphenylene, and coating-type polymer materials, in addition to the compounds of general formula (1) having an indenoacridan ring structure of the present invention. These materials may be formed into a thin film by a vapor deposition method or other known methods such as a spin coating method and an inkjet method.

Examples of material used for the hole transport layer of the organic EL device of the present invention can be benzidine derivatives such as N,N'-diphenyl-N,N'-di(m-tolyl)benzidine (hereinafter referred to as TPD), N,N'-diphenyl-N,N'-di(a-naphthyl)benzidine (hereinafter referred to as NPD), and N,N,N',N'-tetrabiphenylylbenzidine; 1,1-bis[4-(di-4-tolylamino)phenyl]cyclohexane (hereinafter referred to as TAPC); and various triphenylamine trimers and tetramers, in addition to the compounds of general formula (1) having an indenoacridan ring structure of the present invention. These may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer. Examples of material used for the hole injection/transport layer can be coating-type polymer materials such as poly(3,4-ethylenedioxythiophene) (hereinafter referred to as PEDOT)/poly(styrene sulfonate) (hereinafter referred to as PSS). These materials may be formed into a thin-film by a vapor deposition method or other known methods such as a spin coating method and an inkjet method.

Further, material used for the hole injection layer or the hole transport layer may be obtained by p-doping trisbromophenylamine hexachloroantimony or the like into the material commonly used for these layers, or may be, for example, polymer compounds each having a TPD structure as a part of the compound structure.

Examples of material used for the electron blocking layer of the organic EL device of the present invention can be compounds having an electron blocking effect, including, for example, carbazole derivatives such as 4,4',4"-tri(N-carbazolyl)triphenylamine (hereinafter referred to as TCTA), 9,9-bis[4-(carbazol-9-yl)phenyl]fluorene, 1,3-bis(carbazol-9-yl)benzene (hereinafter referred to as mCP), and 2,2-bis(4-carbazol-9-ylphenyl)adamantane (hereinafter referred to as Ad-Cz); and compounds having a triphenylsilyl group and a triarylamine structure, as represented by 9-[4-(carbazol-9-yl)phenyl]-9-[4-(triphenylsilyl)phenyl]-9H-fluorene, in addition to the compounds of general formula (1) having an indenoacridan ring structure of the present invention. These may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer. These materials may be formed into a thin-film by using a vapor deposition method or other known methods such as a spin coating method and an inkjet method.

Examples of material used for the light emitting layer of the organic EL device of the present invention can be various metal complexes, anthracene derivatives, bis(styryl)benzene derivatives, pyrene derivatives, oxazole derivatives, and polyparaphenylene vinylene derivatives, in addition to quinolinol derivative metal complexes such as Alq3. Further, the light emitting layer may comprise a host material and a dopant material. Examples of the host material can be thiazole derivatives, benzimidazole derivatives, and polydialkyl fluorene derivatives, in addition to the above light-emitting materials and the compounds of general formula (1) having an indenoacridan ring structure of the present invention. Examples of the dopant material can be quinacridone, coumarin, rubrene, perylene, derivatives thereof, benzopyran derivatives, rhodamine derivatives, and aminostyryl derivatives. These may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer.

Further, the light-emitting material may be a phosphorescent light-emitting material. Phosphorescent materials as metal complexes of metals such as iridium and platinum may be used as the phosphorescent light-emitting material. Examples of the phosphorescent materials can be green phosphorescent materials such as Ir(ppy)3, blue phosphorescent materials such as FIrpic and FIr6, and red phosphorescent materials such as Btp2Ir(acac). As the hole injecting and transporting host material, the compounds of general formula (1) having an indenoacridan ring structure of the present invention may be used in addition to carbazole derivatives such as 4,4'-di (N-carbazolyl)biphenyl (hereinafter referred to as CBP), TCTA, and mCP. Compounds such as p-bis(triphenylsilyl)benzene (hereinafter referred to as UGH2) and 2,2',2"-(1,3,5-phenylene)-tris(1-phenyl-1H-benzimidazole) (hereinafter referred to as TPBI) may be used as the electron transporting host material to produce a high-performance organic EL device.

In order to avoid concentration quenching, it is preferable to dope the host material with the phosphorescent light-emitting material by co-evaporation in a range of 1 to 30 weight percent to the whole light emitting layer.

Further, Examples of the light-emitting material may be delayed fluorescent-emitting material such as CDCB derivatives of PIC-TRZ, CC2TA, PXZ-TRZ, 4CzIPN or the like (refer to Non-Patent Document 3, for example).

These materials may be formed into a thin-film by using a vapor deposition method or other known methods such as a spin coating method and an inkjet method.

The hole blocking layer of the organic EL device of the present invention may be formed by using hole blocking compounds such as various rare earth complexes, triazole derivatives, triazine derivatives, and oxadiazole derivatives, in addition to the metal complexes of phenanthroline derivatives such as bathocuproin (hereinafter referred to as BCP), and the metal complexes of quinolinol derivatives such as aluminum(III) bis(2-methyl-8-quinolinate)-4-phenylphenolate (hereinafter referred to as BAlq). These materials may also serve as the material of the electron transport layer. These may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer. These materials may be formed into a thin-film by using a vapor deposition method or other known methods such as a spin coating method and an inkjet method.

Examples of material used for the electron transport layer of the organic EL device of the present invention can be various metal complexes, triazole derivatives, triazine derivatives, oxadiazole derivatives, thiadiazole derivatives, carbodiimide derivatives, quinoxaline derivatives, phenanthroline derivatives, and silole derivatives, in addition to the metal complexes of quinolinol derivatives such as Alq3 and BAlq. These may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer. These materials may be formed into a thin-film by using a vapor deposition method or other known methods such as a spin coating method and an inkjet method.

Examples of material used for the electron injection layer of the organic EL device of the present invention can be alkali metal salts such as lithium fluoride and cesium fluoride; alkaline earth metal salts such as magnesium fluoride; and metal oxides such as aluminum oxide. However, the electron injection layer may be omitted in the preferred selection of the electron transport layer and the cathode.

The cathode of the organic EL device of the present invention may be made of an electrode material with a low work function such as aluminum, or an alloy of an electrode material with an even lower work function such as a magnesium-silver alloy, a magnesium-indium alloy, or an aluminum-magnesium alloy.

The following describes an embodiment of the present invention in more detail based on Examples. The present invention, however, is not restricted to the following Examples.

### Reference Example 1

### <Synthesis of 7,7,13,13-tetramethyl-5-phenyl-2-(9-phenyl-9H-carbazol-3-yl)-7,13-dihydro-5H-indeno[1,2-b]acridin (Compound 2)>

2-Amino methyl benzoate (35.4 g), 2-iodo-9,9-dimethyl-9H-fluoren (50.0 g), tert-butoxy sodium (22.51 g), and xylene (500 ml) were added to a nitrogen-substituted reaction vessel, and aerated with nitrogen gas for 1 hour. The mixture was heated after adding tris(dibenzylideneacetone)dipalladium(0) (2.9 g) and a toluene solution of tri (tert-butyl)phosphine (50% (w/v); 3.8g), and stirred at 115°C for 5 hours. After the mixture was allowed to cool to room temperature, water and toluene were added to perform liquid separation in order to collect an organic layer. The organic layer was dried over anhydrous magnesium sulfate, and concentrated under reduced pressure to obtain a crude product. The crude product was purified by column chromatography (support: silica gel, eluent: toluene/n-hexane) to obtain a yellow powder of 2-{(9,9-dimethyl-9H-fluoren-2-yl)amino}methyl benzoate (25.8 g; yield 48%).

The 2-{(9,9-dimethyl-9H-fluoren-2-yl)amino}methyl benzoate (31.0 g) and THF (310 ml) were added to a nitrogen-substituted reaction vessel, and a THF solution of methylmagnesium chloride(3mol/L; 108ml) was dropped. After the mixture was stirred at room temperature for 1 hour, an ammonium chloride aqueous solution (20%; 300 ml) was added and the mixture was extracted by adding toluene in order to collect an organic layer. The organic layer was dried over anhydrous magnesium sulfate, and concentrated under reduced pressure to obtain a light yellow oil of 2-[2-{(9,9-dimethyl-9H-fluoren-2-yl)amino}phenyl]propane-2-ol (31.0 g; yield 100%).

The 2-[2-{(9,9-dimethyl-9H-fluoren-2-yl)amino}phenyl]propane-2-ol (31.0 g) and phosphoric acid (62 ml) were added to a nitrogen-substituted reaction vessel, and stirred at room temperature for 2 hours. The mixture was stirred after adding toluene (300 ml) and water (300ml), and the precipitate was collected by filtration in order to obtain a light yellow powder of 7,7,13,13-tetramethyl-7,13-dihydro-5H⁻indeno[1,2-b]acridin (26.2 g; yield 89%).

The 7,7,13,13-tetramethyl-7,13-dihydro-5H-indeno[1,2-b]acridin (26.2 g), iodobenzene (17.2 g), tert-butoxy sodium (11.6 g), and xylene (260 ml) were added to a nitrogen-substituted reaction vessel, and aerated with nitrogen gas for 1 hour. The mixture was heated after adding tris(dibenzylideneacetone)dipalladium(0) (1.5 g) and a toluene solution of tri-tert-butylphosphine(50 % (w/v); 2.0 g), and stirred at 115°C for 2 hours. After the mixture was allowed to cool to room temperature, water (300 ml) was added and the mixture was extracted by adding toluene in order to collect an organic layer. The organic layer was dried over anhydrous magnesium sulfate, and concentrated under reduced pressure to obtain a crude product. The crude product was purified by column chromatography (support: silica gel, eluent: toluene/n-hexane) to obtain a white powder of 7,7,13,13-tetramethyl-5-phenyl-7,13-dihydro-5H-indeno[1,2-b]acridin (29.6 g; yield 92%).

The 7,7,13,13-tetramethyl-5-phenyl-7,13-dihydro-5H-indeno[1,2-b]acridin (24.4 g), N,N-dimethylformamide (490 ml), and N-succinimide (24.2 g) were added to a nitrogen-substituted reaction vessel, and stirred at 15°C for 2 hours while cooling. After the mixture was added to water, the precipitate was collected by filtration. Methanol was added to the precipitate, the mixture was stirred and filtrated to obtain a white powder of 2-bromo-7,7,13,13-tetramethyl-5-phenyl-7,13-dihydro-5H-indeno[1,2-b]acridin (28.5 g; yield 98%).

The 2-bromo-7,7,13,13-tetramethyl-5-phenyl-7,13-dihydro-5H-indeno[1,2-b]acridin (12.0 g), 9-phenyl-9H-carbazol-3-yl boronic acid (7.5 g), a 2 M potassium carbonate aqueous solution (37 ml), toluene (96 ml), and ethanol (24 ml) were added to a nitrogen-substituted reaction vessel, and aerated with nitrogen gas for 1 hour. The mixture was heated after adding tetrakis(triphenylphosphine)palladium (0.9 g), and stirred at 72°C for 2 hours. After the mixture was allowed to cool to room temperature, the mixture was extracted by adding toluene in order to collect an organic layer. The organic layer was dried over anhydrous magnesium sulfate, and concentrated under reduced pressure to obtain a crude product. The crude product was purified by column chromatography (support: silica gel, eluent: toluene/n-hexane) to obtain a white powder of 7,7,13,13-tetramethyl-5-phenyl-2-(9-phenyl-9H-carbazole-3-yl)-7,13-dihydro-5H-indeno[1,2-b]acridin (Compound 2; 10.2 g; yield 64%).

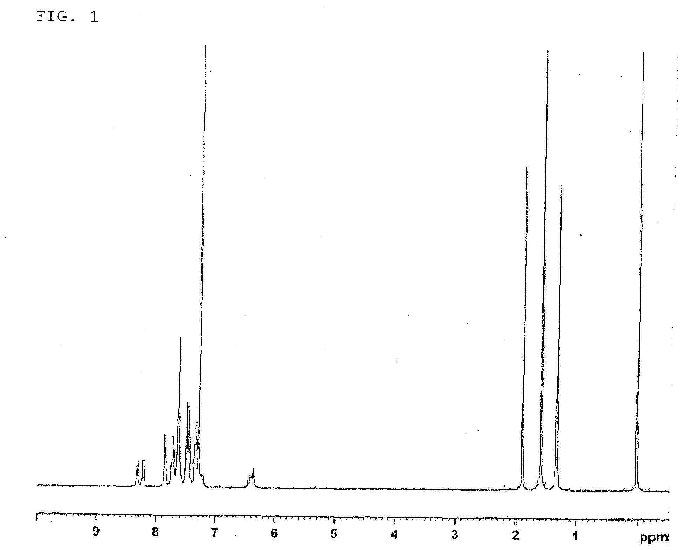

The structure of the product white powder was identified by NMR. The ¹H-NMR measurement result is shown in FIG. 1.
¹H-NMR (CDCl₃) detected 38 hydrogen signals, as follows. d (ppm) = 8.31 (1H), 8.22 (1H), 7.80-7.90 (2H), 7.56-7.80 (9H), 7.40-7.57 (6H), 7.18-7.40 (5H), 6.42 (1H), 6.38 (1H), 1.91 (6H), 1.35 (6H).

### Reference Example 2

### <Synthesis of 2-(3,3-dimethyl-1-phenyl-1,3-dihydroindeno[2,1-b]carbazole-10-yl)-7,7,13,13-tetramethyl-5-phenyl-7,13-dihydro-5H-indeno[1,2-b]acridin (Compound 3)>

2-Bromo-7,7,13,13-tetramethyl-5-phenyl-7,13-dihydro-5H-indeno[1,2-b]acridin synthesized in Example 1 (8.4 g), 3,3-dimethyl-1-phenyl-10-(4,4,5,5-tetramethyl-1,3,2-dioxaborolane-2-yl)-1,3-dihydroindeno[2,1-b]carbazole (8.9 g), a 2 M potassium carbonate aqueous solution (26 ml), toluene (67 ml), and ethanol (17 ml) were added to a nitrogen-substituted reaction vessel, and aerated with nitrogen gas for 1 hour. The mixture was heated after adding tetrakis(triphenylphosphine)palladium (0.6 g), and stirred at 72°C for 5 hours. After the mixture was allowed to cool to room temperature, the mixture was extracted by adding toluene in order to collect an organic layer. The organic layer was dried over anhydrous magnesium sulfate, and concentrated under reduced pressure to obtain a crude product. The crude product was purified by column chromatography (support: silica gel, eluent: methylene chloride/n-hexane) to obtain a white powder of 2-(3,3-dimethyl-1-phenyl-1,3-dihydroindeno[2,1-b]carbazole-10-yl)-7,7,13,13-tetramethyl-5-phenyl-7,13-dihydro-5H-indeno[1,2-b]acridin (8.6 g; yield 65%).

The structure of the product white powder was identified by NMR. The ¹H-NMR measurement result is shown in FIG. 1.
¹H-NMR (CDCl₃) detected 46 hydrogen signals, as follows. d (ppm) = 8.52 (1H), 8.39 (1H), 7.82-7.96 (3H), 7.28-7.80 (20H), 7.23 (1H), 6.30-6.50 (5H), 1.91 (6H), 1.55 (6H), 1.32 (6H).

### Example 3

### <Synthesis of 5-(biphenyl-4-yl)-7,7,13,13-tetramethyl-2-(9-phenyl-9H-carbazole-3-yl)-7,13-dihydro-5H-indeno[1,2-b]acridin (Compound 18)>

2-Bromo-7,7,13,13-tetramethyl-5-(biphenyl-4-yl)-7,13-dihydro-5H-indeno[1,2-b]acridin synthesized in the same manner as Example 1 (15.5 g), 9-phenyl-9H-carbazol-3-yl boronic acid (8.4 g), a 2 M potassium carbonate aqueous solution (42 ml), toluene (124 ml), and ethanol (31 ml) were added to a nitrogen-substituted reaction vessel, and aerated with nitrogen gas for 1 hour. The mixture was heated after adding tetrakis(triphenylphosphine)palladium (1.0 g), and stirred at 72°C for 4 hours. After the mixture was allowed to cool to room temperature, the mixture was extracted by adding toluene in order to collect an organic layer. The organic layer was dried over anhydrous magnesium sulfate, and concentrated under reduced pressure to obtain a crude product. The crude product was purified by column chromatography (support: silica gel, eluent: methylene chloride/n-hexane) to obtain a white powder of 5-(biphenyl-4-yl)-7,7,13,13-tetramethyl-2-(9-phenyl-9H-carbazole-3-yl)-7,13-dihydro-5H-indeno[1,2-b]acridin (5.6 g; yield 28%).

The structure of the product white powder was identified by NMR. The ¹H-NMR measurement result is shown in FIG. 1.
¹H-NMR (CDCl₃) detected 42 hydrogen signals, as follows. d (ppm) = 8.32 (1H), 8.22 (1H), 7.97 (2H), 7.78-7.90 (4H), 7.74 (1H), 7.42-7.70 (14H), 7.28-7.41 (4H), 7.23 (1H), 6.52 (1H), 6.48 (1H), 1.91 (6H), 1.35 (6H).

### Example 4

### <Synthesis of 5-(9,9-dimetyl-9H-fluoren-2-yl)-7,7,13,13-tetramethyl-2-(9-phenyl-9H-carbazole-3-yl)-7,13-dihydro-5H-indeno[1,2-b]acridin (Compound 19)>

2-Bromo-7,7,13,13-tetramethyl-5-(9,9-dimetyl-9H-fluoren-2-yl)-7,13-dihydro-5H-indeno[1,2-b]acridin synthesized in the same manner as Example 1 (12.0 g), 9-phenyl-9H-carbazol-3-yl boronic acid (6.1 g), a 2 M potassium carbonate aqueous solution (30 ml), toluene (96 ml), and ethanol (24 ml) were added to a nitrogen-substituted reaction vessel, and aerated with nitrogen gas for 1 hour. The mixture was heated after adding tetrakis(triphenylphosphine)palladium (0.7 g), and stirred at 72°C for 4 hours. After the mixture was allowed to cool to room temperature, the mixture was extracted by adding toluene in order to collect an organic layer. The organic layer was dried over anhydrous magnesium sulfate, and concentrated under reduced pressure to obtain a crude product. The crude product was purified by column chromatography (support: silica gel, eluent: toluene/n-hexane) to obtain a white powder of 5-(9,9-dimetyl-9H-fluoren-2-yl)-7,7,13,13-tetramethyl-2-(9-phenyl-9H-carbazole-3-yl)-7,13-dihydro-5H-indeno[1,2-b]acridin (12.0 g; yield 79%).

The structure of the product white powder was identified by NMR. The ¹H-NMR measurement result is shown in FIG. 1.
¹H-NMR (CDCl₃) detected 46 hydrogen signals, as follows. d (ppm) = 8.32 (1H), 8.22 (1H), 8.07 (1H), 7.84-7.91 (3H), 7.60-7.77 (6H), 7.28-7.60 (13H), 7.22 (1H), 6.55 (1H), 6.41 (1H), 1.91 (6H), 1.58 (6H), 1.30 (6H).

### Example 5

The glass transition point of the compounds of the present invention were determined using a high-sensitive differential scanning calorimeter (DSC 3100SA produced by Bruker AXS).

| | Glass transition point |
|---|---|
| Compound of Reference Example 1 | 140°C |
| Compound of Reference Example 2 | 187°C |
| Compound of Example 3 of the present invention | 155°C |
| Compound of Example 4 of the present invention | 164°C |

The compounds of the present invention have glass transition points of 100°C or higher, demonstrating that the compounds of the present invention have a stable thin-film state.

### Example 6

A 100 nm-thick vapor-deposited film was fabricated on an ITO substrate using the compounds of the present invention, and a work function was measured using an ionization potential measurement device (PYS-202 produced by Sumitomo Heavy Industries, Ltd.).

| | Work function |
|---|---|
| Compound of Reference Example 1 | 5.48 eV |
| Compound of Reference Example 2 | 5.50 eV |
| Compound of Example 3 of the present invention | 5.45 eV |
| Compound of Example 4 of the present invention | 5.41 eV |

As the results show, the compounds of the present invention have desirable energy levels compared to the work function 5.54 eV of common hole transport materials such as NPD and TPD, and thus possess desirable hole transportability.

### Reference Example 7

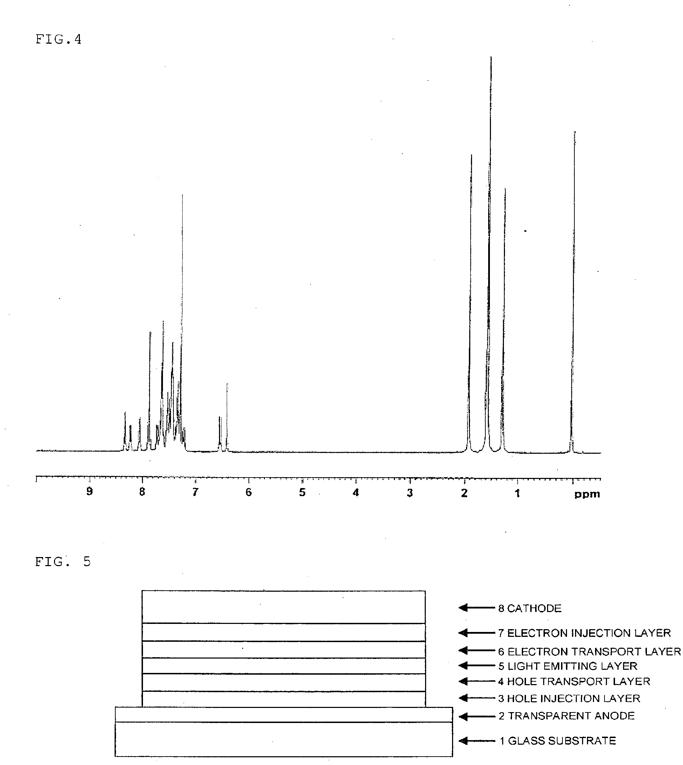

The organic EL device, as illustrated in FIG. 5, was fabricated from a hole injection layer 3, a hole transport layer 4, a light emitting layer 5, an electron transport layer 6, an electron injection layer 7, and a cathode (aluminum electrode) 8 successively formed by vapor deposition on a glass substrate 1 that had been provided beforehand with an ITO electrode as a transparent anode 2.

Specifically, the glass substrate 1 having ITO (thickness 150 nm) formed thereon was washed with an organic solvent, and subjected to an oxygen plasma treatment to wash the surface. The glass substrate with the ITO electrode was then installed in a vacuum vapor deposition apparatus, and the pressure was reduced to 0.001 Pa or less. This was followed by formation of the hole injection layer 3 by forming Compound 41 of the structural formula below over the transparent anode 2 in a thickness of 20 nm. The hole transport layer 4 was then formed on the hole injection layer 3 by forming the compound of Example 1 of the present invention (Compound 2) in a thickness of 40 nm. Then, the light emitting layer 5 was formed on the hole transport layer 4 in a thickness of 30 nm by the dual vapor deposition of Compound 42 of the structural formula below and Compound 43 of the structural formula below at a deposition rate ratio of Compound 42: Compound 43 = 5:95. The electron transport layer 6 was then formed on the light emitting layer 5 by forming Alq3 in a thickness of 30 nm. Then, the electron injection layer 7 was formed on the electron transport layer 6 by forming lithium fluoride in a thickness of 0.5 nm. Finally, the cathode 8 was formed by vapor depositing aluminum in a thickness of 150 nm. The characteristics of the organic EL device thus fabricated were measured in an atmosphere at ordinary temperature.

Table 1 summarizes the results of the emission characteristics measurements performed by applying a DC voltage to the organic EL device fabricated with the compound of Reference Example 1 (Compound 2).

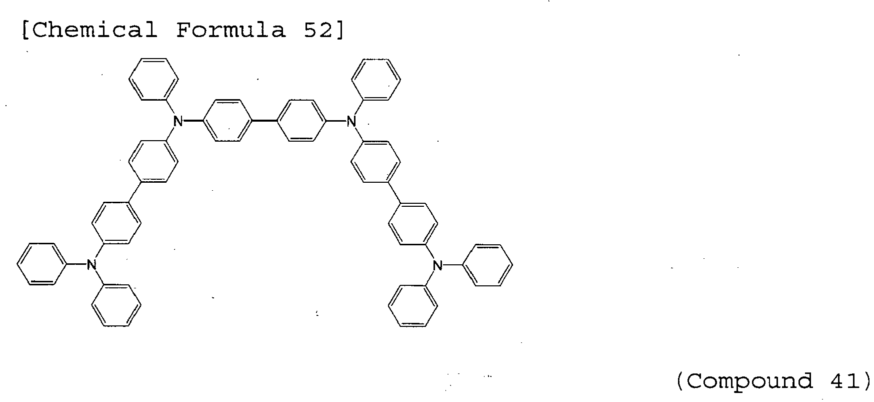

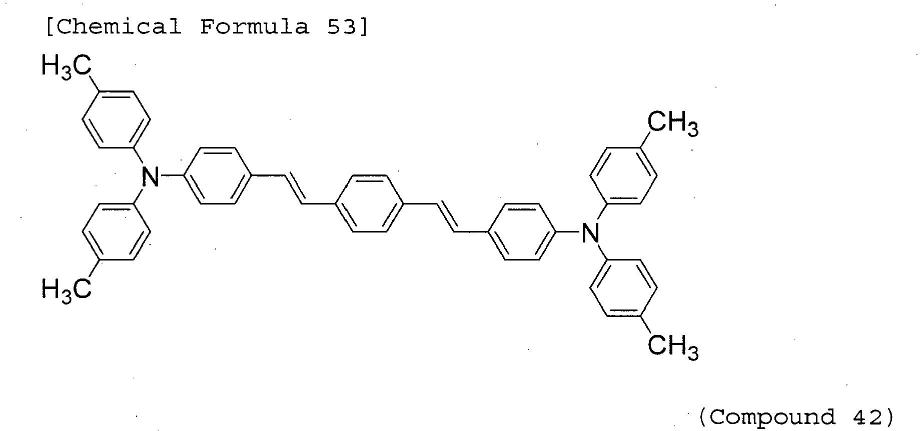

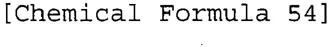

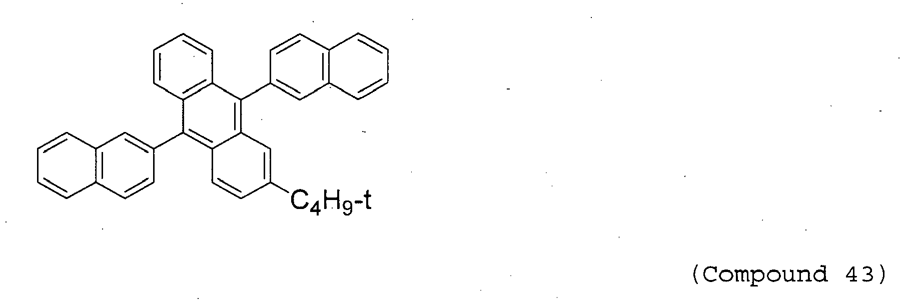

### Reference Example 8

An organic EL device was fabricated under the same conditions used in Reference Example 7, except that the hole transport layer 4 was formed by forming the compound of Reference Example 2 (Compound 3) in a thickness of 40 nm, instead of using the compound of Reference Example 1 (Compound 2). The characteristics of the organic EL device thus fabricated were measured in an atmosphere at ordinary temperature. Table 1 summarizes the results of the emission characteristics measurements performed by applying a DC voltage to the fablicated organic EL device.

### Example 9

An organic EL device was fabricated under the same conditions used in Example 7, except that the hole transport layer 4 was formed by forming the compound of Example 3 of the present invention (Compound 18) in a thickness of 40 nm, instead of using the compound of Reference Example 1 (Compound 2). The characteristics of the organic EL device thus fabricated were measured in an atmosphere at ordinary temperature. Table 1 summarizes the results of the emission characteristics measurements performed by applying a DC voltage to the fablicated organic EL device.

### Example 10

An organic EL device was fabricated under the same conditions used in Example 7, except that the hole transport layer 4 was formed by forming the compound of Example 4 of the present invention (Compound 19) in a thickness of 40 nm, instead of using the compound of Reference Example 1 (Compound 2). The characteristics of the organic EL device thus fabricated were measured in an atmosphere at ordinary temperature. Table 1 summarizes the results of the emission characteristics measurements performed by applying a DC voltage to the fablicated organic EL device.

### Comparative Example 1

For comparison, an organic EL device was fabricated under the same conditions used in Example 7, except that the hole transport layer 4 was formed by forming Compound 44 of the structural formula below in a thickness of 40 nm, instead of using the compound of Reference Example 1 (Compound 2). The characteristics of the organic EL device thus fabricated were measured in an atmosphere at ordinary temperature. Table 1 summarizes the results of the emission characteristics measurements performed by applying a DC voltage to the fablicated organic EL device.

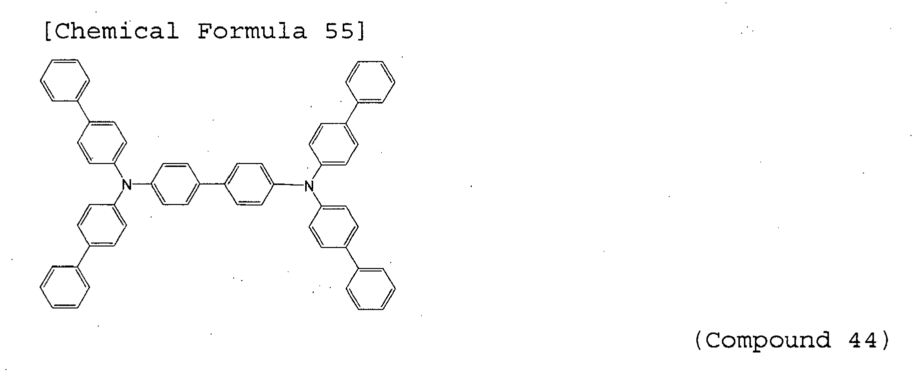

**[Table 1]**

| | Compound | Voltage [V] (@10mA/cm²) | Luminance [cd/m²] (@10mA/cm²) | Current efficiency [cd/A] (@10mA/cm²) | Power efficiency [lm/W] (@10mA/cm²) |
|---|---|---|---|---|---|
| Ref. Ex. 7 | Compound 2 | 5.03 | 983 | 9.84 | 6.15 |
| Ref. Ex. 8 | Compound 3 | 5.07 | 1031 | 10.31 | 6.39 |
| Ex. 9 | Compound 18 | 4.97 | 962 | 9.62 | 6.09 |
| Ex. 10 | Compound 19 | 4.89 | 1002 | 10.03 | 6.45 |
| Comp. Ex. 1 | Compound 44 | 5.17 | 902 | 9.03 | 5.49 |

As can be seen in Table 1, the driving voltage at the current density of 10 mA/cm² was 4.89 V to 5.07 V for the organic EL device that uses the compounds 2, 3, 18, and 19 of Examples 1 to 4, which was lower than 5.17 V for the organic EL device that uses the compound 44. Further, the power efficiency was 6.09 to 6.45 lm/W for the organic EL device that uses the compounds 2, 3, 18, and 19 of Examples 1 to 4, and there was a great improvement in the organic EL device that uses the compound 44 as shown in its power efficiency 5.49 lm/W.

As is clear from these results, the organic EL devices using the compounds having an indenoacridan ring structure of the present invention can greatly improve power efficiency, and can achieve a lower actual driving voltage compared to the organic EL device that uses known Compound 44.

The compounds having an indenoacridan ring structure of the present invention have high hole transportability, excel in electron blocking ability and amorphousness, and have a stable thin-film state. The compounds are therefore excellent as the compounds for organic EL devices. The organic EL devices fabricated with the compounds can have high luminous efficiency and high power efficiency and can have a low actual driving voltage to improve durability. There are potential applications for, for example, home electric appliances and illuminations.

### Description of Reference Numeral

- 1: Glass substrate
- 2: Transparent anode
- 3: Hole injection layer
- 4: Hole transport layer
- 5: Light emitting layer
- 6: Electron transport layer
- 7: Electron injection layer
- 8: Cathode

## Claims

1. A compound of the following general formula (1) having an indenoacridan ring structure, 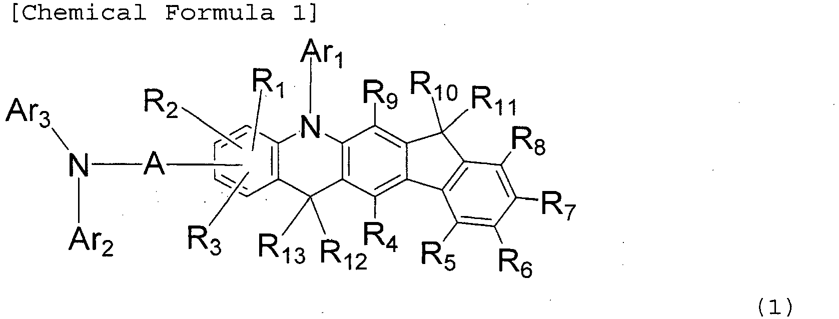
wherein A represents a divalent group of a substituted or unsubstituted aromatic hydrocarbon, a divalent group of a substituted or unsubstituted aromatic heterocyclic ring, or a divalent group of substituted or unsubstituted condensed polycyclic aromatics; Ar₂, and Ar₃ may be the same or different, and represent a substituted or unsubstituted aromatic hydrocarbon group, a substituted or unsubstituted aromatic heterocyclic group, or a substituted or unsubstituted condensed polycyclic aromatic group, and Ar₁ is a substituted or unsubstituted biphenyl group, a substituted or unsubstituted terphenyl group, a substituted or unsubstituted fluorenyl group, and where Ar₂ and Ar₃ may bind to each other via a single bond, substituted or unsubstituted methylene, an oxygen atom, or a sulfur atom to form a ring; R₁ to R₉ may be the same or different, and represent a hydrogen atom, a deuterium atom, a fluorine atom, a chlorine atom, cyano, nitro, linear or branched alkyl of 1 to 6 carbon atoms that may have a substituent, cycloalkyl of 5 to 10 carbon atoms that may have a substituent, linear or branched alkenyl of 2 to 6 carbon atoms that may have a substituent, linear or branched alkyloxy of 1 to 6 carbon atoms that may have a substituent, cycloalkyloxy of 5 to 10 carbon atoms that may have a substituent, a substituted or unsubstituted aromatic hydrocarbon group, a substituted or unsubstituted aromatic heterocyclic group, a substituted or unsubstituted condensed polycyclic aromatic group, or substituted or unsubstituted aryloxy, which may bind to each other via a single bond, substituted or unsubstituted methylene, an oxygen atom, or a sulfur atom to form a ring; R₁₀ to R₁₃ may be the same or different, and represent linear or branched alkyl of 1 to 6 carbon atoms that may have a substituent, cycloalkyl of 5 to 10 carbon atoms that may have a substituent, linear or branched alkenyl of 2 to 6 carbon atoms that may have a substituent, linear or branched alkyloxy of 1 to 6 carbon atoms that may have a substituent, cycloalkyloxy of 5 to 10 carbon atoms that may have a substituent, a substituted or unsubstituted aromatic hydrocarbon group, a substituted or unsubstituted aromatic heterocyclic group, a substituted or unsubstituted condensed polycyclic aromatic group, or substituted or unsubstituted aryloxy, where R₁₀ and R₁₁, or R₁₂ and R₁₃ may bind to each other via a single bond, substituted or unsubstituted methylene, an oxygen atom, or a sulfur atom to form a ring; and when A is a divalent group of a substituted or unsubstituted aromatic hydrocarbon, a divalent group of a substituted or unsubstituted aromatic heterocyclic ring, or a divalent group of substituted or unsubstituted condensed polycyclic aromatics, A and Ar₂ bind to each other via a single bond, substituted or unsubstituted methylene, an oxygen atom, or a sulfur atom to form a ring, and
wherein the substituent of the group represented by Ar₁ in general formula (1) is selected from a deuterium atom; halogen atoms such as a fluorine atom, a chlorine atom, a bromine atom, and an iodine atom; linear or branched alkyl of 1 to 6 carbon atoms such as methyl, ethyl, n-propyl, isopropyl, n-butyl, isobutyl, tert-butyl, n-pentyl, isopentyl, neopentyl, and n-hexyl; linear or branched alkyloxy of 1 to 6 carbon atoms such as methyloxy, ethyloxy, and propyloxy; alkenyls such as allyl; aryloxys such as phenyloxy and tolyloxy; arylalkyloxys such as benzyloxy and phenethyloxy; aromatic hydrocarbon groups or condensed polycyclic aromatic groups such as phenyl, biphenylyl, terphenylyl, naphthyl, anthracenyl, phenanthryl, fluorenyl, indenyl, pyrenyl, perylenyl, fluoranthenyl, and triphenylenyl; aromatic heterocyclic groups such as pyridyl, thienyl, furyl, pyrrolyl, quinolyl, isoquinolyl, benzofuranyl, benzothienyl, indolyl, carbazolyl, benzooxazolyl, benzothiazolyl, quinoxalyl, benzoimidazolyl, pyrazolyl, dibenzofuranyl, dibenzothienyl, and carbolinyl; arylvinyls such as styryl and naphthylvinyl; and acyls such as acetyl and benzoyl, wherein the substituents may be further substituted with the substituents above, and the substituents may bind to each other, or to Ar₁ via a single bond, substituted or unsubstituted methylene, an oxygen atom, or a sulfur atom to form a ring.

2. The compound having an indenoacridan ring structure according to claim 1, wherein the compound is represented by the following general formula (1-1).

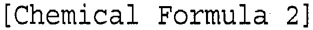

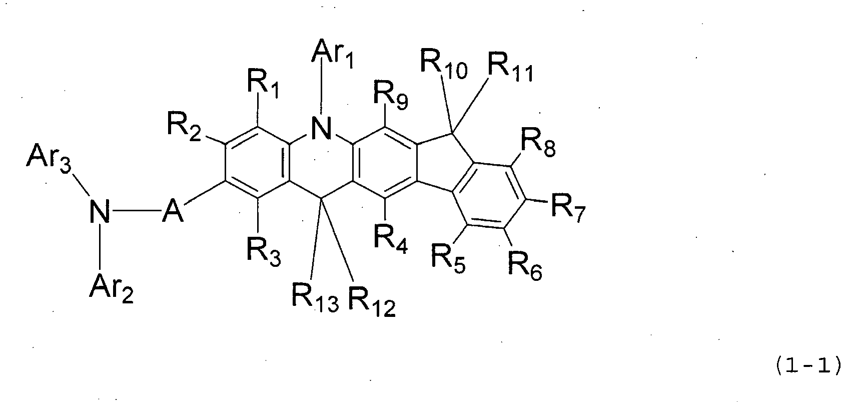

3. The compound having an indenoacridan ring structure according to claim 1 or 2, wherein A in the general formulae (1) or (1-1) is a divalent group of a substituted or unsubstituted aromatic hydrocarbon, or a divalent group of substituted or unsubstituted condensed polycyclic aromatics.

4. The compound having an indenoacridan ring structure according to claim 3, wherein the compound is represented by the following general formula (1-2), 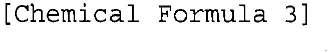 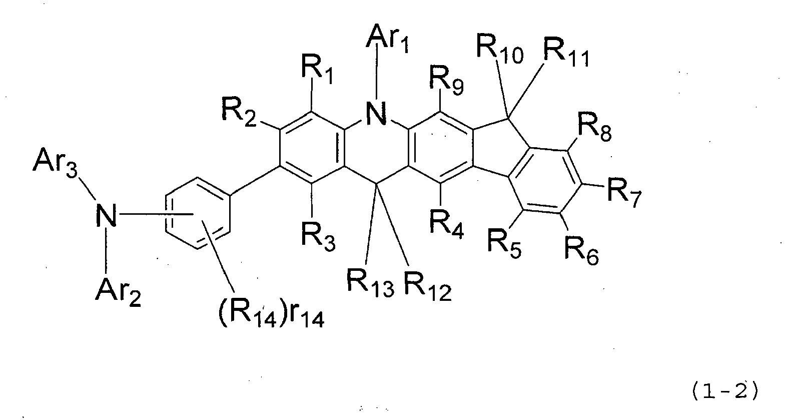 wherein R₁₄ represents a bonding group, r₁₄ is 1, and the benzene ring binding with R₁₄ and Ar₂ bind to each other via a single bond, substituted or unsubstituted methylene, an oxygen atom, or a sulfur atom to form a ring.

5. The compound having an indenoacridan ring structure according to claim 3, wherein the compound is represented by the following general formula (1-3), 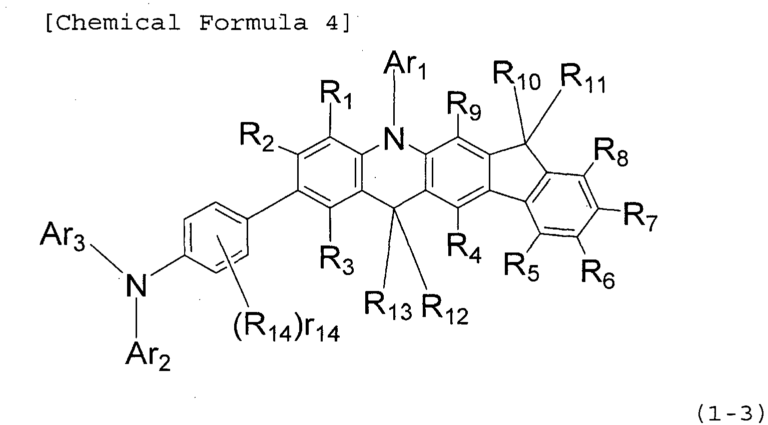 wherein R₁₄ represents a bonding group, r₁₄ is 1, and the benzene ring binding with R₁₄ and Ar₂ bind to each other via a single bond, substituted or unsubstituted methylene, an oxygen atom, or a sulfur atom to form a ring.

6. The compound having an indenoacridan ring structure according to claim 4, wherein the compound is represented by the following general formula (1-4), 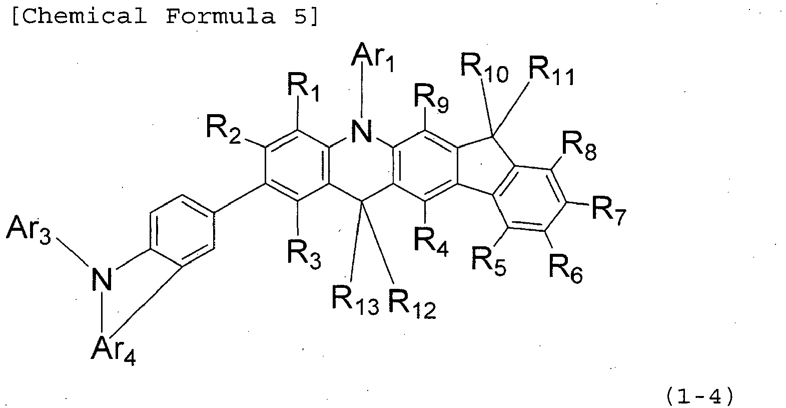 wherein, Ar₃ represents a substituted or unsubstituted aromatic hydrocarbon group, a substituted or unsubstituted aromatic heterocyclic group, or a substituted or unsubstituted condensed polycyclic aromatic group where the substituted or unsubstituted aromatic hydrocarbon group of Ar₁ is a substituted or unsubstituted biphenyl group, a substituted or unsubstituted terphenyl group, a substituted or unsubstituted fluorenyl group; Ar₄ represents a divalent group derived from a substituted or unsubstituted aromatic hydrocarbon group, a divalent group derived from a substituted or unsubstituted aromatic heterocyclic group, or a divalent group derived from a substituted or unsubstituted condensed polycyclic aromatic group; Ar₃ and Ar₄ may bind to each other via a single bond, substituted or unsubstituted methylene, an oxygen atom, or a sulfur atom to form a ring; R₁ to R₁₃ are as defined in claim 1.

7. The compound having an indenoacridan ring structure according to claim 4, wherein the compound is represented by the following general formula (1-4a), 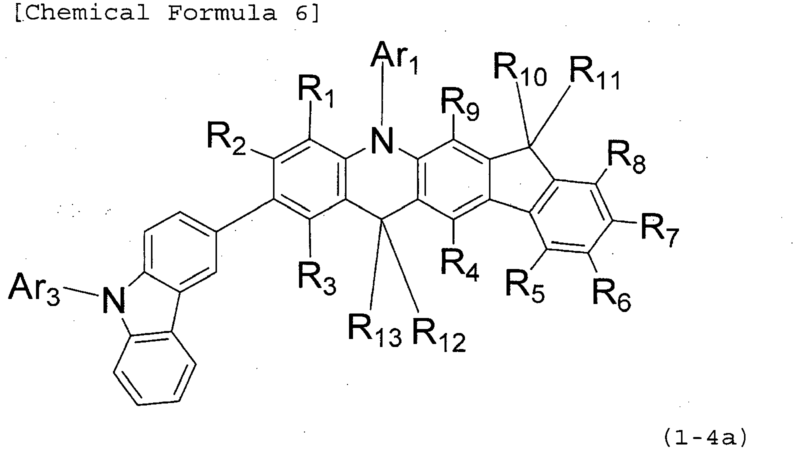 wherein Ar₃ represents a substituted or unsubstituted aromatic hydrocarbon group, a substituted or unsubstituted aromatic heterocyclic group, or a substituted or unsubstituted condensed polycyclic aromatic group where the substituted or unsubstituted aromatic hydrocarbon group of Ar₁ is a substituted or unsubstituted biphenyl group, a substituted or unsubstituted terphenyl group, a substituted or unsubstituted fluorenyl group; R₁ to R₁₃ are as defined in claim 1.

8. The compound having an indenoacridan ring structure according to claim 4, wherein the compound is represented by the following general formula (1-4b), 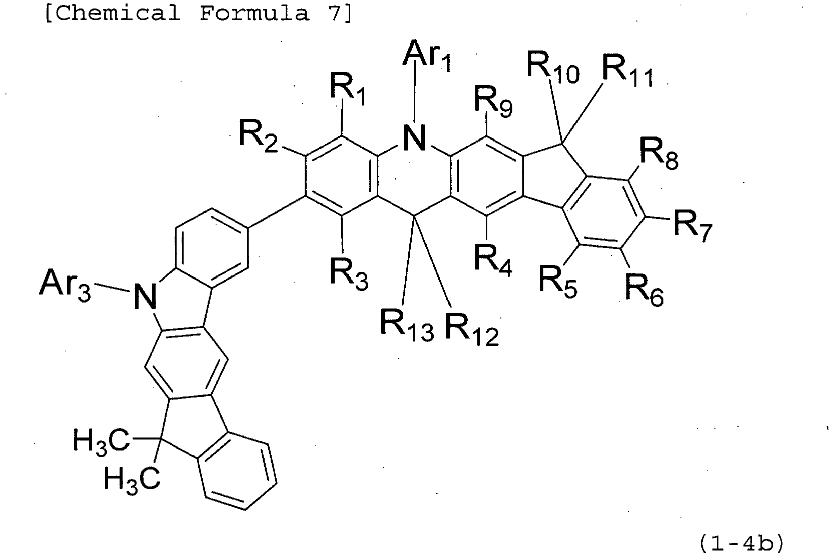 wherein Ar₃ represents a substituted or unsubstituted aromatic hydrocarbon group, a substituted or unsubstituted aromatic heterocyclic group, or a substituted or unsubstituted condensed polycyclic aromatic group where the substituted or unsubstituted aromatic hydrocarbon group of Ar₁ is a substituted or unsubstituted biphenyl group, a substituted or unsubstituted terphenyl group, a substituted or unsubstituted fluorenyl group; R₁ to R₁₃ are as defined in claim 1.

9. An organic electroluminescent device comprising a pair of electrodes, and one or more organic layers sandwiched between the pair of electrodes, wherein the compound having an indenoacridan ring structure of claim 1 is used as a constituent material of at least one of the organic layers.

10. The organic electroluminescent device according to claim 9, wherein the organic layer is a hole transport layer.

11. The organic electroluminescent device according to claim 9, wherein the organic layer is an electron blocking layer.

12. The organic electroluminescent device according to claim 9, wherein the organic layer is a hole injection layer.

13. The organic electroluminescent device according to claim 9, wherein the organic layer is a light emitting layer.

## Patentansprüche

1. Verbindung der folgenden allgemeinen Formel (1) mit einer Indenoacridan-Ringstruktur, 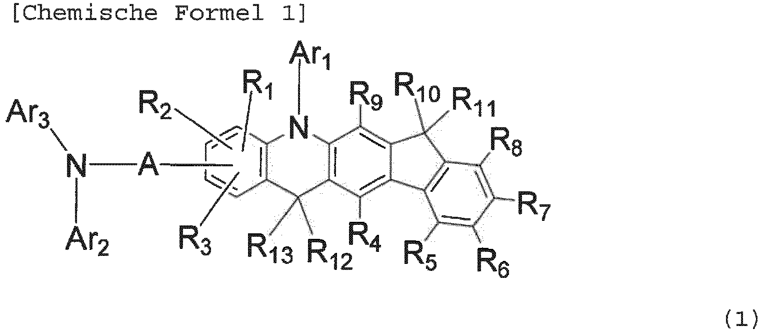
wobei A eine zweiwertige Gruppe eines substituierten oder unsubstituierten aromatischen Kohlenwasserstoffs, eine zweiwertige Gruppe eines substituierten oder unsubstituierten aromatischen heterocyclischen Rings oder eine zweiwertige Gruppe von substituierten oder unsubstituierten kondensierten polycyclischen Aromaten darstellt; Ar₂ und Ar₃ gleich oder verschieden sein können und eine substituierte oder unsubstituierte aromatische Kohlenwasserstoffgruppe, eine substituierte oder unsubstituierte aromatische heterocyclische Gruppe oder eine substituierte oder unsubstituierte kondensierte polycyclische aromatische Gruppe darstellen und Ar₁ eine substituierte oder unsubstituierte Biphenylgruppe, eine substituierte oder unsubstituierte Terphenylgruppe, eine substituierte oder unsubstituierte Fluorenylgruppe darstellt und wobei Ar₂ und Ar₃ aneinander über eine Einfachbindung, substituiertes oder unsubstituiertes Methylen, ein Sauerstoffatom oder ein Schwefelatom gebunden sein können, um einen Ring zu bilden; R₁ bis R₉ gleich oder verschieden sein können und ein Wasserstoffatom, ein Deuteriumatom, ein Fluoratom, ein Chloratom, Cyano, Nitro, lineares oder verzweigtes Alkyl mit 1 bis 6 Kohlenstoffatomen, die einen Substituenten aufweisen können, Cycloalkyl mit 5 bis 10 Kohlenstoffatomen, die einen Substituenten aufweisen können, lineares oder verzweigtes Alkenyl mit 2 bis 6 Kohlenstoffatomen, die einen Substituenten aufweisen können, lineares oder verzweigtes Alkyloxy mit 1 bis 6 Kohlenstoffatomen, die einen Substituenten aufweisen können, Cycloalkyloxy mit 5 bis 10 Kohlenstoffatomen, die einen Substituenten aufweisen können, eine substituierte oder unsubstituierte aromatische Kohlenwasserstoffgruppe, eine substituierte oder unsubstituierte aromatische heterocyclische Gruppe, eine substituierte oder unsubstituierte kondensierte polycyclische aromatische Gruppe oder substituiertes oder unsubstituiertes Aryloxy darstellen, die aneinander über eine Einfachbindung, substituiertes oder unsubstituiertes Methylen, ein Sauerstoffatom oder ein Schwefelatom gebunden sein können, um einen Ring zu bilden; R₁₀ bis R₁₃ gleich oder verschieden sein können und lineares oder verzweigtes Alkyl mit 1 bis 6 Kohlenstoffatomen, die einen Substituenten aufweisen können, Cycloalkyl mit 5 bis 10 Kohlenstoffatomen, die einen Substituenten aufweisen können, lineares oder verzweigtes Alkenyl mit 2 bis 6 Kohlenstoffatomen, die einen Substituenten aufweisen können, lineares oder verzweigtes Alkyloxy mit 1 bis 6 Kohlenstoffatomen, die einen Substituenten aufweisen können, Cycloalkyloxy mit 5 bis 10 Kohlenstoffatomen, die einen Substituenten aufweisen können, eine substituierte oder unsubstituierte aromatische Kohlenwasserstoffgruppe, eine substituierte oder unsubstituierte aromatische heterocyclische Gruppe, eine substituierte oder unsubstituierte kondensierte polycyclische aromatische Gruppe oder substituiertes oder unsubstituiertes Aryloxy darstellen, wobei R₁₀ und R₁₁ oder R₁₂ und R₁₃ aneinander über eine Einfachbindung, substituiertes oder unsubstituiertes Methylen, ein Sauerstoffatom oder ein Schwefelatom gebunden sein können, um einen Ring zu bilden; und wenn A eine zweiwertige Gruppe eines substituierten oder unsubstituierten aromatischen Kohlenwasserstoffs, eine zweiwertige Gruppe eines substituierten oder unsubstituierten aromatischen heterocyclischen Rings oder eine zweiwertige Gruppe von substituierten oder unsubstituierten kondensierten polycyclischen Aromaten darstellt, sind A und Ar₂ aneinander über eine Einfachbindung, substituiertes oder unsubstituiertes Methylen, ein Sauerstoffatom oder ein Schwefelatom gebunden, um einen Ring zu bilden, und
wobei der Substituent der durch Ar₁ in der allgemeinen Formel (1) dargestellten Gruppe ausgewählt ist aus einem Deuteriumatom; Halogenatomen, wie ein Fluoratom, ein Chloratom, ein Bromatom und ein Iodatom; linearem oder verzweigtem Alkyl mit 1 bis 6 Kohlenstoffatomen, wie Methyl, Ethyl, n-Propyl, Isopropyl, n-Butyl, Isobutyl, tert-Butyl, n-Pentyl, Isopentyl, Neopentyl und n-Hexyl; linearem oder verzweigtem Alkyloxy mit 1 bis 6 Kohlenstoffatomen, wie Methyloxy, Ethyloxy und Propyloxy; Alkenylen wie Allyl; Aryloxys, wie Phenyloxy und Tolyloxy; Arylalkyloxys, wie Benzyloxy und Phenethyloxy; aromatischen Kohlenwasserstoffgruppen oder kondensierten polycyclischen aromatischen Gruppen, wie Phenyl, Biphenylyl, Terphenylyl, Naphthyl, Anthracenyl, Phenanthryl, Fluorenyl, Indenyl, Pyrenyl, Perylenyl, Fluoranthenyl und Triphenylenyl; aromatischen heterocyclischen Gruppen, wie Pyridyl, Thienyl, Furyl, Pyrrolyl, Chinolyl, Isochinolyl, Benzofuranyl, Benzothienyl, Indolyl, Carbazolyl, Benzoxazolyl, Benzothiazolyl, Chinoxalyl, Benzoimidazolyl, Pyrazolyl, Dibenzofuranyl, Dibenzothienyl und Carbolinyl; Arylvinylen, wie Styryl und Naphthylvinyl; und Acylen, wie Acetyl und Benzoyl, wobei die Substituenten weiter mit den obigen Substituenten substituiert sein können und die Substituenten aneinander oder an Ar₁ über eine Einfachbindung, substituiertes oder unsubstituiertes Methylen, ein Sauerstoffatom oder ein Schwefelatom gebunden sein können, um einen Ring zu bilden.

2. Verbindung mit einer Indenoacridan-Ringstruktur nach Anspruch 1, wobei die Verbindung durch die folgende allgemeine Formel (1-1) dargestellt ist.

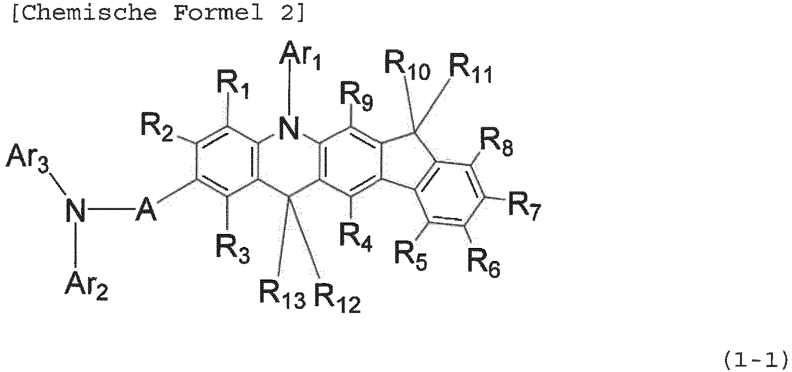

3. Verbindung mit einer Indenoacridan-Ringstruktur nach Anspruch 1 oder 2, wobei A in den allgemeinen Formeln (1) oder (1-1) eine zweiwertige Gruppe eines substituierten oder unsubstituierten aromatischen Kohlenwasserstoffs oder eine zweiwertige Gruppe von substituierten oder unsubstituierten kondensierten polycyclischen Aromaten darstellt.

4. Verbindung mit einer Indenoacridan-Ringstruktur nach Anspruch 3, wobei die Verbindung durch die folgende allgemeine Formel (1-2) dargestellt ist, 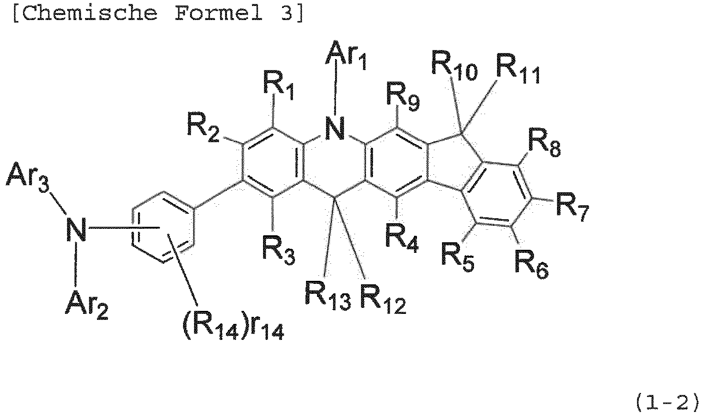 wobei R₁₄ eine verbindende Gruppe darstellt, r₁₄ 1 ist und der Benzolring, der mit R₁₄ bindet, und Ar₂ aneinander über eine Einfachbindung, substituiertes oder unsubstituiertes Methylen, ein Sauerstoffatom oder ein Schwefelatom gebunden sind, um einen Ring zu bilden.

5. Verbindung mit einer Indenoacridan-Ringstruktur nach Anspruch 3, wobei die Verbindung durch die folgende allgemeine Formel (1-3) dargestellt ist, 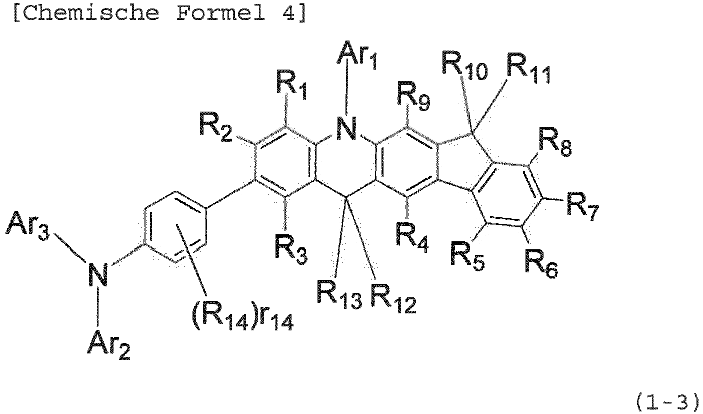 wobei R₁₄ eine verbindende Gruppe darstellt, r₁₄ 1 ist und der Benzolring, der mit R₁₄ bindet, und Ar₂ aneinander über eine Einfachbindung, substituiertes oder unsubstituiertes Methylen, ein Sauerstoffatom oder ein Schwefelatom gebunden sind, um einen Ring zu bilden.

6. Verbindung mit einer Indenoacridan-Ringstruktur nach Anspruch 4, wobei die Verbindung durch die folgende allgemeine Formel (1-4) dargestellt ist, 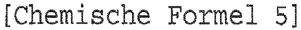 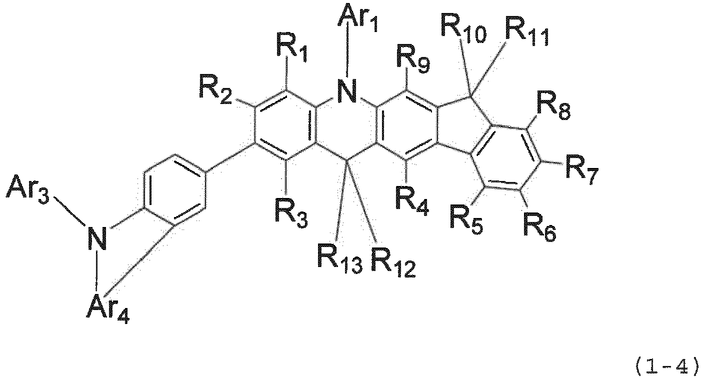 wobei Ar₃ eine substituierte oder unsubstituierte aromatische Kohlenwasserstoffgruppe, eine substituierte oder unsubstituierte aromatische heterocyclische Gruppe oder eine substituierte oder unsubstituierte kondensierte polycyclische aromatische Gruppe darstellt, wobei die substituierte oder unsubstituierte aromatische Kohlenwasserstoffgruppe von Ar₁ eine substituierte oder unsubstituierte Biphenylgruppe, eine substituierte oder unsubstituierte Terphenylgruppe, eine substituierte oder unsubstituierte Fluorenylgruppe darstellt; Ar₄ eine zweiwertige Gruppe, die von einer substituierten oder unsubstituierten aromatischen Kohlenwasserstoffgruppe abgeleitet ist, eine zweiwertige Gruppe, die von einer substituierten oder unsubstituierten aromatischen heterocyclischen Gruppe abgeleitet ist, oder eine zweiwertige Gruppe, die von einer substituierten oder unsubstituierten kondensierten polycyclischen aromatischen Gruppe abgeleitet ist, darstellt; Ar₃ und Ar₄ aneinander über eine Einfachbindung, substituiertes oder unsubstituiertes Methylen, ein Sauerstoffatom oder ein Schwefelatom gebunden sein können, um einen Ring zu bilden; R₁ bis R₁₃ wie in Anspruch 1 definiert sind.

7. Verbindung mit einer Indenoacridan-Ringstruktur nach Anspruch 4, wobei die Verbindung durch die folgende allgemeine Formel (1-4a) dargestellt ist, 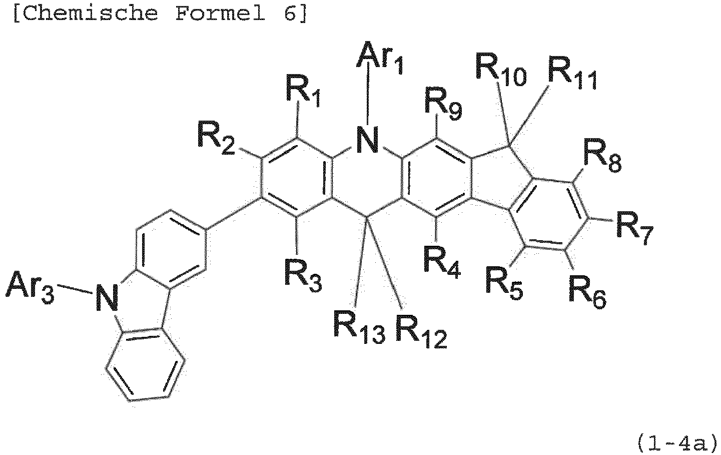 wobei Ar₃ eine substituierte oder unsubstituierte aromatische Kohlenwasserstoffgruppe, eine substituierte oder unsubstituierte aromatische heterocyclische Gruppe oder eine substituierte oder unsubstituierte kondensierte polycyclische aromatische Gruppe darstellt, wobei die substituierte oder unsubstituierte aromatische Kohlenwasserstoffgruppe von Ar₁ eine substituierte oder unsubstituierte Biphenylgruppe, eine substituierte oder unsubstituierte Terphenylgruppe, eine substituierte oder unsubstituierte Fluorenylgruppe darstellt; R₁ bis R₁₃ wie in Anspruch 1 definiert sind.

8. Verbindung mit einer Indenoacridan-Ringstruktur nach Anspruch 4, wobei die Verbindung durch die folgende allgemeine Formel (1-4b) dargestellt ist, 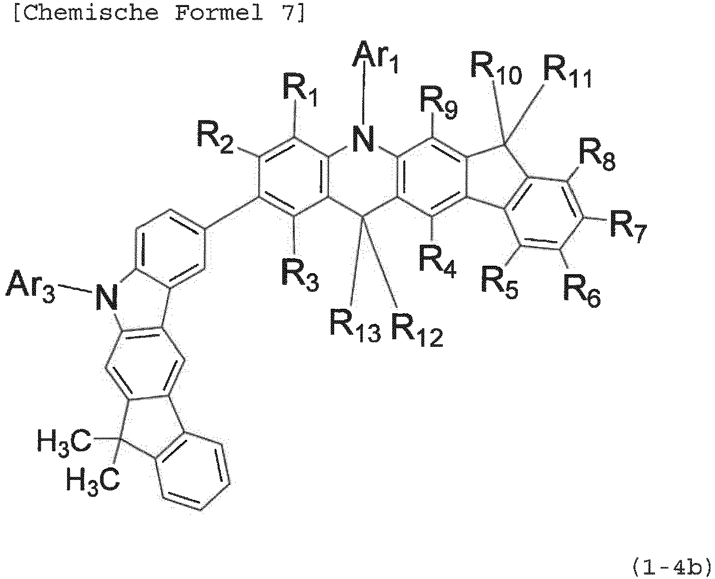 wobei Ar₃ eine substituierte oder unsubstituierte aromatische Kohlenwasserstoffgruppe, eine substituierte oder unsubstituierte aromatische heterocyclische Gruppe oder eine substituierte oder unsubstituierte kondensierte polycyclische aromatische Gruppe darstellt, wobei die substituierte oder unsubstituierte aromatische Kohlenwasserstoffgruppe von Ar₁ eine substituierte oder unsubstituierte Biphenylgruppe, eine substituierte oder unsubstituierte Terphenylgruppe, eine substituierte oder unsubstituierte Fluorenylgruppe darstellt; R₁ bis R₁₃ wie in Anspruch 1 definiert sind.

9. Organische Elektrolumineszenzvorrichtung, umfassend ein Elektrodenpaar und eine oder mehrere organische Schichten, die zwischen dem Elektrodenpaar sandwichartig angeordnet sind, wobei die Verbindung mit einer Indenoacridan-Ringstruktur nach Anspruch 1 als ein konstituierendes Material von mindestens einer der organischen Schichten verwendet wird.

10. Organische Elektrolumineszenzvorrichtung nach Anspruch 9, wobei die organische Schicht eine Lochtransportschicht ist.

11. Organische Elektrolumineszenzvorrichtung nach Anspruch 9, wobei die organische Schicht eine Elektronenblockierschicht ist.

12. Organische Elektrolumineszenzvorrichtung nach Anspruch 9, wobei die organische Schicht eine Lochinjektionsschicht ist.

13. Organische Elektrolumineszenzvorrichtung nach Anspruch 9, wobei die organische Schicht eine lichtemittierende Schicht ist.

## Revendications

1. Composé de la formule générale suivante (1) ayant une structure d'anneau d'indéno-acridane, 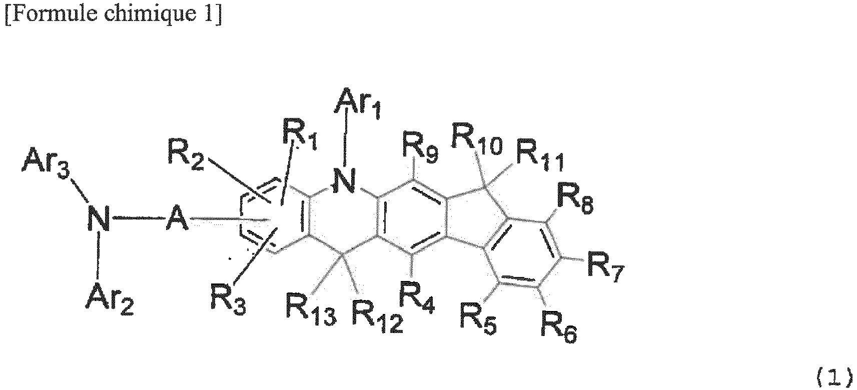
dans laquelle A représente un groupe divalent d'un hydrocarbure aromatique substitué ou non substitué, un groupe divalent d'un anneau hétérocyclique aromatique substitué ou non substitué, ou un groupe divalent de composés aromatiques polycycliques condensés substitués ou non substitués ; Ar₂ et Ar₃ peuvent être identiques ou différents, et représentent un groupe hydrocarbure aromatique substitué ou non substitué, un groupe hétérocyclique aromatique substitué ou non substitué, ou un groupe aromatique polycyclique condensé substitué ou non substitué, et
Ar₁ est un groupe biphényle substitué ou non substitué, un groupe terphényle substitué ou non substitué, un groupe fluorényle substitué ou non substitué, et où Ar₂ et Ar₃ peuvent se lier l'un à l'autre par le biais d'une liaison unique, méthylène substitué ou non substitué, un atome d'oxygène, ou un atome de soufre pour former un anneau ; R₁ à R₉ peuvent être identiques ou différents, et représentent un atome d'hydrogène, un atome de deutérium, un atome de fluor, un atome de chlore, cyano, nitro, alkyle linéaire ou ramifié de 1 à 6 atomes de carbone qui peut avoir un substituant, cycloalkyle de 5 à 10 atomes de carbone qui peut avoir un substituant, alkényle linéaire ou ramifié de 2 à 6 atomes de carbone qui peut avoir un substituant, alkyloxy linéaire ou ramifié de 1 à 6 atomes de carbone qui peut avoir un substituant, cycloalkyloxy de 5 à 10 atomes de carbone qui peut avoir un substituant, un groupe hydrocarbure aromatique substitué ou non substitué, un groupe hétérocyclique aromatique substitué ou non substitué, un groupe aromatique polycyclique condensé substitué ou non substitué, ou aryloxy substitué ou non substitué, qui peuvent se lier l'un à l'autre par le biais d'une liaison unique, méthylène substitué ou non substitué, un atome d'oxygène, ou un atome de soufre pour former un anneau ; R₁₀ à R₁₃ peuvent être identiques ou différents, et représentent alkyle linéaire ou ramifié de 1 à 6 atomes de carbone qui peut avoir un substituant, cycloalkyle de 5 à 10 atomes de carbone qui peut avoir un substituant, alkényle linéaire ou ramifié de 2 à 6 atomes de carbone qui peut avoir un substituant, alkyloxy linéaire ou ramifié de 1 à 6 atomes de carbone qui peut avoir un substituant, cycloalkyloxy de 5 à 10 atomes de carbone qui peut avoir un substituant, un groupe hydrocarbure aromatique substitué ou non substitué, un groupe hétérocyclique aromatique substitué ou non substitué, un groupe aromatique polycyclique condensé substitué ou non substitué, ou aryloxy substitué ou non substitué, où R₁₀ et R₁₁, ou R₁₂ et R₁₃ peuvent se lier l'un à l'autre par le biais d'une liaison unique, méthylène substitué ou non substitué, un atome d'oxygène, ou un atome de soufre pour former un anneau ; et lorsque A est un groupe divalent d'un hydrocarbure aromatique substitué ou non substitué, un groupe divalent d'un anneau hétérocyclique aromatique substitué ou non substitué, ou un groupe divalent de composés aromatiques polycycliques condensés substitués ou non substitués, A et Ar₂ se lient l'un à l'autre par le biais d'une liaison unique, méthylène substitué ou non substitué, un atome d'oxygène, ou un atome de soufre pour former un anneau, et dans laquelle le substituant du groupe représenté par Ar₁ dans la formule générale (1) est sélectionné parmi un atome de deutérium ; des atomes d'halogène tels qu'un atome de fluor, un atome de chlore, un atome de brome, et un atome d'iode ; alkyle linéaire ou ramifié de 1 à 6 atomes de carbone tel que méthyle, éthyle, n-propyle, isopropyle, n-butyle, isobutyle, tert-butyle, n-pentyle, isopentyle, néopentyle, et n-hexyle ; alkyloxy linéaire ou ramifié de 1 à 6 atomes de carbone tel que méthyloxy, éthyloxy, et propyloxy ; des alkényles tels qu'allyle ; des aryloxys tels que phényloxy et tolyloxy ; des arylalkyloxys tels que benzyloxy et phénéthyloxy ; des groupes hydrocarbures aromatiques ou groupes aromatiques polycycliques condensés tels que phényle, biphénylyle, terphénylyle, naphtyle, anthracényle, phénanthryle, fluorényle, indényle, pyrényle, pérylényle, fluoranthényle, et triphénylényle ; des groupes hétérocycliques aromatiques tels que pyridyle, thiényle, furyle, pyrrolyle, quinolyle, isoquinolyle, benzofuranyle, benzothiényle, indolyle, carbazolyle, benzooxazolyle, benzothiazolyle, quinoxalyle, benzoimidazolyle, pyrazolyle, dibenzofuranyle, dibenzothiényle, et carbolinyle ; des arylvinyles tels que styryle et naphtylvinyle ; et des acyles tels qu'acétyle et benzoyle, dans laquelle les substituants peuvent être davantage substitués par les substituants ci-dessus, et les substituants peuvent se lier les uns aux autres, ou à Ar₁ par le biais d'une liaison unique, méthylène substitué ou non substitué, un atome d'oxygène, ou un atome de soufre pour former un anneau.

2. Composé ayant une structure d'anneau d'indéno-acridane selon la revendication 1, dans lequel le composé est représenté par la formule générale suivante (1-1).

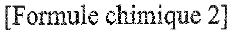

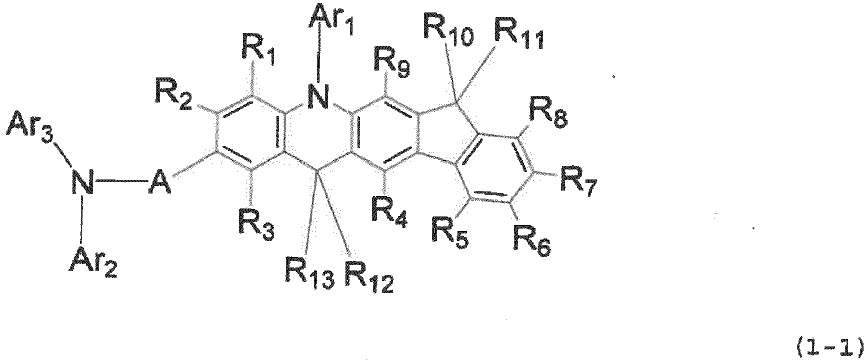

3. Composé ayant une structure d'anneau d'indéno-acridane selon la revendication 1 ou 2, dans lequel A dans les formules générales (1) ou (1-1) est un groupe divalent d'un hydrocarbure aromatique substitué ou non substitué, ou un groupe divalent de composés aromatiques polycycliques condensés substitués ou non substitués.

4. Composé ayant une structure d'anneau d'indéno-acridane selon la revendication 3, dans lequel le composé est représenté par la formule générale suivante (1-2), 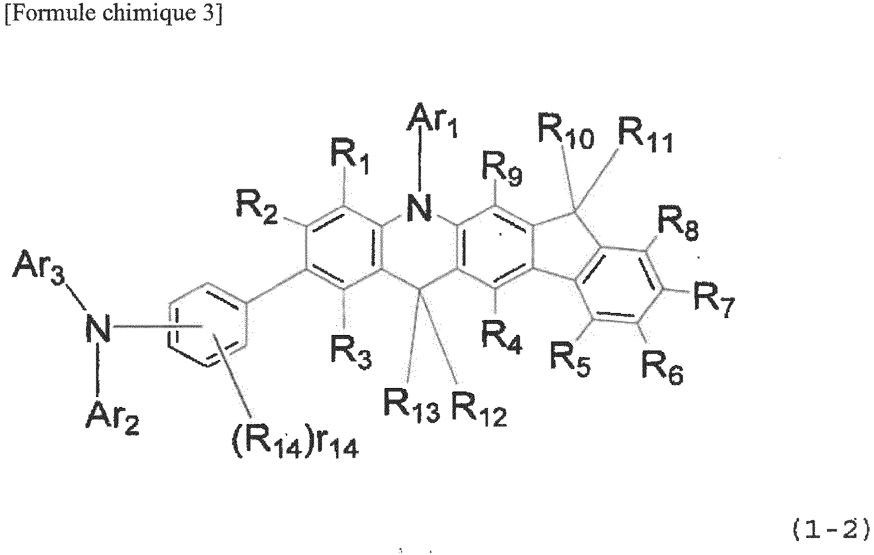 dans laquelle R₁₄ représente un groupe de liaison, r₁₄ vaut 1, et l'anneau de benzène se liant à R₁₄ et Ar₂ se lient l'un à l'autre par le biais d'une liaison unique, méthylène substitué ou non substitué, un atome d'oxygène, ou un atome de soufre pour former un anneau.

5. Composé ayant une structure d'anneau d'indéno-acridane selon la revendication 3, dans lequel le composé est représenté par la formule générale suivante (1-3), 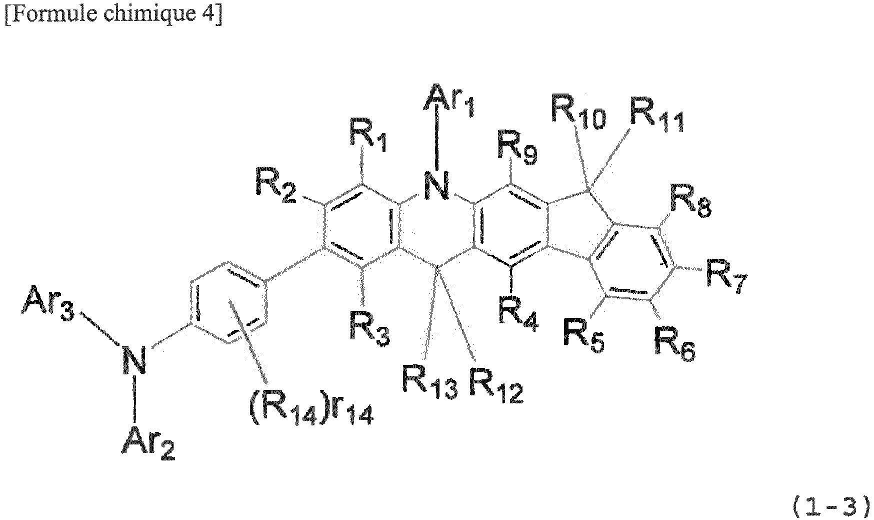 dans laquelle R₁₄ représente un groupe de liaison, r₁₄ vaut 1, et l'anneau de benzène se liant à R₁₄ et Ar₂ se lient l'un à l'autre par le biais d'une liaison unique, méthylène substitué ou non substitué, un atome d'oxygène, ou un atome de soufre pour former un anneau.

6. Composé ayant une structure d'anneau d'indéno-acridane selon la revendication 4, dans lequel le composé est représenté par la formule générale suivante (1-4), 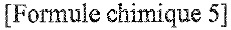 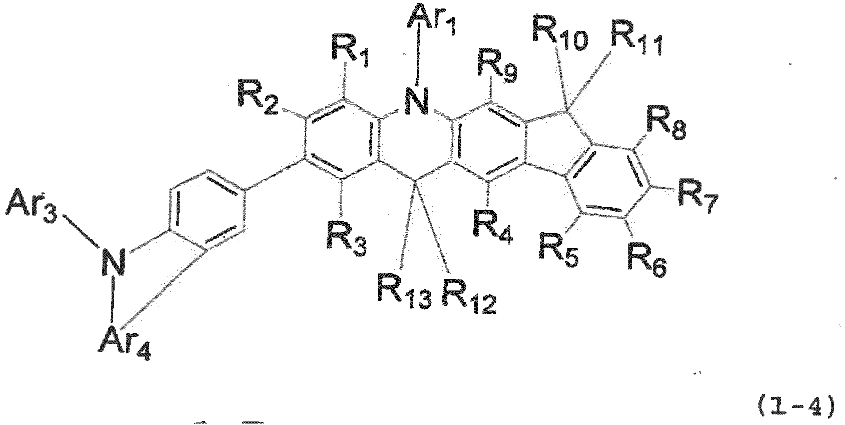 dans laquelle Ar₃ représente un groupe hydrocarbure aromatique substitué ou non substitué, un groupe hétérocyclique aromatique substitué ou non substitué, ou un groupe aromatique polycyclique condensé substitué ou non substitué où le groupe hydrocarbure aromatique substitué ou non substitué de Ar₁ est un groupe biphényle substitué ou non substitué, un groupe terphényle substitué ou non substitué, un groupe fluorényle substitué ou non substitué ; Ar₄ représente un groupe divalent dérivé d'un groupe hydrocarbure aromatique substitué ou non substitué, un groupe divalent dérivé d'un groupe hétérocyclique aromatique substitué ou non substitué, ou un groupe divalent dérivé d'un groupe aromatique polycyclique condensé substitué ou non substitué ; Ar₃ et Ar₄ peuvent se lier l'un à l'autre par le biais d'une liaison unique, méthylène substitué ou non substitué, un atome d'oxygène, ou un atome de soufre pour former un anneau ; R₁ à R₁₃ sont tels que définis à la revendication 1.

7. Composé ayant une structure d'anneau d'indéno-acridane selon la revendication 4, dans lequel le composé est représenté par la formule générale suivante (1-4a), 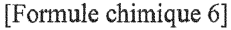 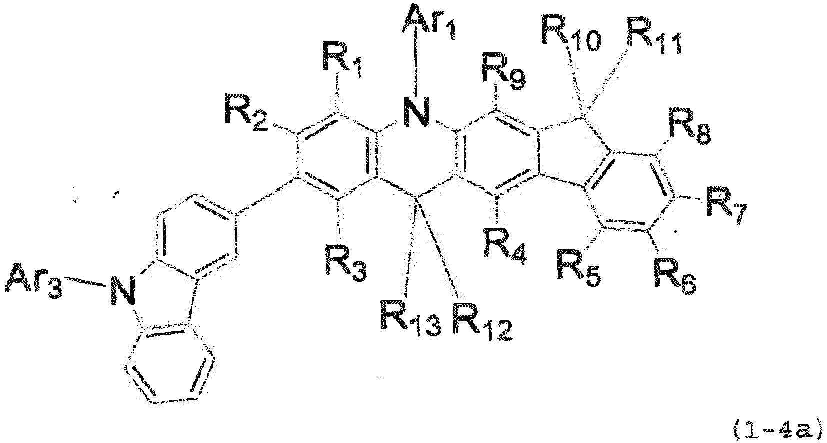 dans laquelle Ar₃ représente un groupe hydrocarbure aromatique substitué ou non substitué, un groupe hétérocyclique aromatique substitué ou non substitué, ou un groupe aromatique polycyclique condensé substitué ou non substitué où le groupe hydrocarbure aromatique substitué ou non substitué de Ar₁ est un groupe biphényle substitué ou non substitué, un groupe terphényle substitué ou non substitué, un groupe fluorényle substitué ou non substitué ; R₁ à R₁₃ sont tels que définis à la revendication 1.

8. Composé ayant une structure d'anneau d'indéno-acridane selon la revendication 4, dans lequel le composé est représenté par la formule générale suivante (1-4b), 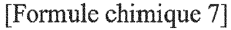 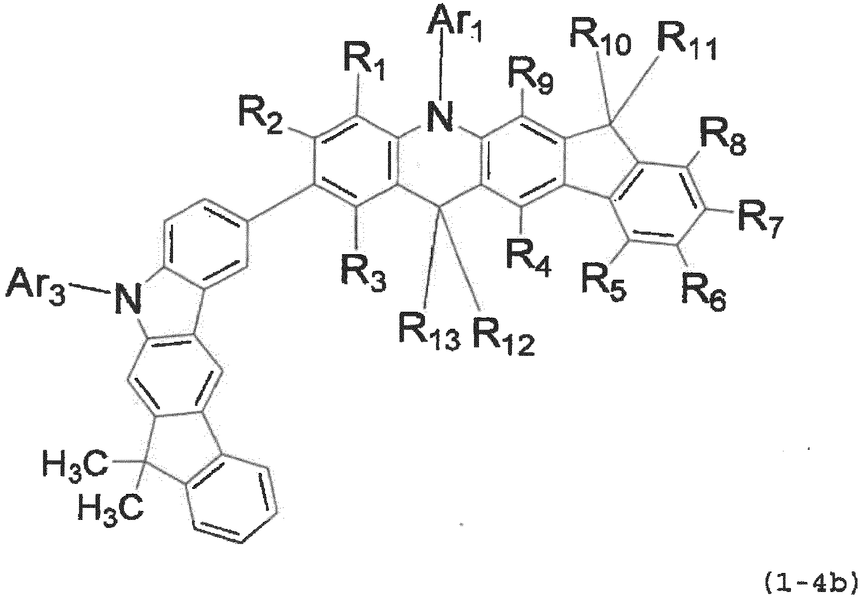 dans laquelle Ar₃ représente un groupe hydrocarbure aromatique substitué ou non substitué, un groupe hétérocyclique aromatique substitué ou non substitué, ou un groupe aromatique polycyclique condensé substitué ou non substitué où le groupe hydrocarbure aromatique substitué ou non substitué de Ar₁ est un groupe biphényle substitué ou non substitué, un groupe terphényle substitué ou non substitué, un groupe fluorényle substitué ou non substitué ; R₁ à R₁₃ sont tels que définis à la revendication 1.

9. Dispositif électroluminescent organique comprenant une paire d'électrodes, et une ou plusieurs couches organiques prises en sandwich entre la paire d'électrodes, dans lequel le composé ayant une structure d'anneau d'indéno-acridane selon la revendication 1 est utilisé comme un matériau constituant d'au moins une des couches organiques.

10. Dispositif électroluminescent organique selon la revendication 9, dans lequel la couche organique est une couche de transport de trous.

11. Dispositif électroluminescent organique selon la revendication 9, dans lequel la couche organique est une couche de blocage d'électrons.

12. Dispositif électroluminescent organique selon la revendication 9, dans lequel la couche organique est une couche d'injection de trous.

13. Dispositif électroluminescent organique selon la revendication 9, dans lequel la couche organique est une couche d'émission lumineuse.
